# EUROPÄISCHE PATENTSCHRIFT

(11) **EP 1 427 730 B1**
(45) Veröffentlichungstag und Bekanntmachung des Hinweises auf die Patenterteilung: **12.07.2006**
(21) Anmeldenummer: 02772249.5
(22) Anmeldetag: 30.08.2002
(51) Int. Cl.: C07D 487/04, A61K 31/505, A61P 35/00, A61P 31/18

(54) **NEUE DIHYDROPTERIDINONE, VERFAHREN ZU DEREN HERSTELLUNG UND DEREN VERWENDUNG ALS ARZNEIMITTEL**
NOVEL DIHYDROPTERIDINONES, METHOD FOR PRODUCING THE SAME AND THE USE THEREOF AS MEDICAMENTS
NOUVELLES DIHYDROPTERIDINONES, PROCEDES POUR LES PRODUIRE ET LEUR UTILISATION EN TANT QUE MEDICAMENTS

(30) Priorität: 04.09.2001 DE 10143272
(43) Veröffentlichungstag der Anmeldung: 16.06.2004
(73) Patentinhaber: Boehringer Ingelheim Pharma GmbH & Co.KG, 55216 Ingelheim am Rhein (DE)
(72) Erfinder: HOFFMANN, Matthias, 88441 MITTELBIBERACH (DE); GRAUERT, Matthias, 88400 BIBERACH (DE); BREITFELDER, Steffen, 88433 ASSMANNSHARDT (DE); EICKMEIER, Christian, 88441 MITTELBIBERACH (DE); POHL, Gerald, 88400 BIBERACH (DE); LEHMANN-LINTZ, Thorsten, 88416 OCHSENHAUSEN (DE); REDEMANN, Norbert, 88400 BIBERACH (DE); SCHNAPP, Gisela, 88400 BIBERACH-RINDENMOOS (DE); STEEGMAIER, Martin, A-1120 WIEN (AT); BAUER, Eckhart, 88400 BIBERACH (DE); QUANT, Jens, Jürgen, A-2353 GUNTRAMSDORF (AT)
(86) Internationale Anmeldenummer: PCT/EP2002/009728
(87) Internationale Veröffentlichungsnummer: WO 2003/020722

(56) Entgegenhaltungen:
- EP-A- 0 399 856
- EP-A- 0 429 149
- WO-A-01/19825
- WO-A-02/076954
- US-A- 5 698 556

## Beschreibung

Die vorliegende Erfindung betrifft neue Dihydropteridinone der allgemeinen Formel (I) 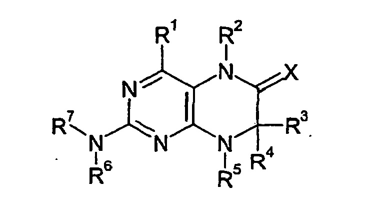 wobei die Reste X, R¹, R², R³, R⁴, R⁵, R⁶ und R⁷ die in den Ansprüchen und der Beschreibung genannten Bedeutungen haben, deren Isomere,
Verfahren zur Herstellung dieser Dihydropteridinone sowie deren Verwendung als Arzneimittel.

### Hintergrund der Erfindung

Pteridinon-Derivate sind als Wirkstoffe mit antiproliferativer Wirkung aus dem Stand der Technik bekannt. WO 01/019825 beschreibt die Verwendung von Pteridinonderivaten zur Behandlung von Tumor- und Viruserkrankungen.
Die Resistenz vieler Tumorarten erfordert die Entwicklung neuer Arzneimittel zur Tumorbekämpfung.
Es ist die Aufgabe der vorliegenden Erfindung neue Verbindungen mit antiinflammatorischer und antiproliferativer Wirkung bereitzustellen.

### Detaillierte Beschreibung der Erfindung

Überraschenderweise wurde gefunden, daß Verbindungen der allgemeinen
Formel (I), worin die Reste X und R¹ bis R⁷ die nachstehend genannten Bedeutungen haben, als Inhibitoren spezifischer Zellzykluskinasen wirken. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

Die vorliegende Erfindung betrifft daher Verbindungen der allgemeinen Formel (I) 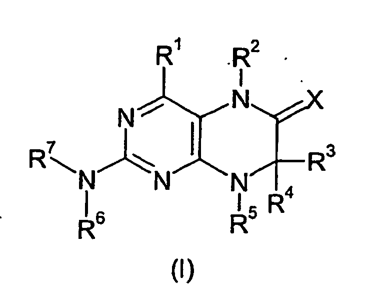 worin
R¹ ein Rest ausgewählt aus der Gruppe bestehend aus Wasserstoff, NH₂, XH, Halogen und einer gegebenenfalls durch ein oder mehrere Halogenatome substituierten C₁-C₃-Alkyl-Gruppe,
R² ein Rest ausgewählt aus der Gruppe bestehend aus Wasserstoff, CHO, XH, -X-C₁-C₂-Alkyl und einer gegebenenfalls substituierten C₁-C₃-Alkyl-Gruppe,
R³, R⁴ gleich oder verschieden, ein Rest ausgewählt aus der Gruppe bestehend aus gegebenenfalls substituiertem C₁-C₁₀-Alkyl, C₂-C₁₀-Alkenyl, C₂-C₁₀-Alkinyl, Aryl, Heteroaryl, C₃-C₈-Cycloalkyl, C₃-C₈-Heterocycloalkyl, -X-Aryl, -X-Heteroaryl, -X-Cycloalkyl, -X-Heterocycloalkyl, -NR⁸-Aryl, -NR⁸-Heteroaryl, -NR⁸-Cycloalkyl, - und -NR⁸-Heterocycloalkyl, oder
ein Rest ausgewählt aus der Gruppe bestehend aus Wasserstoff, Halogen, COXR⁸, CON(R⁸)₂, COR⁸ und XR⁸, oder
R³ und R⁴ gemeinsam eine 2- bis 5-gliedrige Alkylbrücke, die 1 bis 2 Heteroatome enthalten kann,
R⁵ Wasserstoff oder ein Rest ausgewählt aus der Gruppe bestehend aus gegebenenfalls substituiertem C₁-C₁₀-Alkyl, C₂-C₁₀-Alkenyl, C₂-C₁₀-Alkinyl, Aryl, Heteroaryl und -C₃-C₆-Cycloalkyl , oder
R³ und R⁵ oder R⁴und R⁵ gemeinsam eine gesättigte oder ungesättigte C₃-C₄-Alkylbrücke, die 1 bis 2 Heteroatome enthalten kann,
R⁶ gegebenenfalls substituiertes Aryl oder Heteroaryl,
R⁷ Wasserstoff oder -CO-X-C₁-C₄-Alkyl, und
X jeweils unabhängig voneinander, O oder S,
R⁸ jeweils unabhängig voneinander, Wasserstoff oder ein Rest ausgewählt aus der Gruppe bestehend aus gegebenenfalls substituiertem C₁-C₄-Alkyl, C₂-C₄₋Alkenyl, C₂-C₄-Alkinyl und Phenyl,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.
Bevorzugt sind Verbindungen der Formel (I), worin
X und R⁶ die angegebene Bedeutung aufweisen, und
R¹ Wasserstoff,
R² ein Rest ausgewählt aus der Gruppe bestehend aus einer CHO, OH, und CH₃-Gruppe,
R³, R⁴ gleich oder verschieden, ein Rest ausgewählt aus der Gruppe bestehend aus Wasserstoff, gegebenenfalls substituiertem C₁-C₆-Alkyl, C₂-C₆-Alkenyl, C₂-C₆₋Alkinyl, C₃-C₇-Cycloalkyl, oder
R³ und R⁴ gemeinsam eine C₂-C₅-Alkylbrücke,
R⁵ ein Rest ausgewählt aus der Gruppe bestehend aus gegebenenfalls substituiertem C₁-C₁₀-Alkyl, C₂-C₁₀-Alkenyl, C₂-C₁₀-Alkinyl, C₃-C₆-Cycloalkyl und C₃-C₆-Cycloalkenyl, oder
R³ und R⁵ oder R⁴und R⁵ gemeinsam eine gesättigte oder ungesättigte C₃-C₄-Alkylbrücke, die 1 bis 2 Heteroatome enthalten kann, und
R⁷ Wasserstoff
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

Besonders bevorzugt sind Verbindungen der Formel (I), worin
R¹-R⁵, R⁷, R⁸ und X die angegebene Bedeutung aufweisen, und
R⁶ ein Rest der allgemeinen Formel 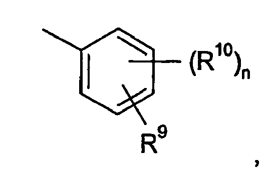 worin
n 1, 2, 3 oder 4,
R⁹ ein Rest ausgewählt aus der Gruppe bestehend aus gegebenenfalls substituiertem C₁-C₆-Alkyl, C₂-C₆-Alkenyl, C₂-C₆-Alkinyl, -CONH-C₁-C₁₀-Alkylen, -O-Aryl, -O-Heteroaryl, -O-Cycloalkyl, -O-Heterocycloalkyl, Aryl, Heteroaryl, Cycloalkyl und Heterocycloalkyl oder
ein Rest ausgewählt aus der Gruppe bestehend aus -O-C₁-C₆-Alkyl-Q¹, -CONR⁸-C₁-C₁₀-Alkyl-Q¹, -CONR⁸-C₂-C₁₀-Alkenyl-Q¹, -CONR⁸-Q², Halogen, OH, -SO₂R⁸, -SO₂N(R⁸)₂, -COR⁸, -COOR⁸, -N(R⁸)₂, -NHCOR⁸, CONR⁸OC₁-C₁₀ AlkylQ¹ und CONR⁸OQ²,
Q¹ Wasserstoff, -NHCOR⁸, oder ein Rest ausgewählt aus der Gruppe bestehend aus einer gegebenenfalls substituierten -NH-Aryl-, -NH-Heteroaryl-, Aryl-, Heteroaryl-, C₃-C₈-Cycloalkyl- und Heterocycloalkyl-Gruppe,
Q² Wasserstoff oder ein Rest ausgewählt aus der Gruppe bestehend aus einer gegebenenfalls substituierten Aryl-, Heteroaryl-, C₃-C₈-Heterocycloalkyl- ,C₃-C₈₋Cycloalkyl- und C₁-C₄-Alkyl-C₃-C₈-cycloalkyl-Gruppe,
R¹⁰ gleich oder verschieden, ein Rest ausgewählt aus der Gruppe bestehend aus gegebenenfalls substituiertem C₁-C₆-Alkyl , C₂-C₆-Alkenyl und C₂-C₆-Alkinyl, -O-C₁-C₆-Alkyl, -O-C₂-C₆-Alkenyl, -O-C₂-C₆-Alkinyl, C₃-C₆-Heterocycloalkyl und C₃-C₆-Cycloalkyl, oder
   ein Rest ausgewählt aus der Gruppe bestehend aus Wasserstoff, -CONH₂, -COOR⁸, -OCON(R⁸)₂, -N(R⁸)₂, -NHCOR⁸, -NHCON(R⁸)₂, -NO₂ und Halogen, oder
   benachbarte Reste R⁹ und R¹⁰ gemeinsam eine Brücke der allgemeinen Formel 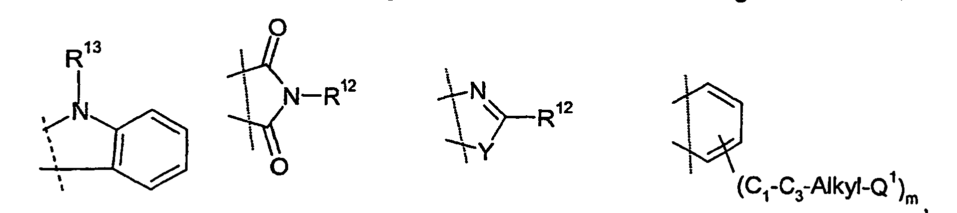
Y O, S oder NR¹¹,
m 0, 1 oder 2
R¹¹ Wasserstoff oder C₁-C₂-Alkyl, und
R¹² Wasserstoff oder ein Rest ausgewählt aus der Gruppe bestehend aus gegebenenfalls substituiertem Phenyl, Pyridyl, Pyrazinyl, Pyrimidinyl, Pyridazinyl, -C₁-C₃-Alkyl-Phenyl, -C₁-C₃-Alkyl-Pyridyl, -C₁-C₃-Alkyl-Pyrazinyl, -C₁-C₃-Alkyl-Pyrimidinyl und -C₁-C₃-Alkyl-Pyridazinyl,
R¹³ C₁-C₆-Alkyl
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

Insbesondere bevorzugt sind Verbindungen der Formel (I), worin
R³-R⁶, R⁸ und X die angegebene Bedeutung aufweisen, und
R¹ Wasserstoff,
R² CH₃, und
R⁷ Wasserstoff,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

Ein weiterer Gegenstand der Erfindung sind Verbindungen der Formel (I), worin X und R¹-R⁷ die angegebene Bedeutung aufweisen, zur Verwendung als Arzneimittel.

Erfindungsgemäß von besonderer Bedeutung sind Verbindungen der Formel (I), worin X und R¹-R⁷ die angegebene Bedeutung aufweisen, zur Verwendung als Arzneimittel mit antiproliferativer Wirkung.

Ein weiterer Gegenstand der Erfindung ist die Verwendung einer Verbindung der Formel (I), worin X und R¹-R⁷ die angegebene Bedeutung aufweisen, zur Herstellung eines Arzneimittels zur Behandlung und/oder Prävention von Krebs, Infektionen, Entzündungs- und Autoimmunerkrankungen.

Ein weiterer Gegenstand der Erfindung ist eine Methode zur Behandlung und/oder Prävention von Krebs, Infektionen, Entzündungs- und Autoimmunerkrankungen, dadurch gekennzeichnet, daß man einem Patienten eine effektive Menge einer Verbindung der Formel (I), worin X und R¹-R⁷ die angegebene Bedeutung aufweisen, verabreicht.

Ein weiterer Gegenstand der Erfindung sind pharmazeutische Zubereitungen, enthaltend als Wirkstoff eine oder mehrere Verbindungen der allgemeinen Formel (I), worin X und R¹-R⁷ die angegebene Bedeutung aufweisen, oder deren physiologisch verträgliche Salze gegebenenfalls in Kombination mit üblichen Hilfs- und/oder Trägerstoffen.

Ein weiterer Gegenstand der Erfindung ist ein Verfahren zur Herstellung einer Verbindung der allgemeinen Formel (I), 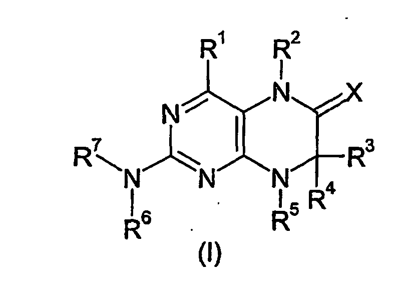 worin
R¹-R⁷ und X die vorstehend genannten Bedeutungen aufweisen,
dadurch gekennzeichnet, daß eine Verbindung der allgemeinen Formel (II) 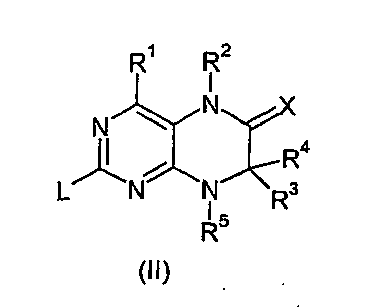 worin
R¹-R⁵ und X die vorstehend genannten Bedeutungen aufweisen und L eine Abgangsgruppe ist,
mit einer gegebenenfalls substituierten Verbindung der allgemeinen Formel (III) 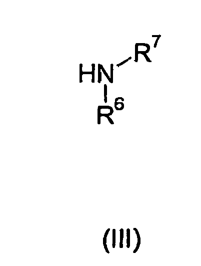 worin
R⁶ und R⁷ die vorstehend genannten Bedeutungen aufweisen,
umgesetzt wird.

Ein weiterer Gegenstand der Erfindung ist eine Verbindung der Formel (II), 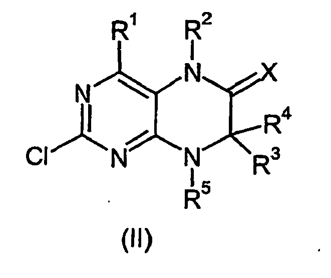 worin
R¹-R⁵ und X die vorstehend genannten Bedeutungen aufweisen. Verbindungen der Formel (II) stellen wichtige Zwischenprodukte zur Herstellung der erfindungsgemäßen Verbindungen der Formel (I) dar.

Ein weiterer Gegenstand der Erfindung ist ein Verfahren zur Herstellung einer Verbindung der allgemeinen Formel (I), 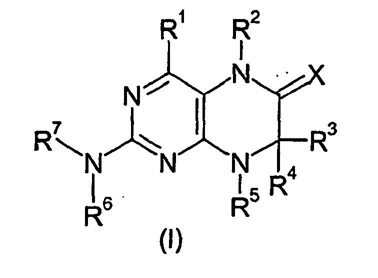 worin
R⁶ ein Rest der allgemeinen Formel, 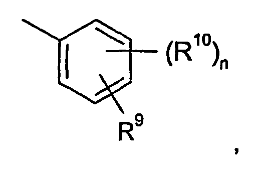 R⁹ ein gegebenenfalls substituierter Rest -CONH-C₁-C₁₀-Alkylen oder ein Rest ausgewählt aus der Gruppe bestehend aus -CONR⁸-C₁-C₁₀-Alkyl-Q¹, -CONR⁸-C₂-C₁₀-Alkenyl-Q¹, -CONR⁸-Q², und -COOR⁸ bedeutet, und R¹-R⁵, R⁷, R¹⁰,n und X die vorstehend genannten Bedeutungen aufweisen, dadurch gekennzeichnet, daß eine Verbindung der allgemeinen Formel (IA) 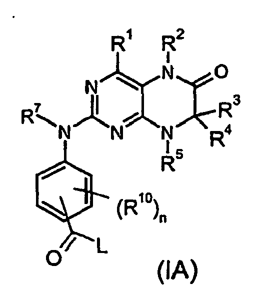 worin
R¹ bis R⁵, R⁷ und R¹⁰ vorstehend genannten Bedeutungen aufweisen, und
L eine Abgangsgruppe bedeutet,
mit einem primären oder sekundären Amin zu dem entsprechenden Amid oder mit einem Alkohol zu dem entsprechenden Ester umsetzt wird.

Als Alkylgruppen sowie Alkylgruppen, welche Bestandteil anderer Reste sind, werden verzweigte und unverzweigte Alkylgruppen mit 1 bis 10 Kohlenstoffatomen bevorzugt 1-6, besonders bevorzugt 1-4 Kohlenstoffatomen bezeichnet, beispielsweise werden genannt: Methyl, Ethyl, Propyl, Butyl, Pentyl, Hexyl, Heptyl, Octyl, Nonyl und Decyl. Sofern nicht anders genannt, sind von den vorstehend genannten Bezeichnungen Propyl, Butyl, Pentyl, Hexyl, Heptyl, Octyl, Nonyl und Decyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl, die Bezeichnung Pentyl, iso-Pentyl, Neopentyl etc.
In den vorstehend genannten Alkylgruppen können gegebenenfalls ein oder mehrere Wasserstoffatome durch andere Reste ersetzt sein. Beispielsweise können diese Alkylgruppen durch die Halogenatome Fluor, Chlor, Brom oder lod substituiert sein. Bevorzugt sind die Substituenten Fluor und Chlor. Besonders bevorzugt ist der Substituent Chlor. Es können gegebenenfalls auch alle Wasserstoffatome der Alkylgruppe ersetzt sein.
Ebenso können in den vorstehend genannten Alkylgruppen, soweit nicht anders beschrieben, gegebenenfalls ein oder mehrere Wasserstoffatome beispielsweise durch einen gegebenenfalls substituierten Rest ausgewählt aus der Gruppe bestehend aus CN, OCOCH₃, Aryl, vorzugsweise Phenyl, Heteroaryl, vorzugsweise Thienyl, Thiazolyl, Imidazolyl, Pyridyl, Pyrimidyl oder Pyrazinyl, gesättigtes oder ungesättigtes Heterocycloalkyl, vorzugsweise Pyrazolyl, Pyrrolidinyl, Piperidinyl, Piperazinyl oder Tetrahydro-oxazinyl, einen Aminrest, vorzugsweise Methylamin, Benzylamin, Phenylamin oder Heteroarylamin, gesättigte oder ungesättigte bicyclische Ringsysteme, vorzugsweise Benzimidazolyl und Cycloalkyl, vorzugsweise Cyclohexyl oder Cyclopropyl, ersetzt sein.

Als Alkylbrücke werden, soweit nicht anders angegeben, verzweigte und unverzweigte Alkylgruppen mit 2 bis 5 Kohlenstoffatomen, beispielsweise Propylen- , Isopropylen-, n-Butylen, iso-Butyl, sec. Butyl und tert.-Butyl etc. Brücken bezeichnet. Besonders bevorzugt sind Propylen- und Butylen-Brücken. In den genannten Alkylbrücken können gegebenenfalls 1 bis 2 C-Atome durch ein oder mehrere Hetaroatome ausgewählt aus der Gruppe Sauerstoff, Stickstoff oder Schwefel ersetzt sein

Als Alkenylgruppen (auch soweit sie Bestandteil anderer Reste sind) werden verzweigte und unverzweigte Alkylengruppen mit 2 bis 10 Kohlenstoffatomen, bevorzugt 2 - 6 Kohlenstoffatomen, besonders bevorzugt 2 - 3 Kohlenstoffatomen betrachtet, soweit sie mindestens eine Doppelbindung aufweisen. Beispielsweise werden genannt: Ethenyl, Propenyl, Butenyl, Pentenyl etc. Sofern nicht anders genannt, sind von den vorstehend genannten Bezeichnungen Propenyl, Butenyl etc. sämtliche der möglichen isomeren Formen umfaßt. Beispielsweise umfaßt die Bezeichnung Butylen n-Butenyl, 1-Methylpropenyl, 2-Methylpropenyl, 1.1-Dimethylethenyl, 1.2-Dimethylethenyl etc.
In den vorstehend genannten Alkenylgruppen können, soweit nicht anders beschrieben, gegebenenfalls ein oder mehrere Wasserstoffatome durch andere Reste ersetzt sein. Beispielsweise können diese Alkylgruppen durch die Halogenatome Fluor, Chlor, Brom oder lod substituiert sein. Bevorzugt sind die Substituenten Fluor und Chlor. Besonders bevorzugt ist der Substituent Chlor. Es können gegebenenfalls auch alle Wasserstoffatome der Alkenylgruppe ersetzt sein.

Als Alkinylgruppen (auch soweit sie Bestandteil anderer Reste sind) werden verzweigte und unverzweigte Alkinylgruppen mit 2 bis 10 Kohlenstoffatomen bezeichnet, soweit sie mindestens eine Dreifachbindung aufweisen, beispielsweise Ethinyl, Propargyl, Butinyl, Pentinyl, Hexinyl etc., vorzugsweise Ethinyl oder Propinyl.

In den vorstehend genannten Alkinylgruppen können, soweit nicht anders beschrieben, gegebenenfalls ein oder mehrere Wasserstoffatome durch andere Reste ersetzt sein. Beispielsweise können diese Alkylgruppen durch die Halogenatome Fluor, Chlor, Brom oder Iod substituiert sein. Bevorzugt sind die Substituenten Fluor und Chlor. Besonders bevorzugt ist der Substituent Chlor. Es können gegebenenfalls auch alle Wasserstoffatome der Alkinylgruppe ersetzt sein.

Der Begriff Aryl steht für ein aromatisches Ringsystem mit 6 bis 14 Kohlenstoffatomen, vorzugsweise 6 oder 10 Kohlenstoffatomen, bevorzugt Phenyl, das, soweit nicht anders beschrieben, beispielsweise einen oder mehrere der nachfolgend genannten Substituenten tragen kann: OH, NO₂, CN, -OCHF₂, -OCF₃, -NH₂, Halogen, beispielsweise Fluor, Chlor, Brom oder lod, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder -O-Ethyl, - N-methyl -tetrahydro-oxazinyl, -COOH, -COO-C₁-C₄-Alkyl, vorzugsweise -COOCH₂CH₃, -COO-C(CH₃)₃ oder -COOCH₃, -CONH₂, -CONH-C₁-C₁₀-Alkyl, wobei dieses Alkyl gegebenenfalls weiterhin substituiert sein kann, gegebenenfalls substituiertes -CONH-C₃-C₆-Cycloalkyl, vorzugsweise gegebenenfalls substituiertes -CONH-Cyclopentyl, gegebenenfalls substituiertes -CONH-Heterocycloalkyl, vorzugsweise piperidinyl, pyrrolidinyl oder piperazinyl, gegebenenfalls substituiertes -CONH-Heteroaryl, vorzugsweise gegebenenfalls substituiertes -CONH-Pyridyl, - gegebenenfalls substituiertes -CONH-Aryl, vorzugsweise gegebenenfalls substituiertes -CONH-Phenyl, -CONMeC₁-C₃-Alkyl, wobei dieses Alkyl gegebenenfalls weiterhin substituiert sein kann, vorzugsweise -CONMeCH₂-Pyridyl, Benzimidazol oder
einen Rest der Formel

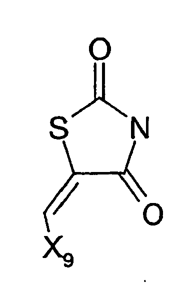

Als 5-10-gliedrige mono- oder bicyclische Heteroarylringe in denen bis zu drei C-Atome durch ein oder meherere Hetaroatome ausgewählt aus der Gruppe Sauerstoff, Stickstoff oder Schwefel ersetzt sein können werden beispielsweise Furan, Thiophen, Pyrrol, Pyrazol, Imidazol, Triazol, Tetrazol, Pyridin, Pyridazin, Pyrimidin, Pyrazin, Triazin, Oxazol, Isoxazol, Thiazol, Thiadiazol, Oxadiazol genannt, wobei jeder der vorstehend genannten Heterocyclen gegeberienfalls ferner an einen Benzolring anneliert sein kann, vorzugsweise benzimidazol, und wobei diese Heterocyclen ,soweit nicht anders beschrieben, beispielsweise einen oder mehrere der nachfolgend genannten Substituenten tragen können: OH, NO₂, CN, -OCHF₂, -OCF₃, -NH₂, Halogen, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder -O-Ethyl, -methyl-N-tetrahydro-oxazinyl, -COOH, -COO-C₁-C₄-Alkyl, vorzugsweise -COO-C(CH₃)₃ oder -COOCH₃, -CONH₂,
gegebenenfalls substituiertes Phenyl, gegebenenfalls substituiertes Heteroaryl, vorzugsweise gegebenenfalls substituiertes Pyridyl oder Pyrazinyl, -CONH-C₁-C₁₀-Alkyl, wobei dieses Alkyl seinerseits gegebenenfalls substituiert sein kann, gegebenenfalls substituiertes -CONH-C₃-C₆-Cycloalkyl, vorzugsweise gegebenenfalls substituiertes -CONH-Cyclopentyl, gegebenenfalls substituiertes -CONH-Heteroaryl, vorzugsweise gegebenenfalls substituiertes -CONH-Pyridyl, - gegebenenfalls substituiertes -CONH-Aryl, vorzugsweise gegebenenfalls substituiertes -CONH-Phenyl, -CONMeC₁-C₃-Alkyl, wobei dieses Alkyl seinerseits gegebenenfalls substituiert sein kann, vorzugsweise -CONMeCH₂-Pyridyl, benzimidazol oder einen Rest der Formel

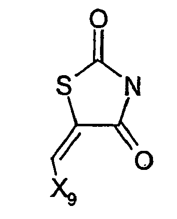

Als Cycloalkylreste werden gesättigte oder ungesättigte Cycloalkylreste mit 3 - 8 Kohlenstoffatomen beispielsweise Cyclopropyl, Cyclobutyl, Cyclopentyl, Cyclopentenyl, Cyclohexyl, Cyclohexenyl, Cycloheptyl oder Cyxclooctyl, vorzugsweise Cyclopropyl, Cyclopentyl oder Cyclohexyl bezeichnet, wobei jeder der vorstehend genannten Cycloalkylreste gegebenenfalls ferner einen oder mehrere Substituenten tragen, vorzugsweise =O, oder an einen Benzolring anneliert sein kann.

"=O" bedeutet ein über eine Doppelbindung verknüpftes Sauerstoffatom.

Als Heterocycloalkylreste werden, soweit in den Definitionen nicht anders beschrieben, 5- ,6- oder 7-gliedrige, gesättigte oder ungesättigte Heterocyclen, die als Heteroatome Stickstoff, Sauerstoff oder Schwefel enthalten können, beispielsweise Tetrahydrofuran, Tetrahydrofuranon, γ-Butylrolacton, α-Pyran, γ-Pyran, Dioxolan, Tetrahydropyran, Dioxan, Dihydrothiophen, Thiolan, Dithiolan, Pyrrolin, Pyrrolidin, Pyrazolin, Pyrazolidin, Imidazolin, Imidazolidin, Tetrazol, Piperidin, Pyridazin, Pyrimidin, Pyrazin, Piperazin, Triazin, Tetrazin, Morpholin, Thiomorpholin, Diazepan, Oxazin, Tetrahydro-oxazinyl, Isothiazol, Pyrazolidin genannt, vorzugsweise Pyrazolyl, Pyrrolidinyl, Piperidinyl, Piperazinyl oder Tetrahydro-oxazinyl, genannt, wobei der Heterocyclus gegebenenfalls substituiert sein kann.

Als Halogen wird im allgemeinen Fluor, Chlor, Brom oder Jod bezeichnet.

Als Abgangsgruppe L wird, gleich oder verschieden, eine Abgangsgruppe wie beispielsweise Chlor, Brom, Iod, Methansulfonyl, Trifluormethansulfonyl oder p-Toluolsulfonyl, vorzugsweise Chlor bezeichnet.

Die erfindungsgemäßen Verbindungen können in Form der einzelnen optischen Isomeren, Mischungen der einzelnen Enantiomeren, Diastereomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren, beispielsweise Chlor- oder Bromwasserstoffsäure, oder organische Säuren, wie beispielsweise Oxal-, Fumar-, Diglycol- oder Methansulfonsäure, vorliegen.

Der Substituent R¹ kann einen Rest ausgewählt aus der Gruppe bestehend aus Wasserstoff, NH₂, XH, vorzugsweise OH, Halogen, vorzugsweise Fluor oder Chlor und einer gegebenenfalls durch ein oder mehrere, vorzugsweise ein, zwei oder drei Halogenatome, vorzugsweise Fluor oder Chlor, substituierten C₁-C₃₋Alkyl-Gruppe, vorzugsweise Methyl oder Ethyl, bedeuten. Insbesondere bevorzugt bedeutet der Substituent R1 Wasserstoff .

Der Substituent R² kann einen Rest ausgewählt aus der Gruppe bestehend aus Wasserstoff, CHO, XH, vorzugsweise OH, -X-C₁-C₂-Alkyl, vorzugsweise -O-CH₃ oder -O-CH₂CH₃, und einer gegebenenfalls substituierten C₁-C₃-Alkyl-Gruppe, wobei die Alkylgruppe vorzugsweise aus 1 bis 2 Kohlenstoffatomen, insbesondere bevorzugt aus einem Kohlenstoffatom besteht und gegebenenfalls substituiert sein kann, bevorzugt durch Halogenatome, insbesondere bevorzugt durch Fluoratome, bedeuten. Insbesondere bevorzugt bedeutet der Substituent R² Methyl.

Die Substituenten R³ und R⁴ können gleich oder verschieden sein und einen Rest ausgewählt aus der Gruppe bestehend aus gegebenenfalls substituiertem C₁-C₁₀₋Alkyl, vorzugsweise C₁-C₆-Alkyl, bevorzugt C₁-C₄-Alkyl, besonders bevorzugt Methyl, Ethyl oder Propyl, insbesondere bevorzugt Methyl oder Ethyl, C₂-C₁₀-Alkenyl, vorzugsweise Ethenyl oder Propenyl, bevorzugt Ethenyl, C₂-C₁₀-Alkinyl, vorzugsweise Ethinyl oder Propinyl, Aryl, bevorzugt gegebenenfalls substituiertes Phenyl, Heteroaryl, C₃-C₈-Cycloalkyl, vorzugsweise Cyclopropyl und Cyclobutyl, C₃-C₈-Heterocycloalkyl, -X-Aryl, -X-Heteroaryl, -X-Cycloalkyl, , -X-Heterocycloalkyl, -NR⁸-Aryl, -NR⁸-Heteroaryl, -NR⁸-Cycloalkyl und -NR⁸-Heterocycloalkyl, oder
ein Rest ausgewählt aus der Gruppe bestehend aus Wasserstoff, Halogen, COXR⁸, CON(R⁸)₂, COR⁸ und XR⁸, vorzugsweise Wasserstoff , bedeuten oder
Die Reste R³ und R⁴ können gemeinsam eine 2- bis 5-gliedrige Alkylbrücke, vorzugsweise eine Ethylen-, Propylen- oder Butylenbrücke, wobei die Propylen- oder Butylenbrücke 1 bis 2 Heteroatome, vorzugsweise Sauerstoff , Stickstoff oder Schwefel enthalten kann, besonders bevorzugt eine Ethylenbrücke bedeuten. Insbesondere bevorzugt bedeutet der Substituent R³ Methyl oder Ethyl. Der Substituent R⁴ bedeutet insbesondere bevorzugt Wasserstoff oder Methyl. Besonders bevorzugt sind Verbindungen, in denen R³ und R⁴ Methyl bedeuten. Alle in der Bedeutung für R³ und R⁴ genannten Reste können gegebenenfalls substituiert sein.

Der Rest R⁵ kann Wasserstoff oder ein Rest ausgewählt aus der Gruppe bestehend aus gegebenenfalls substituiertem C₁-C₁₀-Alkyl, beispielsweise C₁-C₆₋Alkyl-Aryl oder C₁-C₆-Alkyl-Heteroaryl, vorzugsweise C₁-C₆-Alkyl, besonders bevorzugt C₁-C₅-Alkyl, insbesondere bevorzugt Propyl, Butyl, Pentyl, Hexyl, -CH₂₋Cyclohexyl, (CH₂)₁₋₂Cyclopropyl oder (CH₂)₄-OCOCH₃, C₂-C₁₀-Alkenyl, vorzugsweise Propenyl, Butenyl, Pentenyl oder Hexenyl, bevorzugt Propenyl oder Hexenyl,
C₂-C₁₀-Alkinyl, vorzugsweise Propinyl, Butinyl oder Pentinyl, bevorzugt Propinyl, Aryl, vorzugsweise Phenyl, Heteroaryl, -C₃-C₆-Cycloalkyl, vorzugsweise Cyclopropyl, Cyclobutyl, Cyclopentyl oder Cyclohexyl und -C₃-C₆-Cycloalkenyl, vorzugsweise Cyclohexenyl oder Cyclopentenyl, bedeuten, oder die Substituenten R³ und R⁵ oder R⁴ und R⁵ bilden gemeinsam eine gesättigte oder ungesättigte C₃-C₄-Alkylbrücke, die 1 bis 2 Heteroatome, vorzugsweise Sauerstoff, Schwefel oder Stickstoff, enthalten kann,
Alle in der Bedeutung für R⁵ genannten Reste können gegebenenfalls substituiert sein.

Der Substituent R⁶ kann gegebenenfalls substituiertes Aryl, oder Heteroaryl, vorzugsweise Aryl, bevorzugt Phenyl, bedeuten.
Insbesondere bevorzugt bedeutet der Substituent R⁶ einen Phenyl- Rest, der substituiert sein kann durch einen der nachstehend beschriebenen Reste R⁹ und
R10, wobei der Phenylring einen der Reste R⁹, vorzugsweise in para-Stellung, und einen, zwei, drei oder vier, bevorzugt einen oder zwei, der Reste R¹⁰, vorzugsweise in ortho- oder meta-Stellung tragen kann.

Der Substituent R⁷ kann Wasserstoff oder -CO-X-C₁-C₄-Alkyl, vorzugsweise Wasserstoff bedeuten.
X bedeutet jeweils unabhängig voneinander O oder S, vorzugsweise O.

Die in den Definitionen der Substituenten R³ und R⁴ genannten Reste R⁸ bedeuten jeweils unabhängig voneinander, Wasserstoff oder ein Rest ausgewählt aus der Gruppe bestehend aus gegebenenfalls substituiertem C₁-C₄-Alkyl, C₂-C₄₋Alkenyl, C₂-C₄-Alkinyl und Phenyl, vorzugsweise Wasserstoff oder C₁-C₂-Alkyl.

Der Substituent R⁹ kann einen Rest ausgewählt aus der Gruppe bestehend aus gegebenenfalls substituiertem C₁-C₆-Alkyl, vorzugsweise C₁-C₄-Alkyl, bevorzugt Methyl, Ethyl oder Propyl, besonders bevorzugt Methyl, C₂-C₆-Alkenyl, C₂-C₆₋Alkinyl, -CONH-C₁-C₁₀-Alkylen, vorzugsweise -CONH-C₁-C₃-Alkylen, bevorzugt -CONH-C₁-C₂-Alkylen, -O-Aryl, bevorzugt O-C₆-C₁₀-Aryl, besonders bevorzugt O-Phenyl, -O-Heteroaryl, -O-Cycloalkyl, bevorzugt O-C₃-C₆-Cycloalkyl, besonders bevorzugt O-Cyclopropyl, -O-Heterocycloalkyl, Aryl, bevorzugt C₆-C₁₀-Aryl, besonders bevorzugt Phenyl, Heteroaryl, Cycloalkyl, bevorzugt C₃-C₆-Cycloalkyl, besonders bevorzugt Cyclopropyl, und Heterocycloalkyl, oder ein Rest ausgewählt aus der Gruppe bestehend aus -O-C₁-C₆-Alkyl-Q¹, -CONR⁸-C₁-C₁₀-Alkyl-Q¹, -CONR⁸-C₁-C₁₀-Alkenyl-Q¹, -CONR⁸-Q², Halogen, beispielsweise Fluor, Chlor, Brom oder lod, OH, -SO₂R⁸, -SO₂N(R⁸)₂, -COR⁸, -COOR⁸,-N(R⁸)₂, -NHCOR⁸, CONR⁸OC₁-C₁₀-AlkylQ¹'und CONR⁸OQ², bedeuten, wobei Q1 und Q2 die vorstehend genannten Bedeutungen haben. Vorzugsweise bedeutet R⁹ einen der folgenden Reste -CONH-C₁-C₁₀-Alkyl, bevorzugt -CONH-C₁-C₃-Alkyl, besonders bevorzugt -CONH-C₁-C₂-Alkyl, wobei dieses Alkyl gegebenenfalls seinerseits substituiert sein kann, durch CN, gegebenenfalls substituiertes Aryl, vorzugsweise gegebenenfalls substituiertes Phenyl, Heteroaryl, vorzugsweise Thienyl, Thiazolyl, Imidazolyl, Pyridyl, Pyrimidyl oder Pyrazinyl, gesättigtes oder ungesättigtes Heterocycloalkyl, vorzugsweise Pyrazolyl, Pyrrolidinyl, Piperidinyl, Piperazinyl oder Tetrahydro-oxazinyl, einen Aminrest, vorzugsweise Methylamin, Benzylamin, Phenylamin oder Heteroarylamin, gesättigte oder ungesättigte bicyclische Ringsysteme, vorzugsweise Benzimidazolyl und Cycloalkyl, vorzugsweise Cyclohexyl. Weiterhin bedeutet R⁹ vorzugsweise -CONH-Heteroaryl, bevorzugt -CONH-Pyridyl, -CONH-C₃-C₁₀-Cycloalkyl, bevorzugt -CONH-Cyclopropyl -CONH-Cyclobutyl oder -CONH-Cyclopentyl, insbesondere bevorzugt -CONH-Cyctopropyl; -CONH-C₃-C₁₀-Heterocycloalkyl , -CONH-C₆-C₁₀-Aryl, bevorzugt -CONH-Phenyl, COO-C₁-C₃-Alkyl, besonders bevorzugt COOCH₃, COOH, Halogen, bevorzugt F oder Chlor, OH oder ein Rest der Formel

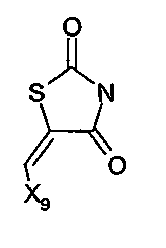

Alle in der Definition von R⁹ aufgeführten Reste können gegebenenfalls substituiert sein, vorzugsweise durch einen oder mehrere der Reste ausgewählt aus der Gruppe bestehend aus OH, OCH₃, Cl, F, CH₃, COOH, CONHCH₂Ph und CONHCH₂-pyrazinyl-CH₃.

Der Substituent R¹⁰ kann jeweils gleich oder verschieden sein und einen Rest ausgewählt aus der Gruppe bestehend aus gegebenenfalls substituiertem C₁-C₆₋Alkyl , vorzugsweise C₁-C₃-Alkyl, C₂-C₆-Alkenyl, vorzugsweise C₂-C₃-Alkenyl und C₂-C₆-Alkinyl, vorzugsweise C₂-C₃-Alkinyl, -O-C₁-C₆-Alkyl, vorzugsweise -O-C₁₋C₃-Alkyl, -O-C₂-C₆-Alkenyl, -O-C₂-C₆-Alkinyl, C₃-C₆-Heterocycloalkyl und C₃-C₆₋Cycloalkyl, oder
ein Rest ausgewählt aus der Gruppe bestehend aus Wasserstoff, -CONH₂, -COOR⁸, -OCON(R⁸)₂, -N(R⁸)₂, -NHCOR⁸, -NHCON(R⁸)₂ , -NO₂ und Halogen, beispielsweise Fluor, Chlor, Brom oder lod, bedeuten.
Vorzugsweise bedeutet der Substituent R¹⁰ Wasserstoff, Methyl, Methoxy, Fluor oder Chlor, besonders bevorzugt Wasserstoff oder Methoxy, insbesondere bevorzugt Methoxy.
Benachbarte Reste R⁹ und R¹⁰ können gemeinsam eine Brücke der allgemeinen Formel 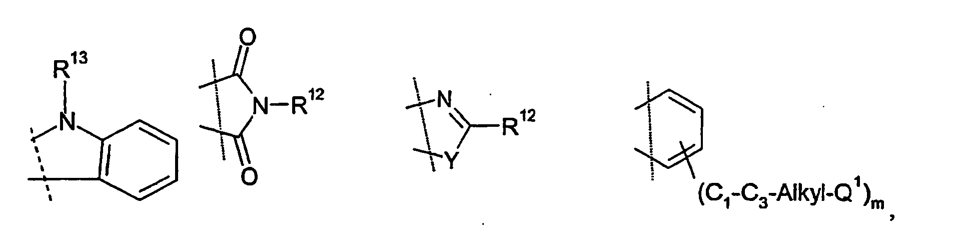 bedeuten, wobei
Y O, S oder NR¹¹, vorzugsweise NR¹¹,
m 0, 1 oder 2, vorzugsweise 1 ,
R¹¹ Wasserstoff oder C₁-C₂-Alkyl, vorzugsweise Wasserstoff oder Methyl, besonders bevorzugt Wasserstoff,
R¹² Wasserstoff oder ein Rest ausgewählt aus der Gruppe bestehend aus gegebenenfalls substituiertem Phenyl, Pyridyl, Pyrazinyl, Pyrimidinyl, Pyridazinyl, -C₁-C₃-Alkyl-Phenyl, -C₁-C₃-Alkyl-Pyridyl, -C₁-C₃-Alkyl-Pyrazinyl, -C₁-C₃-Alkyl-Pyrimidinyl und -C₁-C₃-Alkyl-Pyridazinyl, vorzugsweise Phenyl, Pyridyl und Pyrazinyl, und
R¹³ C₁-C₆-Alkyl, vorzugsweise Methyl oder Ethyl,
bedeuten.

Die Herstellung der erfindungsgemäßen Verbindungen kann nach den im folgenden beschriebenen Syntheseverfahren A und B erfolgen, wobei die Substituenten der allgemeinen Formeln (A1) bis (A6) die zuvor genannten Bedeutungen haben. Diese Verfahren sind als Erläuterung der Erfindung zu verstehen ohne selbige auf deren Gegenstand zu beschränken.

### Verfahren A

### Stufe 1 A

Eine Verbindung der Formel (A1) wird mit einer Verbindung der Formel (A2) zu einer Verbindung der Formel (A3) umgesetzt (Schema 1A). Diese Reaktion kann gemäß WO 0043369 oder WO 0043372 durchgeführt werden. Verbindung (A1) ist kommerziell, beispielsweise bei City Chemical LLC, 139 Allings Crossing Road, West Haven, CT, 06516, USA, erhältlich. Verbindung (A2) kann nach literaturbekannten Vorschriften (a) F. Effenberger, U. Burkhart, J. Willfahrt *Liebigs Ann. Chem.* **1986**, 314-333; b) T. Fukuyama, C.-K. Jow, M. Cheung, *Tetrahedron Lett*. **1995,** *36*, 6373-6374; c) R. K. Olsen, *J. Org. Chem*. **1970,** *35*, 1912-1915; d) F.E. Dutton, B.H. Byung *Tetrahedron Lett.* **1998**, *30,* 5313-5316; e) J. M. Ranajuhi, M. M. Joullie *Synth. Commun.* **1996,** *26*, 1379-1384.) hergestellt werden.

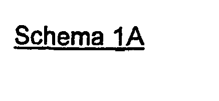

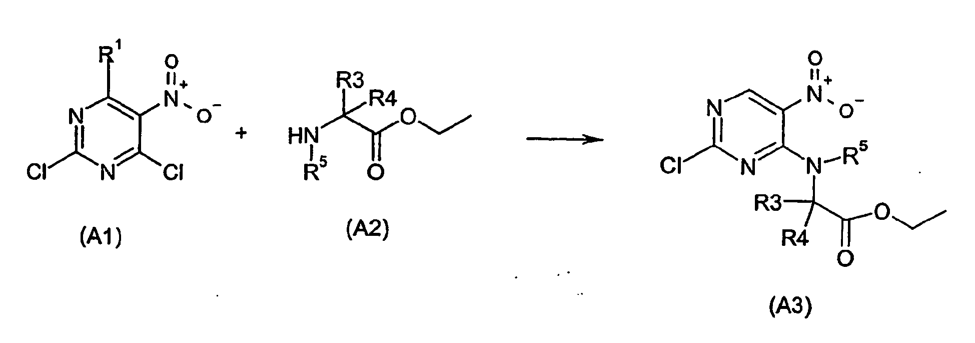

In Stufe 1 A werden 1 Äquivalent der Verbindung (A1) und 1 bis 1,5 Äquivalente" bevorzugt 1,1 Äquivalente einer Base, vorzugsweise Kaliumcarbonat, Kaliumhydrogencarbonat, Natriumcarbonat oder Natriumhydrogencarbonat, Calciumcarbonat, besonders bevorzugt Kaliumcarbonat, in einem Verdünnungsmittel, beispielsweise Aceton, wässriges Aceton, Tetrahydrofuran, Diethylether oder Dioxan, vorzugsweise Aceton oder Diethylether, besonders bevorzugt Aceton, gerührt.
Bei einer Temperatur von 0 bis 15 °C, bevorzugt 5 bis 10 °C, wird 1 Äquivalent einer Aminosäure der Formel (A2) gelöst in einem organischen Lösungsmittel, beispielsweise Aceton, Tetrahydrofuran, Diethylether oder Dioxan, bevorzugt Aceton, zugetropft. Das Reaktionsgemisch wird unter Rühren auf eine Temperatur von 18°C bis 30 °C, vorzugsweise etwa 22°C, erwärmt und anschließend weitere 10 bis 24 Stunden, vorzugsweise etwa 12 Stunden, weitergerührt. Danach wird das Verdünnungsmittel abdestilliert, der Rückstand mit Wasser versetzt und das Gemisch zwei bis dreimal eines organischen Lösungsmittels beispielsweise, Diethylether oder Ethylacetat, vorzugsweise Ethylacetat extrahiert. Die vereinigten organischen Extrakte werden getrocknet und das Lösungsmittel abdestilliert. Der Rückstand (Verbindung A3) kann ohne vorherige Aufreinigung in Stufe 2 eingesetzt werden.

### Stufe 2A

Die in Stufe 1A erhaltene Verbindung (A3) wird an der Nitrogruppe reduziert und zur Verbindung der Formel (A4) zyklisiert (Schema 2A).

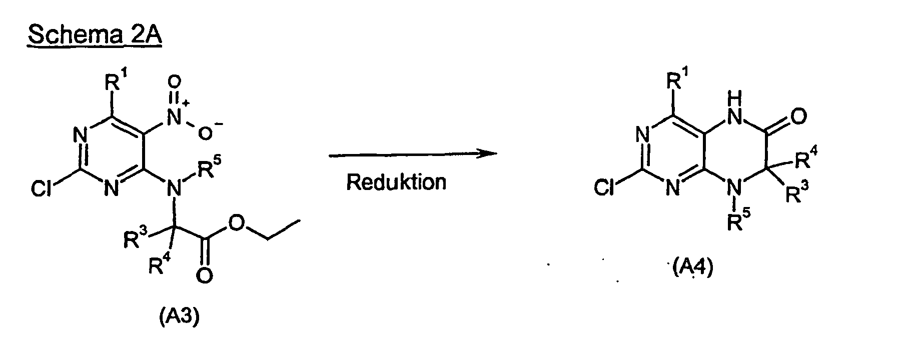

In Stufe 2A werden 1 Äquivalent der Nitroverbindung (A3) in einer Säure gelöst, vorzugsweise Eisessig, Ameisensäure oder Salzsäure, bevorzugt Eisessig, gelöst und auf 50 bis 70 °C, vorzugsweise etwa 60 °C, erwärmt. Anschließend wird ein Reduktionsmittel, beispielsweise Zink, Zinn, oder Eisen, bevorzugt Eisenpulver, bis zum Abschluß der exothermen Reaktion hinzugefügt und 0,2 bis 2 Stunden, vorzugsweise 0,5 Stunden, bei 100 bis 125 °C, vorzugsweise bei etwa 117 °C, gerührt. Nach Abkühlung auf Raumtemperatur wird das Eisensalz abfiltriert und das Lösungsmittel abdestilliert. Der Rückstand wird in einem Lösungsmittel oder Lösungsmittelgemisch, beispielsweise Ethylacetat oder Dichlormethan/ Methanol 9/1 und halbgesättigte NaCl-Lösung, aufgenommen und beispielsweise über Kieselgur Kieselgur filtriert. Die organische Phase wird getrocknet und eingeengt. Der Rückstand (Verbindung (A4)) kann chromatographisch oder mittels Kristallisation gereinigt oder als Rohprodukt in Stufe 3A der Synthese eingesetzt werden.

### Stufe 3A

Die in Stufe 2A erhaltene Verbindung (A4) kann durch elektrophile Substitution gemäß Schema 3A zur Verbindung der Formel (A5) umgesetzt werden.

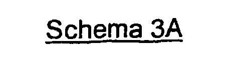

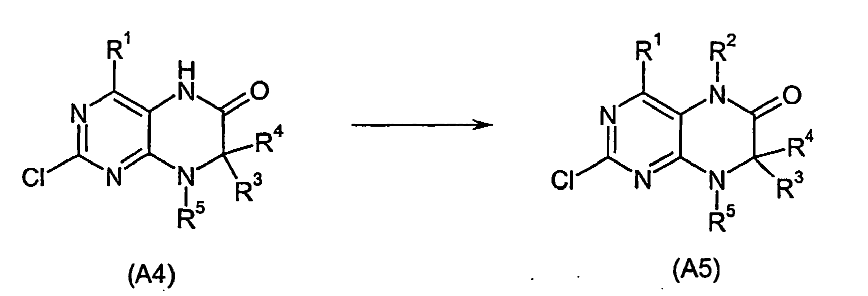

In Stufe 3A werden 1 Äquivalent des Amides der Formel (A4) in einem organischen Lösungsmittels, beispielsweise Dimethylformamid oder Dimethylacetamid, vorzugsweise Dimethylacetamid, gelöst und auf etwa -5 bis 5 °C, vorzugsweise 0°C, abgekühlt.
Anschließend wird 0,9 bis 1,3 Äquivalente Natriumhydrid und 0,9 bis 1,3 Äquivalente Äquivalent Alkylhalogenid, beispielsweise Methyliodid zugegeben. Das Reaktionsgemisch wird 0,1 -3 Stunden, vorzugsweise etwa 1 Stunde, bei etwa 0 bis 10 °C, vorzugsweise bei etwa 5 °C, gerührt und kann gegebenenfalls weitere 12 Stunden bei diesem Temperaturbereich stehengelassen werden. Die Reaktionsmischung wird eingeengt und mit Wasser und einem organischen Lösungsmittel, vorzugsweise Dichlormethan, Ethylacetat, extrahiert. Die organischen Phasen werden eingeengt. Der Rückstand (Verbindung (A5)) kann chromatographisch, vorzugsweise über Kieselgel, gereinigt werden.

### Stufe 4A

Die Aminierung der in Stufe 3A erhaltenen Verbindung (A5) zur Verbindung der Formel (A7) (Schema 4A) kann nach den literaturbekannten Methoden der Varianten 4.1 A (a) M.P.V. Boarland, J.F.W. McOmie J. *Chem. Soc.* **1951,** 1218-1221; b) F. H. S. Curd, F. C. Rose J. *Chem. Soc.* **1946,** 343-348., 4.2 A (a) Banks *J. Am. Chem. Soc.* **1944,** *66*, 1131 b) Ghosh and Dolly *J*. *Indian Chem. Soc.* **1981**, *58*, 512-513 durchgeführt werden.

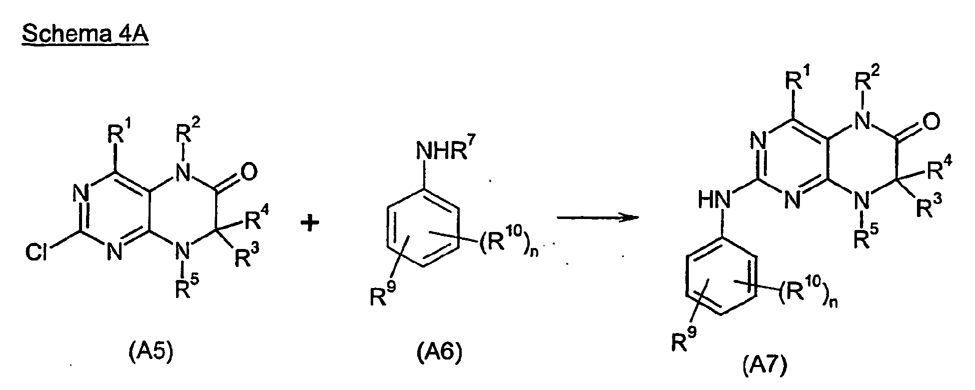

Beispielsweise werden in Variante 4.1 A 1 Äquivalent der Verbindung (A5) und 1 bis 3 Äquivalente, vorzugsweise etwa 2 Äquivalent der Verbindung (A6) ohne Lösungsmittel oder einem organischen Lösungsmittel wie beispielsweise Sulfolan, Dimethylformamid, Dimethylacetamid, Toluol, N-Methylpyrrolidon, Dimethylsulfoxid, oder Dioxan, bevorzugt Sulfolan über 0,1 bis 4 Stunden, vorzugsweise 1 Stunde, bei 100 bis 220 °C, vorzugsweise bei etwa 160 °C erhitzt. Nach dem Abkühlen wird durch Zugabe von org. Lösungsmitteln oder Lösungsmittelgemischen bespielsweise Diethylether/Methanol, Ethylacetat, Methylenchlorid, oder Diethylether, vorzugsweise Diethylether/Methanol 9/1, das Produkt (A7) kristallisiert oder chromatographisch gereinigt.

Beispielsweise wird in Variante 4.2 A 1 Äquivalent der Verbindung (A5) und 1 bis 3 Äquivalente der Verbindung (A6) mit Säure, beispielsweise 1-10 Äquivalente 10-38%ige Salzsäure und/oder einem Alkohol beispielsweise Ethanol, Propanol, Butanol, bevorzugt Ethanol unter Rückfluß 1 bis 48 Stunden, vorzugsweise etwa 5 Stunden, gerührt.
Das ausgefallene Produkt (A7) wird abfiltriert und gegebenenfalls mit Wasser gewaschen, getrocknet und aus einem geeigneten org. Lösungsmittel kristallisiert.

Im Falle, daß R⁶ die Bedeutung eines gegebenenfalls substituierten Benzimidazols hat, kann die Herstellung der Verbindungen (A6) nach literaturbekannten Methoden beispielsweise entsprechend folgendem Schema erfolgen:

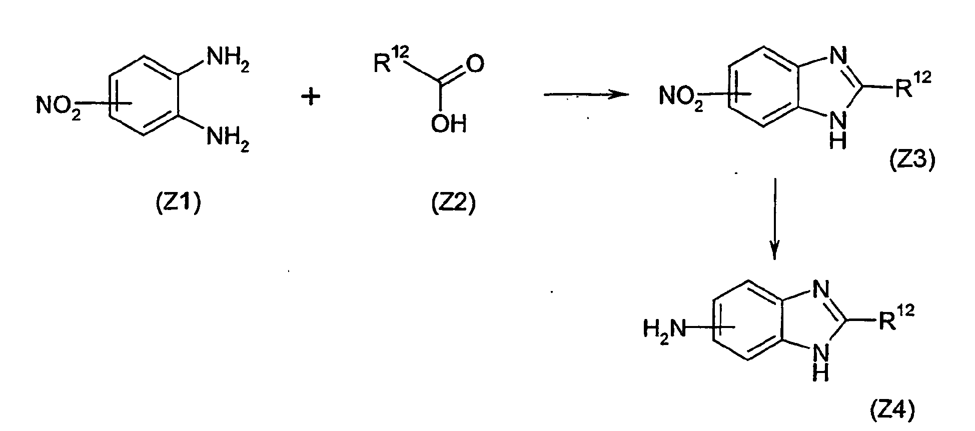

Demgemäß werden beispielsweise, 33 mmol, der Verbindung (Z1), 49 mmol, der Verbindung (Z2) und 49 mmol, 1-Ethoxycarbonyl-2-ethoxydihydroquinolin (EEDQ) in 50 ml, eines organischen Lösungsmittels, vorzugsweise Dimethylformamid, bei etwa 100 bis 130 °C, vorzugsweise bei etwa 115 °C, 1 bis 4 Stunden, vorzugsweise etwa 3 Stunden, gerührt. Anschließend wird die abgekühlte Reaktionslösung auf 50 bis 400 ml, vorzugsweise etwa 200 ml einer Wasser/Ethylacetat Mischung (Mischungsverhältnis etwa 1:1) gegeben. Die entstandenen Kristalle (Z3) werden abgesaugt und gewaschen. Anschließend werden, 4,2 mmol, der Verbindung (Z3), mit 12,5 mmol, Zinn(II)chlorid und 30 mmol Kaliumcarbonat in etwa 50 ml, eines organischen Verdünnungsmittels, vorzugsweise Ethylacetat bei etwa 22 °C, 4 bis 48 Stunden, vorzugsweise etwa 24 Stunden, gerührt. Nach Zugabe von 22 g Kieselgur wird mit einem organischen Verdünnungsmittel oder Verdünnungsmittelgemisch, vorzugsweise mit einem Gemisch Dichlormethan / Methanol (9:1) extrahiert, die vereinigten Extrakte eingeengt und der entstandene Niederschlag (Z4) oder entstandene Kristalle (Z4), isoliert.

### Stufe 5A

Im Falle, daß R⁹ die Bedeutung, -CONR⁸-C₁-C₁₀-Alkyl-Q¹, -CONH-C₁-C₅-Alkylen oder -CONR⁸-Q² besitzt, wobei die Substituenten die zuvor genannten Bedeutungen haben, kann die Herstellung der erfindungsgemäßen Verbindungen nach literaturbekannten Methoden beispielsweise entsprechend Schema 5A erfolgen.
Die in Stufe 4A erhaltene Verbindung (A7') kann entweder durch Verseifung und anschließende Aminierung zum Amid der allgemeinen Formel (A10) (Schema (5A) Variante 5.1A, oder durch Verseifung, mit anschließender Überführung in das Säurechlorid (A9) und nachfolgende Aminierung (Schema (5A) Variante 5.2A, umgesetzt werden.

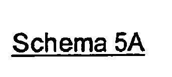

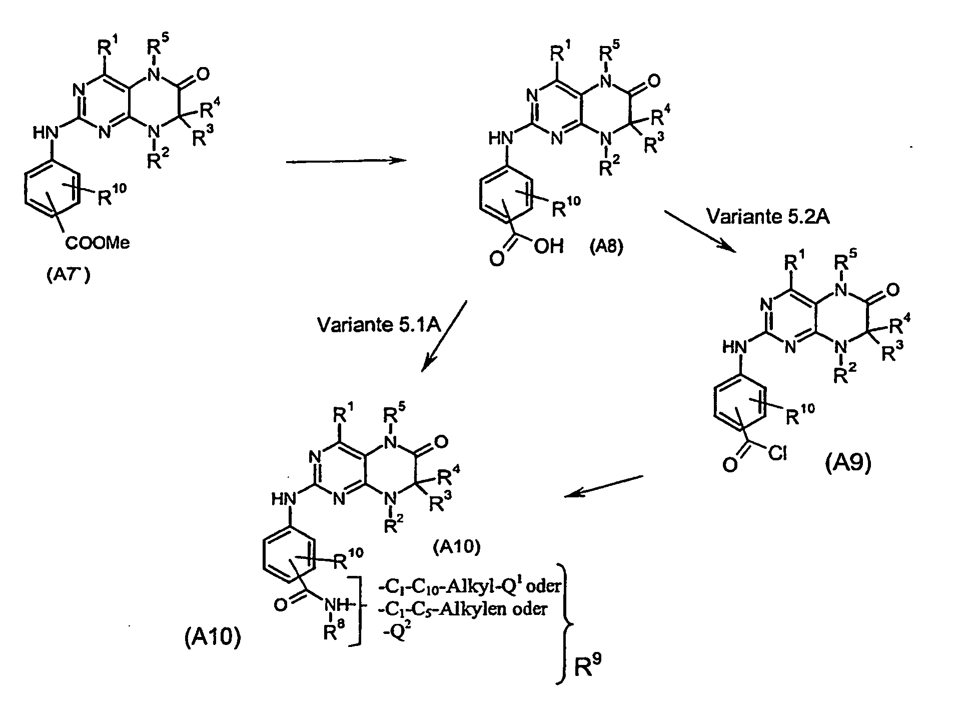

### Variante 5.1 A:

In Variante 5.1 A werden beispielsweise 20 mmol des Esters (A7') in etwa 100ml einer Base, vorzugsweise 1 N Natriumhydroxidlösung oder Lithiumhydroxidlösung und etwa 500 ml eines Alkohols, beispielsweise mit Ethanol, Dioxan oder Methanol, vorzugsweise Methanol, bis zur vollständigen Umsetzung des Esters erhitzt. Anschließend wird der Alkohol abdestilliert. Der Rückstand wird in etwa 200 ml Wasser aufgenommen und unter Kühlung mit Säure beispielsweise Salzsäure, vorzugsweise mit 2 N Salzsäure, angesäuert. Das Produkt (A8) wird abfiltriert und getrocknet.
Bespielsweise werden etwa 0,5 mmol der Verbindung (A8) mit etwa 0,5 mmol O-Benzotriazolyl-N,N,N',N'-tetramethyluroniumtetrafluoroborat(TBTU) und etwa 1,4 mmol, Düsopropylethylamin (DIPEA) in etwa 5 ml eines organischen Verdünnungsmittels, beispielsweise Tetrahydrofuran, Dimethylformamid, N-Methylpyrrolidion, Dimethylacetamid, vorzugsweise Dimethylformamid, gelöst. Nach Zugabe von etwa 0,75 mmol, eines den Substituenten R⁹ bildenden Amins wird das Reaktionsgemisch 0,1 bis 24 Stunden, vorzugsweise etwa 12 Stunden bei 20°C bis 100°C gerührt. Über beispielsweise Kristallisation oderchromatographische Reinigung wird das Produkt der Formel (A10) erhalten.

### Variante 5.2 A:

In Variante 5.2 A werden beispielsweise etwa 1 mmol der Säure (A8) in etwa 2,7 ml, Thionylchlorid suspendiert. Das Gemisch wird auf 40°C bis 80 °C, vorzugsweise etwa 50 °C erwärmt und bei konstanter Temperatur dem Reaktionsgemisch unter Rühren 2 bis 10 Tropfen, vorzugsweise etwa 3 Tropfen, Dimethylformamid zugefügt. Anschließend wird bis zum Reaktionsende bei 90°C, gerührt. Überschüssiges Thionylchlorid wird abdestilliert.
Etwa 1 mmol des entstandenen Säurechlorids (A9) werden in etwa 30 ml eines organischen Verdünnungsmittels, beispielsweise Dichlormethan, gelöst. Nach Zugabe eines den Substituenten R⁹ bildenden Amins wird bei etwa 22°C, gerührt. Der entstandene Niederschlag wird abfiltriert und mit Wasser gewaschen. Der verbleibende Rückstand wird mit einem organischen Verdünnungsmittel, beispielsweise Methanol, gewaschen. Die Mutterlauge wird, beispielsweise chromatographisch, gereinigt und eingeengt. Es verbleibt das Produkt (A10).

### Verfahren B

Alternativ zu oben beschriebenen Verfahren kann wie in Schema B dargestellt nach literaturbekannten Methoden im Anschluß an Stufe 1A zunächst eine Aminierung der Verbindung (A3) und eine anschließende Zyklisierung des Produktes (B1) zur Verbindung (B2) erfolgen. Die weitere Substitution der Verbindung (B2) zur Verbindung (A7) kann beispielsweise analog Stufe 3A erfolgen.

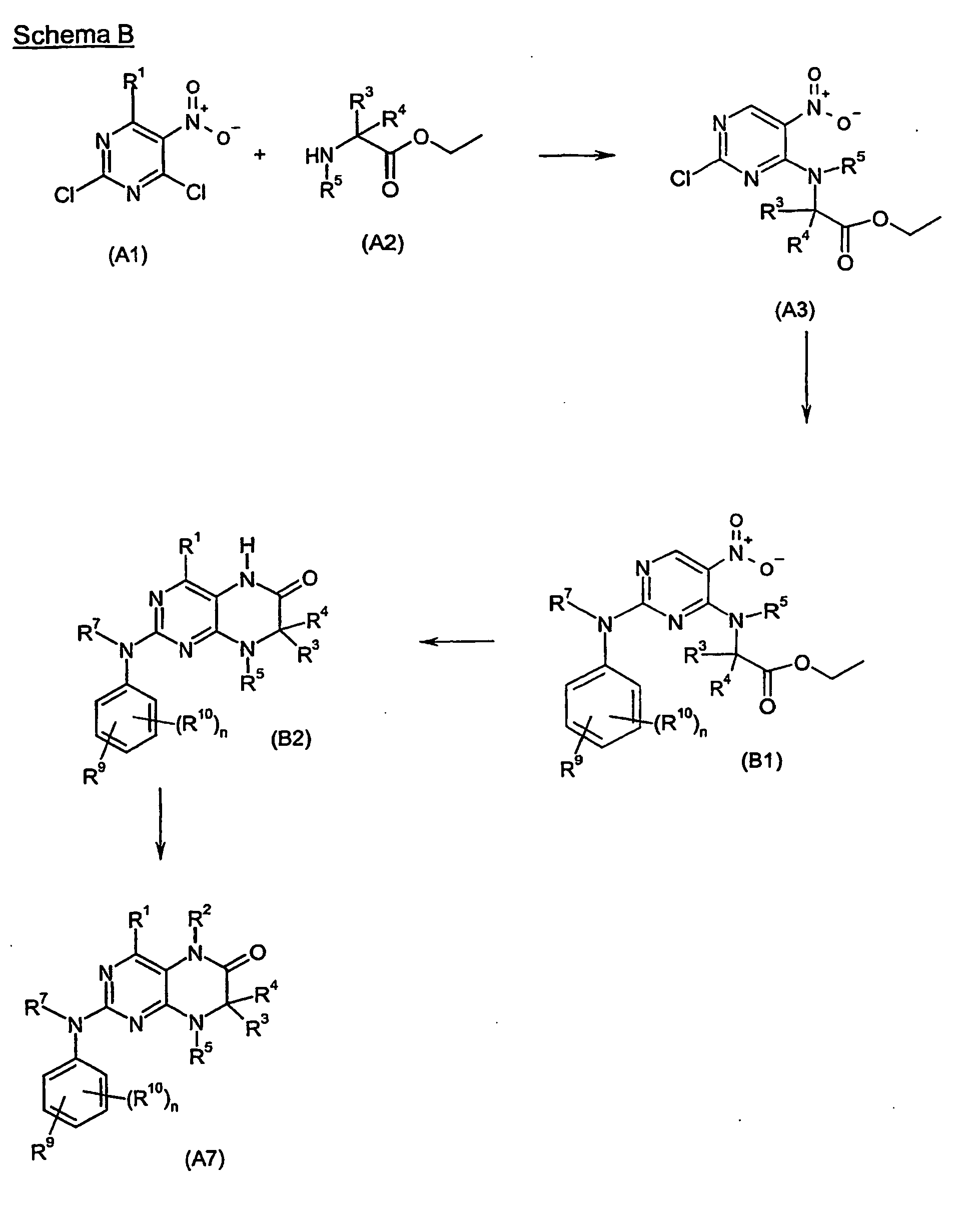

Die neuen Verbindungen der allgemeinen Formel (I) können in Analogie zu nachfolgenden Synthesebeispielen synthetisiert werden. Diese Beispiele sind allerdings nur als exemplarische Vorgehensweise zur weitergehenden Erläuterung der Erfindung zu verstehen, ohne selbige auf dessen Gegenstand zu beschränken.

### Beispiel 63 und Beispiel 109 :

Zur Synthese der Verbindungen 63 und 109 wird zunächst eine Zwischenverbindung 4 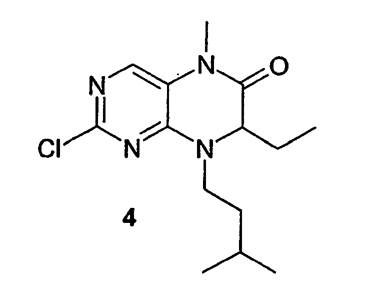 wie im folgenden beschrieben hergestellt.

38,9 ml (0,263 mol) 2-Brombuttersäureethylester und 36,4 g (0,263 mol) Kaliumcarbonat wurden in 350 ml Ethylacetat vorgelegt, und anschließend 46,7 ml (0,402 mol) Isoamylamin, in 70 ml Ethylacetat gelöst, schnell zugetropft. Es wurde 20 h unter Rückfluß gekocht. Entstandenes Salz wurde abfiltriert, das Filtrat eingedampft, mit 50 ml Toluol versetzt und erneut zur Trockene eingedampft. Ausbeute: 54,3 g einer Verbindung 1 (rotes Öl)

54,3 g der Verbindung 1, gelöst in 400 ml Aceton und 30,7 g (0,222 mol) Kaliumcarbonat wurden unter Rühren auf 8° C gekühlt, mit einer Lösung von 43,1 g (0,222 mol) 2,4-Dichlor-5-nitropyrimidin in 250 ml Aceton versetzt und anschließend 24 h bei RT gerührt.

Die entstandene Suspension wurde eingedampft, der Rückstand mit Wasser und Ethylacetat extrahiert, die organische Phase mit Wasser und NaCl-Lösung gewaschen, über MgSO₄ getrocknet und zur Trockne eingedampft. Ausbeute: 87,3 g einer Verbindung 2 (braunes Öl)

44,1 g der Verbindung 2 wurden in 800 ml Eisessig gelöst, auf 65° C erwärmt und mit 36 g Eisenpulver portionsweise versetzt. Danach wurde 3 h bei 70° C gerührt, der Niederschlag abfiltriert und das Filtrat eingedampft.
Der Rückstand wurde in Dichlormethan / Methanol 90:10 auf Kieselgel aufgezogen, eingedampft und mittels Säulenchromatographie (Laufmittel:
Ethylacetat / Cyclohexan 1:1) gereinigt.
Der Rückstand wurde aus Ethylacetat / Petrolether gefällt.
Ausbeute: 16,1 g einer Verbindung 3 (beiges Pulver)

16,1 g der Verbindung **3** wurden in 75 ml Dimethylacetamid gelöst und unter Stickstoff-Atmosphäre unter Rühren auf 5° C gekühlt. Dann wurde 2,51 g (0,063 mol) NaH, 60%ige Dispersion in Mineralöl, zugegeben, wobei die Temperatur vorübergehend auf 16° C anstieg. Nach 30 Min. wurde 3,94 ml (0,063 mol) Methyljodid, gelöst in 75 ml Dimethylacetamid zugegeben, und 24 h bei 22°C gerührt.
Das Lösungsmittel wurde eingedampft, mit 200 ml Wasser versetzt und der entstandene Niederschlag abgesaugt, anschließend mit Petrolether ausgerührt. Ausbeute: 15,1 g einer Verbindung 4 (gelbes Pulver)
¹H-NMR (250 MHz): = 7.80 (1 H, s), 4.35 (m, 1 H), 3.92 (m, 1 H), 3.22 (s, 3H), 3,14 (m, 1H), 1,81 (m, 2H), 1,60-1,40 (m, 3H), 0,90 (m, 6H), 0,70 (t, 3H).

### Synthese von Beispiel 63

2,5 g der Verbindung **4,** 1,43 g 4-Amino-3-methoxybenzoesäure, 1,25 mL konz. Salzsäure, 150 mL dest. Wasser und 37,5 mL Ethanol wurde 10 h unter Rückfluß gekocht. Der Niederschlag wurde abfiltriert, mit Wasser gewaschen und in Methanol ausgerührt. Anschließend wurde der Niederschlag mit Hilfe von Petrolether und Ether umkristallisiert.
Ausbeute: 1,6 g einer Verbindung 5 (weißes Pulver)

0,2 g der Verbindung 5, 5 mL Benzylamin, 0,16 g TBTU, 0,17 g DIPEA wurden in 2 ml Dimethylformamid (DMF) gelöst und 48 h bei Raumtemperatur gerührt.
Anschließend wurde die Reaktionsmischung in Methylenchlorid aufgenommen, mit Wasser gewaschen und die organische Phase eingeengt. Bei der Zugabe von Petrolether/Ethylacetat 9:1 fällt das Produkt als hellbeige Kristalle aus.
Ausbeute: 0.18 g. Smp.: 178°C

### Synthese von Beispiel 109 :

5 g 2 Amino-5-nitroanilin, 6,03 g 4-Pyridylcarbonsäure, 12,1 g EEDQ werden in 50 mL DMF gelöst und bei 115°C 1,75 h gerührt, dann wird im Vakuum das DMF abdestilliert und die Reaktionsmischung anschließend bei 180°C 1 h erhitzt. Der Rückstand wird in 30 mL DMF aufgenommen, mit 200 mL Wasser und 100 mL Ethylacetat versetzt. Der entstandene Kristallbrei wird abfiltriert und mit Wasser, Ethylacetat und Ether gewaschen.
Ausbeute: 5.8 g einer Verbindung 6

2g der Verbindung 6 wird mit 0,2 g Pd/C 5 %ig in 30 mL Ethanol versetzt und in Gegenwart von Wasserstoff hydriert. Anschließend wird eingeengt und aus Ethanol und Toluol kristallisiert.
Ausbeute: 1.75 g weißes Pulver einer Verbindung 7.

0.2 g der Verbindung 5, 0,28 g der Verbindung 7, 0,001 g Natrium-tert.butylat, 2.5 mL Ethylenglycoldimethylether, 0,006 g Palladium(II) acetat und 0,22 g 2-(Di-tert.-butylphospino)biphenyl werden in 1,5 mL N-Methylpyrrolidon (NMP) gelöst. Dann wird für 0,5 h auf 160°C erhitzt. Die Reaktionsmischung wird anschließend über 20 g Kieselgel gereinigt und das Produkt aus Ether, Ethylacetat und Petrolether kristallisiert.
Ausbeute: 0,04 g gelbe Kristalle. Smp.: 180°C

### Beispiel 218 , 58 und 4:

Zur Synthese der Verbindungen 218, 58 und 4 wird zunächst eine Zwischenverbindung **11** 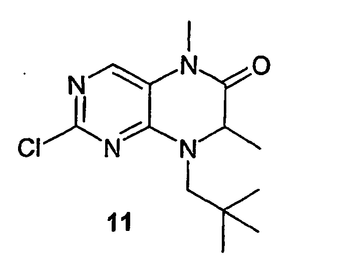 wie im folgenden beschrieben hergestellt.

55,8 g DL-Alaninmethylester x HCl wurden in 500 ml Methanol gelöst, dann wurde 76,1 ml 30%ige Natriummethylat-Lösung zugegeben und das Salz abfiltriert. Dem Filtrat wurden 37,8 g Trimethylacetaldehyd zugesetzt, dann 22 h stehen gelassen. Danach erfolgte die Zugabe von 9,5 g 10%igem Pd/C, es wurde 3,1 h bei 0,5 bar und 20° C hydriert. Das Reaktionsgemisch wurde über Kieselgur abgesaugt und eingedampft. Der Rückstand wurde in Diethylether aufgenommen, die Salze über Kieselgur filtriert und das Filtrat eingedampft.
Ausbeute: 55,8 g einer Verbindung 8 (klare Flüssigkeit)

48,5 g 2,4-Dichlor-5-nitropyrimidin wurden in 400 ml Diethylether vorgelegt, 41,0 g Kaliumhydrogencarbonat in 400 ml Wasser zugegeben, auf -5° C gekühlt. 43,3 g der Verbindung 8 wurden in 400 ml Diethylether gelöst und bei -5° C zugetropft.
Es wurde 1 h bei -5° C und 2 h bei 0° C gerührt, anschließend auf Raumtemperatur erwärmt und das Reaktionsgemisch 24 h stehen gelassen.
Die organische Phase wurde abgetrennt, über MgSO₄ getrocknet und zur Trockene eingedampft.

Ausbeute: 79,2 g einer Verbindung 9 (gelbes Harz)

79,0 g der Verbindung 9 wurden in 1000 ml Eisessig gelöst und auf 70° C erwärmt. Nach Entfernen der Heizquelle wurden 52 g Eisen portionsweise zugegeben. Die Temperatur stieg auf etwa 110° C an, es wurde 1 h bei dieser Temperatur gerührt. Die Suspension wurde heiß filtriert und das Filtrat eingedampft.
Der Rückstand wurde in Ethylacetat aufgenommen und mit 150 ml konz. HCl versetzt, die organische Phase abgetrennt und die wässrige Phase mehrmals mit Dichlormethan extrahiert. Die vereinten organischen Phasen wurden eingedampft, auf Kieselgel aufgezogen und mittels Säulenchromatographie (Laufmittel: Petrolether/Ethylacetat 1:1) gereinigt.
Da die isolierte Substanz noch stark verunreinigt war, wurde nochmals über Kieselgel gereinigt. Die gewünschte Verbindung kristallisierte aus, die Kristalle wurden abgesaugt. Die Mutterlauge wurde eingedampft und aus Ethylacetat / Diethylether umkristallisiert.
Ausbeute: 17,63 g einer Verbindung 10

7,6 g der Verbindung 10 und 6,4 ml Methyljodid wurden in 75 ml Dimethylacetamid (DMA) vorgelegt und auf -15° C gekühlt. 1,25 g NaH, 60%ige Dispersion in Mineralöl, wurde portionsweise zugegeben, 30 min. bei -10° bis -5° C gerührt. Anschließend wurden 150 ml Eiswasser zugesetzt, Kristalle abgesaugt, mit Wasser und Petrolether gewaschen. Die Kristalle wurden in Dichlormethan aufgenommen, über Kieselgur filtriert und das Filtrat zur Trockene eingedampft. Es wurde aus Petrolether umkristallisiert.
Ausbeute: 6,3 g der Verbindung 11 (beige Kristalle)
¹H-NMR (250 MHz): = 7,73 (1 H, s), 4,35 (d, 1 H), 4,25 (m, 1 H), 3,35 (s, 3H), 2,55 (d, 1H), 1,31 (d, 3H), 0,95 (s, 9H).

### Synthese von Beispiel 218

0,2 g der Verbindung 11, 3,5-Difluor-4-hydroxyanilin und 0,75 mL Sulfolan wurden 15 min auf 130°C, 15 min auf 140°C und 10 min auf 170°C erhitzt. Anschließend wurde mit Ether versetzt, die überstehende Lösung abdekantiert und der Rückstand aus Methanol/Ether kristallisiert und erneut aus Methanol umkristallisiert.
Ausbeute: 0,15 g weiße Kristalle. Smp.: >250°C

### Synthese von Beispiel 4

6,3 g der Verbindung 11 werden in 25 mL Sulfolan bei 100°C gelöst, dann mit 4,0 g 4-Aminobenzoesäureethylester versetzt und 1 h auf 170°C erhitzt. Anschließend wurde der Ansatz mit 50 mL Ether versetzt. Nach einsetzender Kristallisation wurden weitere 50 mL Ether und 50 mL Methanol zugegeben. Die Kristalle wurden aus Methanol umkristallisiert.
Ausbeute: 6,6 g einer Verbindung 12 (gelbliche Kristalle), Smp: ab 65°C tritt Zersetzung ein

3,55 g der Verbindung 12 wurden in 250 mL Methanol suspendiert und bei 60°C mit 25 mL 4N Natronlauge versetzt. Nach 6 h wurde 15 mL Eisessig zugegeben, die entstandenen Kristalle abfiltriert und mit Methanol/Ether gewaschen.
Ausbeute: 1,2 g einer Verbindung 13 (weiße Kristalle)

1,5 g der Verbindung 13 wurden in 7,5 mL Thionylchlorid gelöst und für 1 h auf 80°C erwärmt. Dann wurde vom Thionylchlorid abdestilliert, der Rückstand mit Ether verrührt, die Kristalle abgesaugt und mit Ether gewaschen.
Ausbeute: 1,7 g einer Verbindung 14 (gelbe Kristalle)

0,18 g 3-Aminopyridin wurden in 10 mL Tetrahydrofuran(THF) gelöst und mit 0,4 mL Triethylamin versetzt. Anschließend wurde 0,22 g der Verbindung 14 zugegeben und für 16h bei Raumtemperatur gerührt. Der Ansatz wurde zur Trockne eingedampft, in Ethylacetat aufgenommen, mit Wasser extrahiert, erneut eingedampft und das Produkt aus Ethylacetat kristallisiert.
Ausbeute: 0,07 g (beige Kristalle), Smp.: 215-216°C

### Synthese von Beispiel 58

0,05 g der Verbindung **13** wurde in 10 mL Dichlormethan suspendiert, dann mit 0,15 mL DIPEA und 0,05 g TBTU versetzt. Anschließend wurde die Lösung für 30 min gerührt und mit 0,01 mL 4-Picolylamin versetzt. Nach 18 h wurde der Ansatz mit 20 mL Wasser versetzt, die organische Phase abgetrennt und das Produkt mittels Kieselgelchromatographie gereinigt, anschließend aus Ethylacetat /Petrolether umkristallisiert.
Ausbeute: 0,044 g (weiße Kristalle), Smp.: 238-240°C

### Beispiel 65 und 125

Zur Synthese der Verbindungen 65. und125 wird zunächst eine Zwischenverbindung **18** 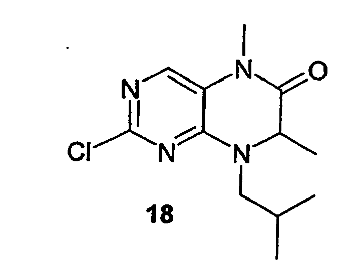 wie im folgenden beschrieben hergestellt.

28,3 g Isobutylamin, 36 g R,S-2-Brompropionsäureethylester und 28 g Kaliumcarbonat wurden in 150 ml Ethylacetat 6 h unter Rückfluß gekocht. Nach Abkühlen wurde das Salz abgesaugt, die Mutterlauge eingedampft. Der Rückstand wurde mit 100 ml Toluol versetzt und zur Trockene eingedampft. Ausbeute: 37,2 g einer Verbindung 15 (gelbes Öl)

38,4 g 2,4-Dichlor-5-nitropyrimidin wurden in 300 ml Diethylether vorgelegt, 30 g Kaliumhydrogencarbonat in 300 ml Wasser zugegeben und auf 0° C gekühlt. 37,0 g der Verbindung 15 wurden in 300 ml Diethylether gelöst und bei 0°-3° C zugetropft. Nach 3 h wurden die Phasen getrennt, die organische Phase getrocknet und zur Trockene eingedampft.
Ausbeute: 71,6 g einer Verbindung **16**

40,0 g der Verbindung **16** wurden in 300 ml Eisessig gelöst und auf 70° C erwärmt. Nach Entfemung der Heizquelle wurden 30 g Eisen portionsweise zugegeben. Die Temperatur stieg auf 110° C an. Das Reaktionsgemisch wurde auf 90° C abgekühlt und 20 min. bei dieser Temperatur gerührt. Anschließend wurde heiß filtriert und das Filtrat eingedampft. Der Rückstand wurde mit 300 ml Wasser und 300 ml Dichlormethan verrührt und über Kieselgur filtriert. Die Phasen wurden getrennt. Die organische Phase wurde mit Wasser gewaschen, über MgSO₄ getrocknet und zur Trockene eingedampft. Es wurde aus Petrolether ausgerührt.
Ausbeute: 26,7 g einer Verbindung **17**

15,0 g der Verbindung **17** wurden in 100 ml DMA vorgelegt, 4,13 ml Methyljodid zugegeben und auf 5° C gekühlt. 2,60 g NaH wurden als 60%ige Dispersion in Mineralöl portionsweise zugegeben. Die Temperatur stieg auf 13° C.
Nach 30 min. wurden 300 ml Eiswasser zugegeben, die ausgefallenen Kristalle abgesaugt und mit Petrolether gewaschen.
Ausbeute: 13, 9 g einer Verbindung **18**
¹H-NMR (250 MHz): = 7.95 (1H, s), 4.30 (m, 1H), 3,95 (m, 1H), 3.24 (s, 3H), 2,95 (m, 1H), 2,05 (m, 1H), 1,30 (d, 3H), 0,96 (d, 3H), 0,92 (d, 3H).

### Synthese von Beispiel 65

2,1 g der Verbindung 18 wurden in 10 mL Sulfolan mit 4-Aminobenzoesäureethylester versetzt und für 2 h bei 160°C gerührt. Anschließend wurde mit Ether versetzt und die ausgefallenen Kristalle mit Ether gewaschen:
Ausbeute: 3,0 g einer Verbindung 19

3 g der Verbindung 19 wurden mit 200 mL Methanol und 25 mL 4N NaOH versetzt und 4 h bei 60°C gerührt. Anschließend wurde mit Eisessig versetzt, die ausgefallenen Kristalle abfiltriert und mit Ether nachgewaschen.
Ausbeute: 2,3 g einer Verbindung 20 (weiße Kristalle)

0,1 g der Verbindung 20 wurde in 3 mL Dichlormethan und 3 mL DMF suspendiert, und anschließend mit 0,13 g DIPEA, 0,095 g TBTU und 0,045 g Hydroxybenzotriazol (HOBt) versetzt. Anschließend wurde die Lösung für 30 min gerührt und mit 0,035 g N-Methyl-3-Picolylamin versetzt. Nach 0,5 h wurde der Ansatz mit Wasser und 1 g Kaliumcarbonat versetzt, die wässrige Phase zweimal mit je 50 mL Ethylacetat extrahiert und das Produkt mittels Kieselgelchromatographie gereinigt und anschließend aus Ethanol/Aceton umkristallisiert.
Ausbeute: 0.08 g

### Synthese von Beispiel 125

3,7 g der Verbindung 20, 3,8 g TBTU, 1,6 g HOBt, 5 mL DIPEA wurde in 40 mL DMF gelöst und 4 h bei Raumtemperatur gerührt. Der Ansatzt wurde eingeengt, in 200 mL Ethylacetat aufgenommen und zweimal mit je 5 mL 5%iger Kaliumcarbonatlösung extrahiert. Die organische Phase wurde eingeengt, die ausgefallenen Kristalle abfiltriert und mit Etylacetat und Ether gewaschen.
Ausbeute: 1,65 g einer Verbindung **21** (gelbliche Kristalle)

0,486 g der Verbindung **21** wurden mit 0,33 g 1,2 Phenylendiamin in 10 mL Toluol 0,5 h unter Rückfluß gekocht, anschließend wurde der Ansatz eingeengt. Der Rückstand wurde mit 100 mL Ethylacetat versetzt, die org. Phase zweimal mit Wasser extrahiert. Die organische Phase wurde eingeengt, die ausgefallenen Kristalle abgesaugt und mit wenig Ethylacetat gewaschen.
Ausbeute: 0,25 g einer Verbindung 22 (weiße Kristalle)

0,22g der Verbindung 22 wurden in 20 g Polyphosphorsäure 0,5 h bei 150°C gerührt, dann auf Eis gegeben und mit Ammoniak versetzt. Anschließend wurde zweimal mit je 100 mL Ethylacetat extrahiert, die org. Phase mit Wasser gewaschen und eingeengt. Das ausgefallene Produkt (Kristalle) wurde abgesaugt und mit Ethylacetat und Ether gewaschen.
Ausbeute: 0,115 g gelbliche Kristalle, Smp.: 287°C (Zersetzung)

### Beispiel 171

Zur Synthese der Verbindung 171 wird zunächst eine Zwischenverbindung **27**

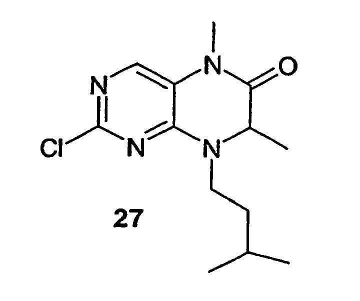

34,4 g N-isopentyl-benzylamin, 36,2 g 2-Brom-propionsäureethylester und 42,0 g Kaliumcarbonat wurden in 250 ml DMF vorgelegt und 3 h bei 110° C gerührt. Nach Abkühlen wurden die anorganischen Salze abfiltriert, das Filtrat eingedampft. Der Rückstand wurde mit Wasser und Diethylether extrahiert, die org. Phase mit Wasser gewaschen, getrocknet und zur Trockene eingedampft.
Ausbeute: 55,5 g einer Verbindung **23**

55,5 g der Verbindung 23 wurden in 600 ml Ethanol vorgelegt, mit 20 ml 32%iger HCl und 6 g 10%iger Pd/C bei 20°C und 5 bar 20 min. hydriert. Anschließend wurde über Kieselgur filtriert und eingedampft. Der Rückstand wurde mit 400 ml Diethylether versetzt, der Niederschlag abgesaugt und mit Diethylether gewaschen.
Ausbeute: 23,5 g einer Verbindung 24, Smp. 105°C

23,5 g der Verbindung 24 wurden in 200 ml Wasser gelöst, und mit 20,0 g (0,103 mol) 2,4 -Dichlor-5-nitropyrimidin in 400 ml Diethylether versetzt. Nach Kühlung des Reaktionsgemisches auf -10° C wurden 50,0 g (0,499 mol) Kaliumcarbonat portionsweise zugegeben. Es wurde 1 h bei -5°C und 1 h bei 0° C gerührt, dann auf Raumtemperatur erwärmt. Die Wasserphase wurde abgetrennt, die organische Phase mit Wasser gewaschen, getrocknet und zur Trockene eingedampft.
Ausbeute: 36,9 g einer Verbindung 25

20,0 g der Verbindung 25 wurden in 280 ml Eisessig gelöst und auf 70° C erwärmt. Nach entfernen der Heizquelle wurden 17 g Eisen zugegeben. Die Temperatur stieg auf 100° C an, dann wurde 30 min. bei dieser Temperatur gerührt.
Anschließend wurde heiß filtriert und das Filtrat eingedampft. Der Rückstand wurde mit 300 ml Dichlormethan und 30 ml 32%iger HCl versetzt, die Phasen getrennt, die wässrige Phase mit Dichlormethan extrahiert, die vereinten organischen Phasen mit Wasser und wässriger Ammoniaklösung gewaschen, getrocknet und zur Trockene eingedampft. Der Rückstand wurde mit Diethylether ausgerührt.
Ausbeute: 10,5 g einer Verbindung **26,** Smp.: 182°-185° C

2,7 g der Verbindung **26** und 2,5 ml Methyljodid wurden in 27 ml DMA vorgelegt und auf-10° C gekühlt. 0,45 g NaH, 60%ige Dispersion in Mineralöl, wurden zugegeben, 30 min. bei -5° C gerührt. Anschließend wurden 10 g Eis und 5 ml 2N HCl zugegeben und eingedampft. Der Rückstand wurde mit Ethylacetat und Wasser extrahiert, die organische Phase getrocknet, zur Trockene eingedampft und über Kieselgel filtriert.
Ausbeute: 3,0 g der Verbindung 27 (Öl)
¹H-NMR (250 MHz): = 7.67 (1 H, s), 4,32-4,07 (m, 2H), 3,32 (s, 3H), 3.08 (m, 1 H), 1,70-1,50 (m, 3H), 1,42 (d, 3H), 0,95 (m, 6H).

### Synthese von Beispiel 171

0,28 g der Verbindung 27, 0,9 mL Sulfolan und 0,22 g p-Aminobenzoesäurebenzylamid wurden für 0,5 h bei 170°C gerührt, der Ansatz anschließend mit Ether versetzt und die Kristalle abfiltriert. Das Produkt wurde aus Ethanol umkristallisiert. Ausbeute: 0,15 g,. Smp.: 228-240°C, (gelbliche Kristalle)

Analog zu vorstehend beschriebener Vorgehensweise werden u.a. die in Tabelle 1 aufgeführten Verbindungen der Formel (I) erhalten.
Die in Tabelle 1 verwendeten Abkürzungen X₂, X₃, X₄, X₅ und X₆ stehen jeweils für eine Verknüpfung mit einer Position in der unter Tabelle 1 aufgeführten allgemeinen Formel anstelle der entsprechenden Reste R², R³, R⁴, R⁵ und R⁶.

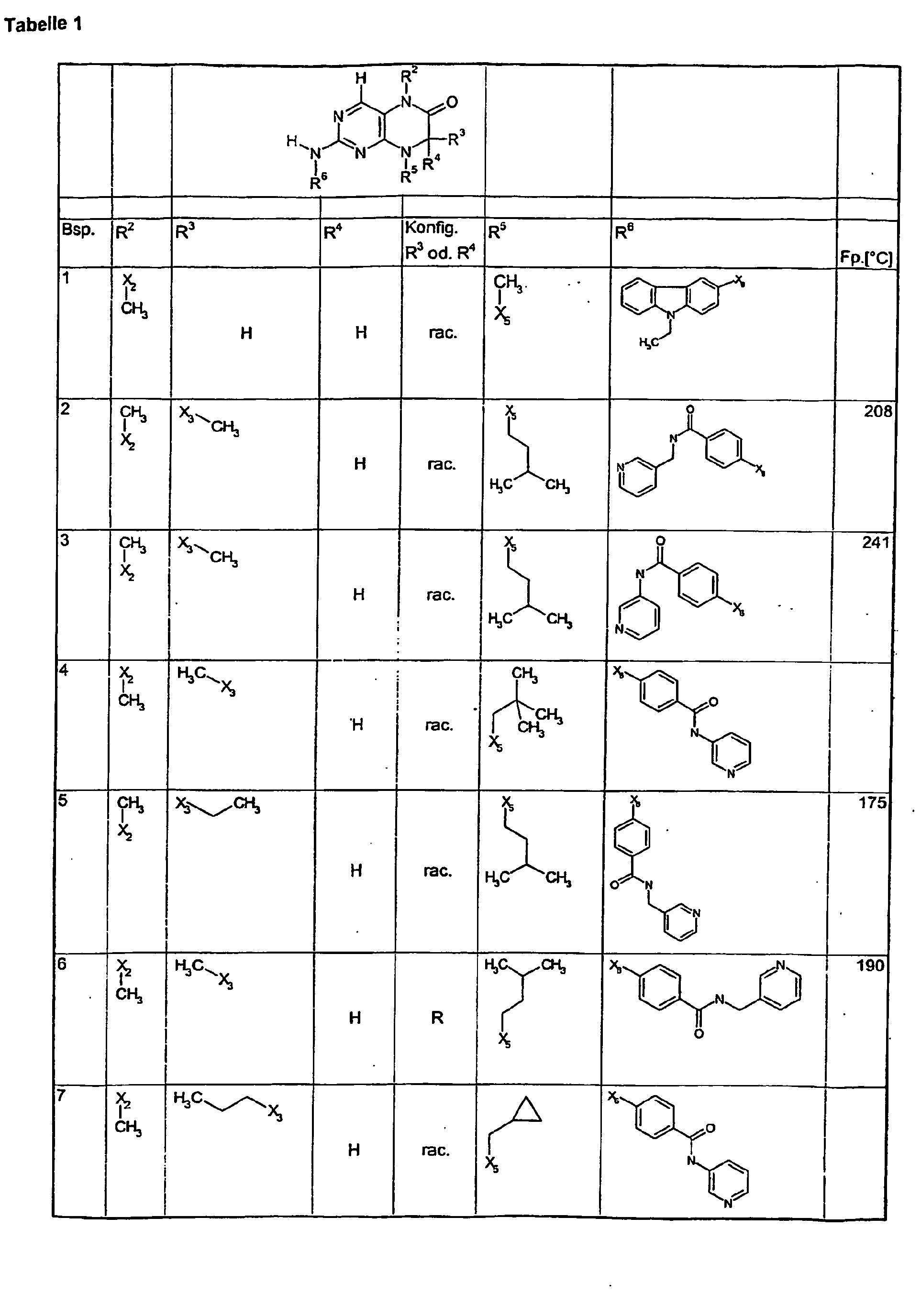

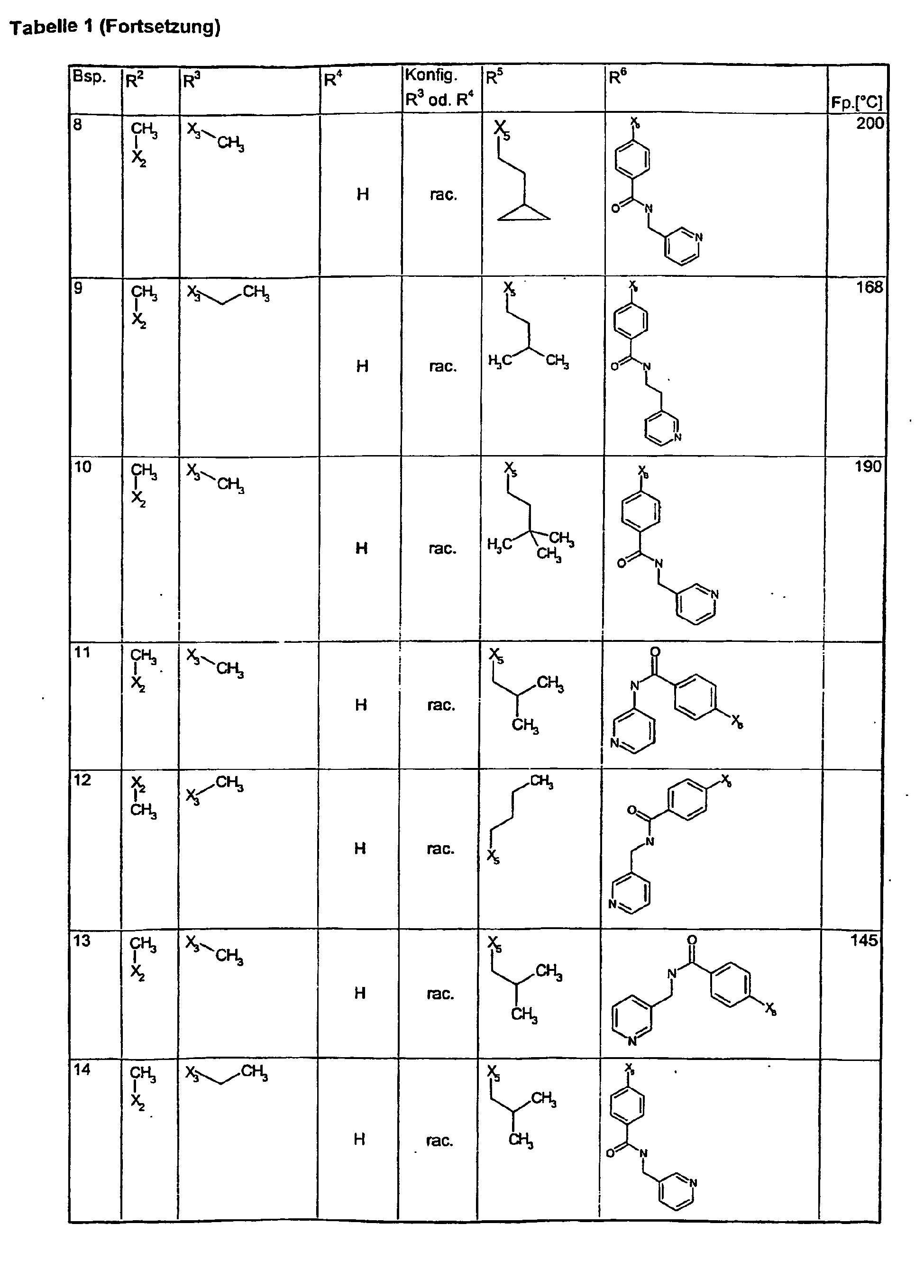

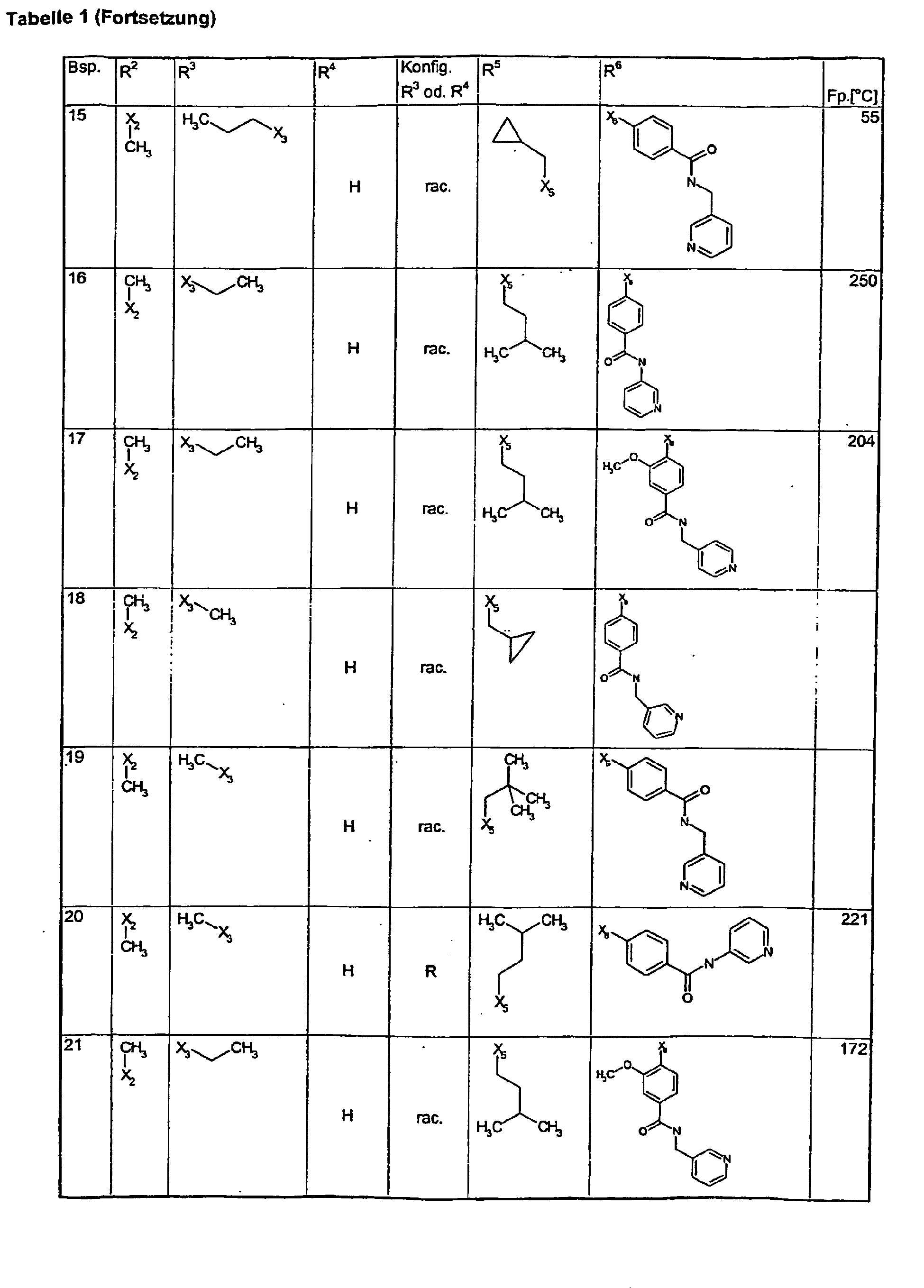

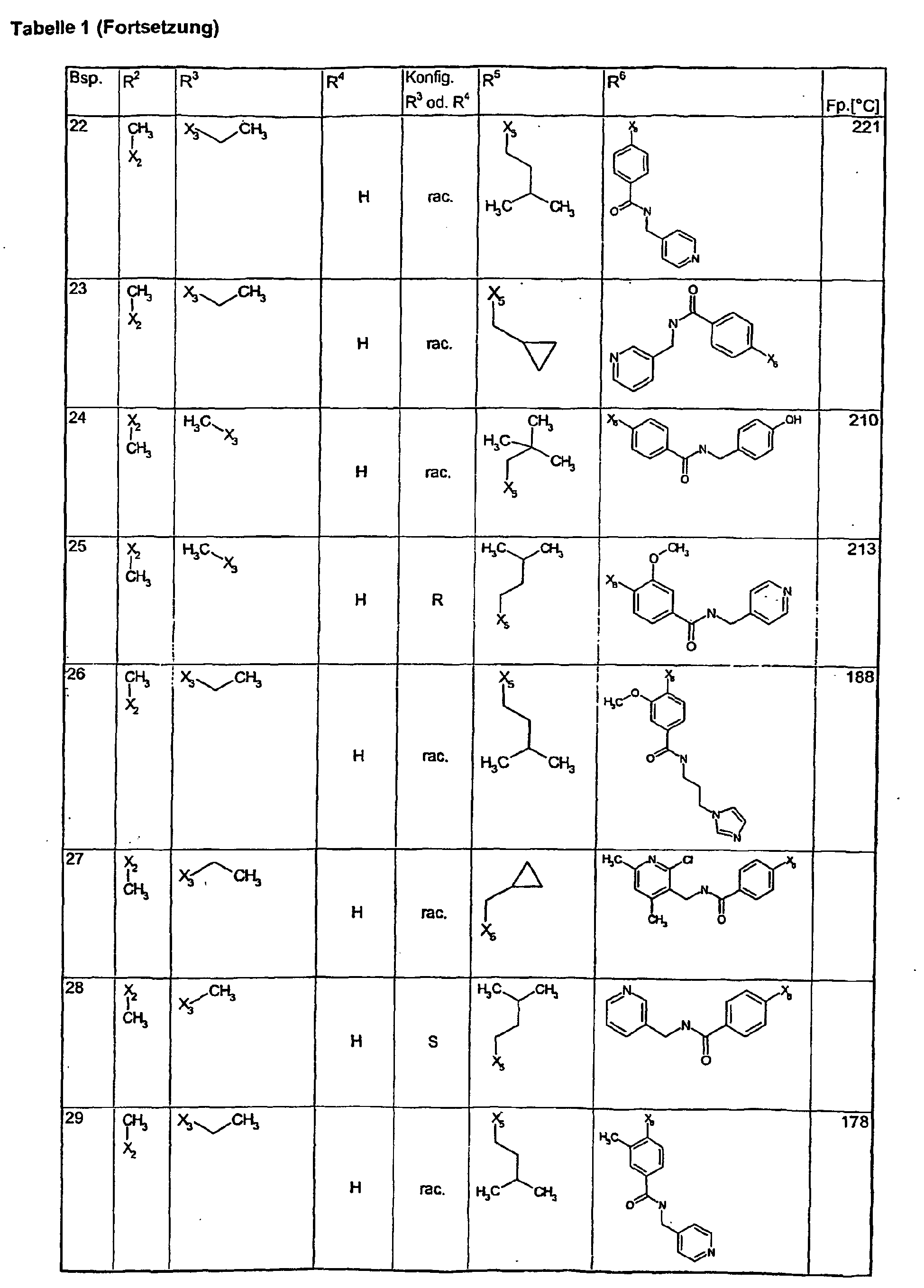

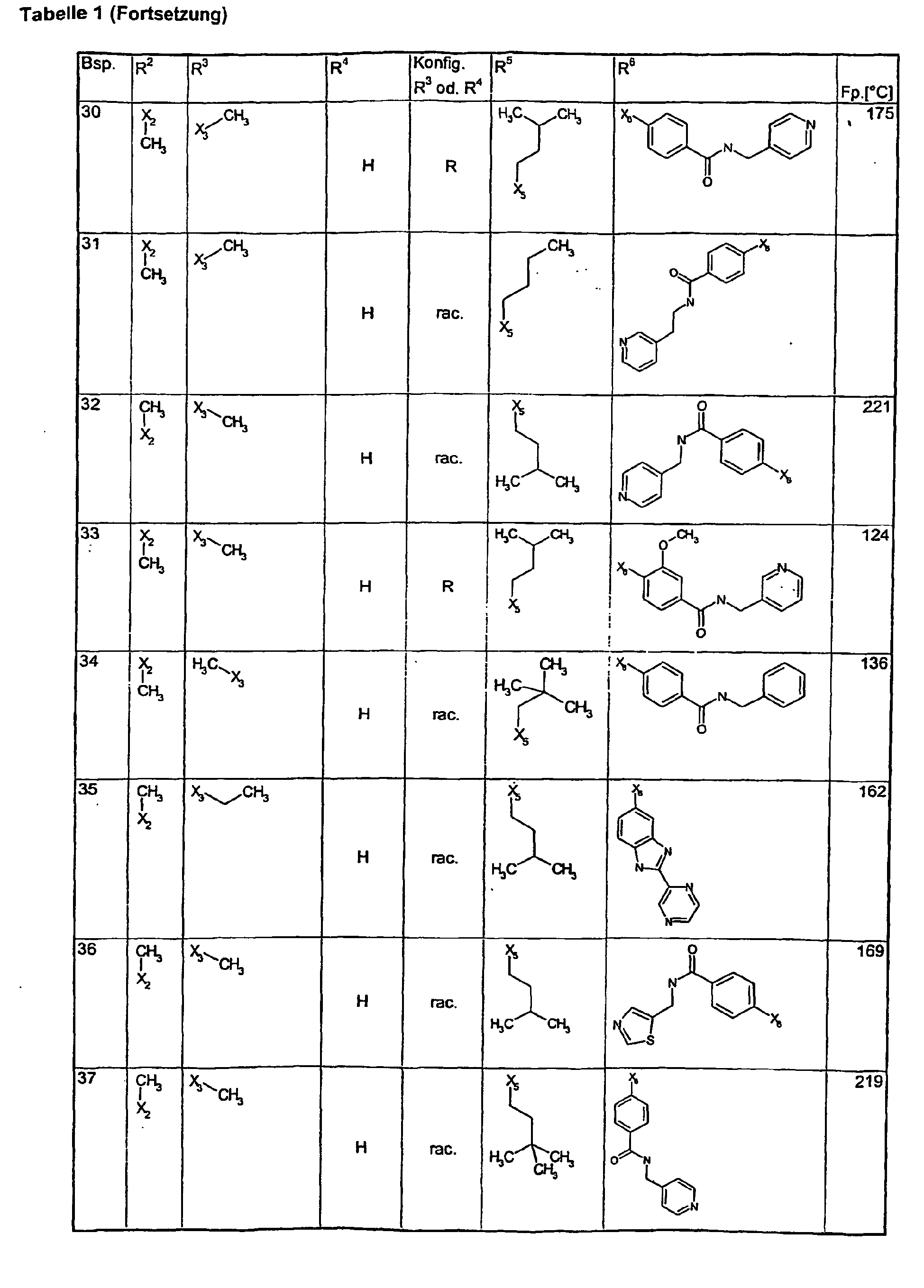

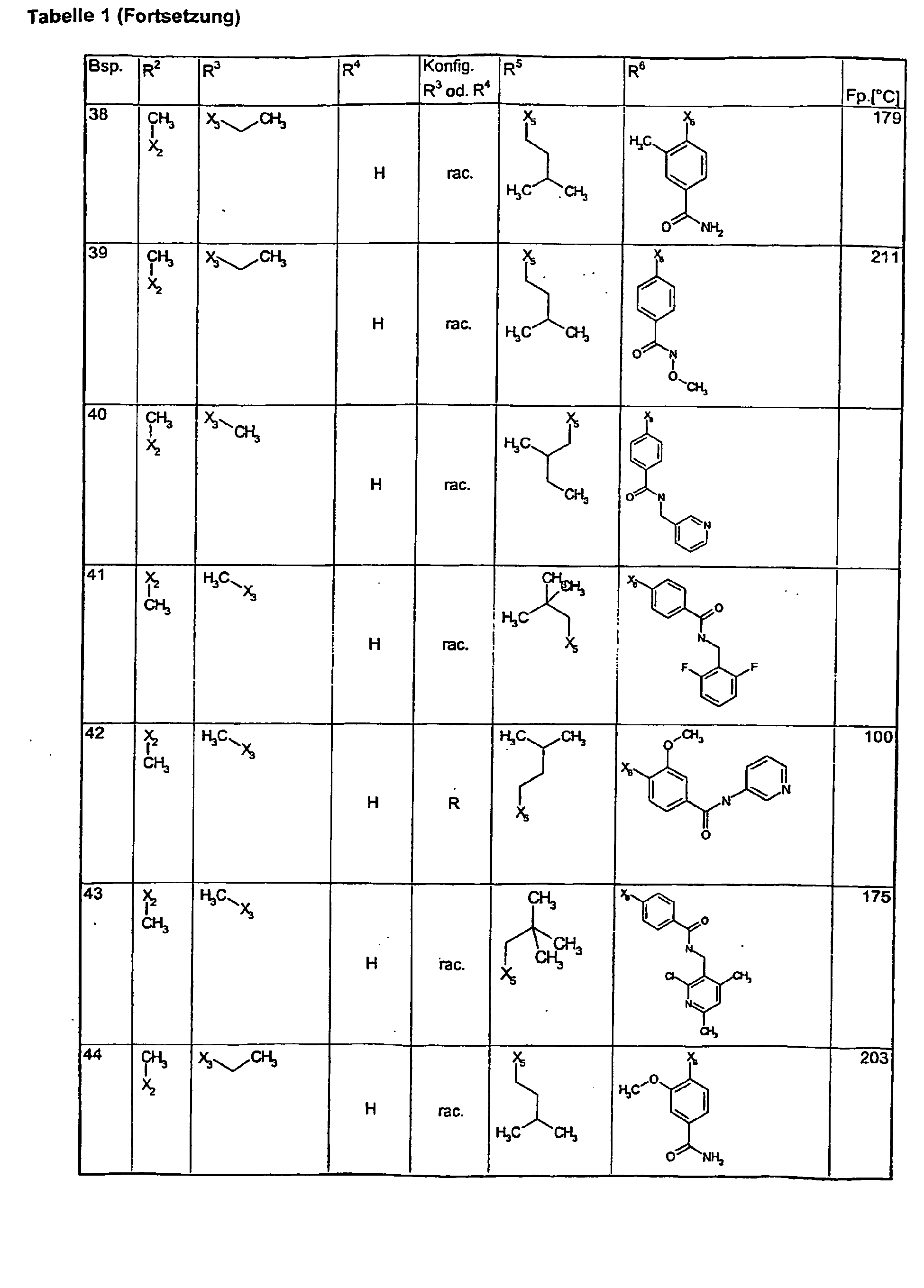

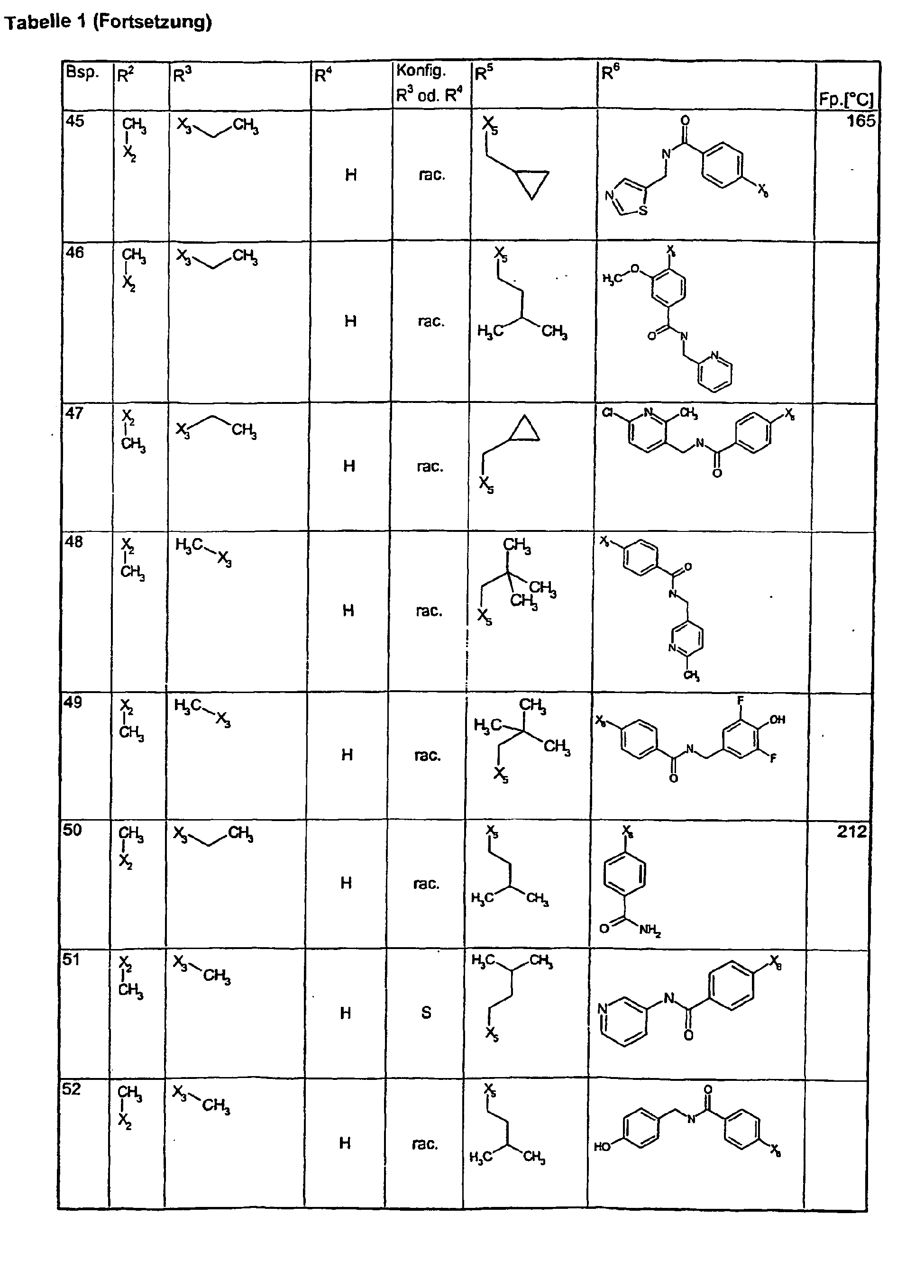

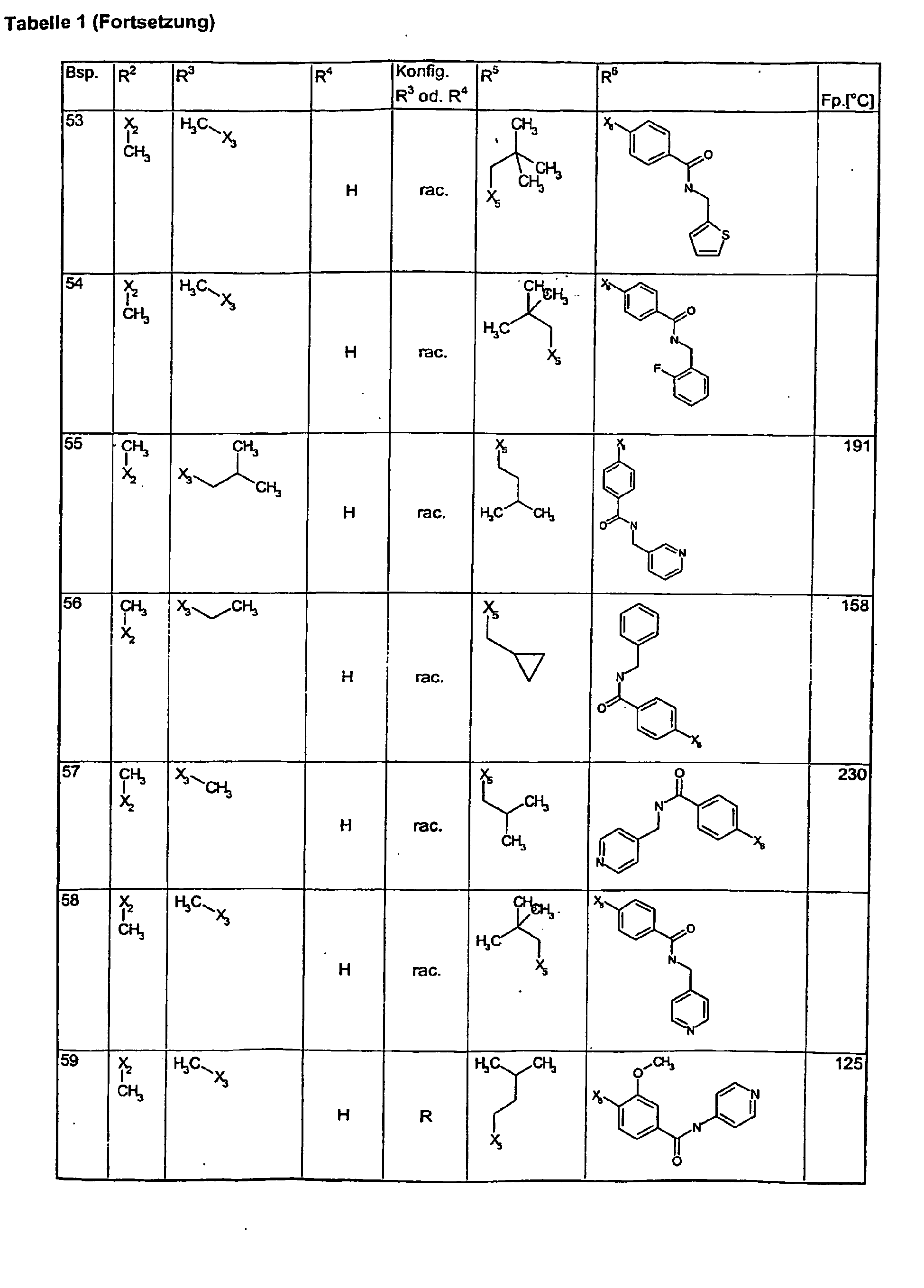

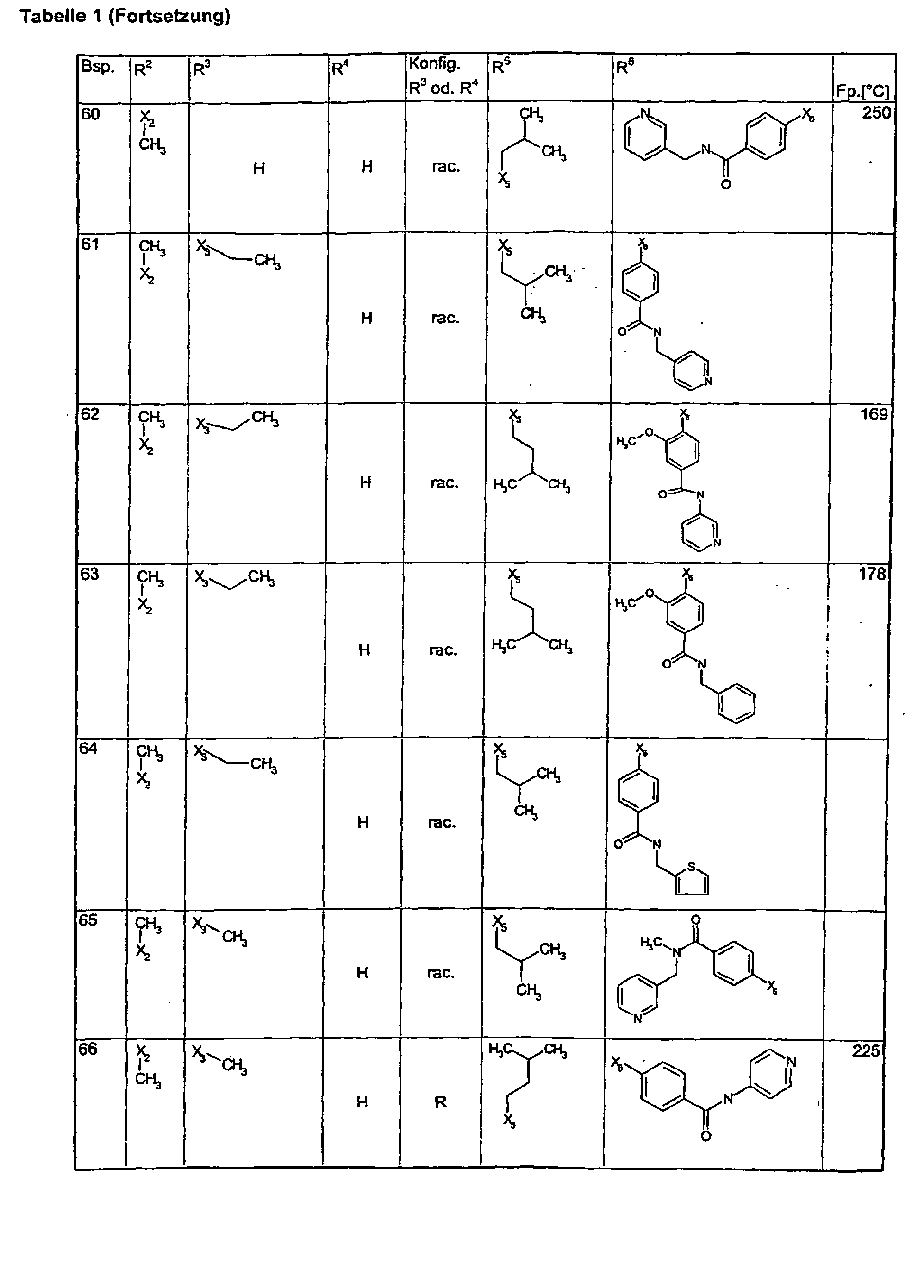

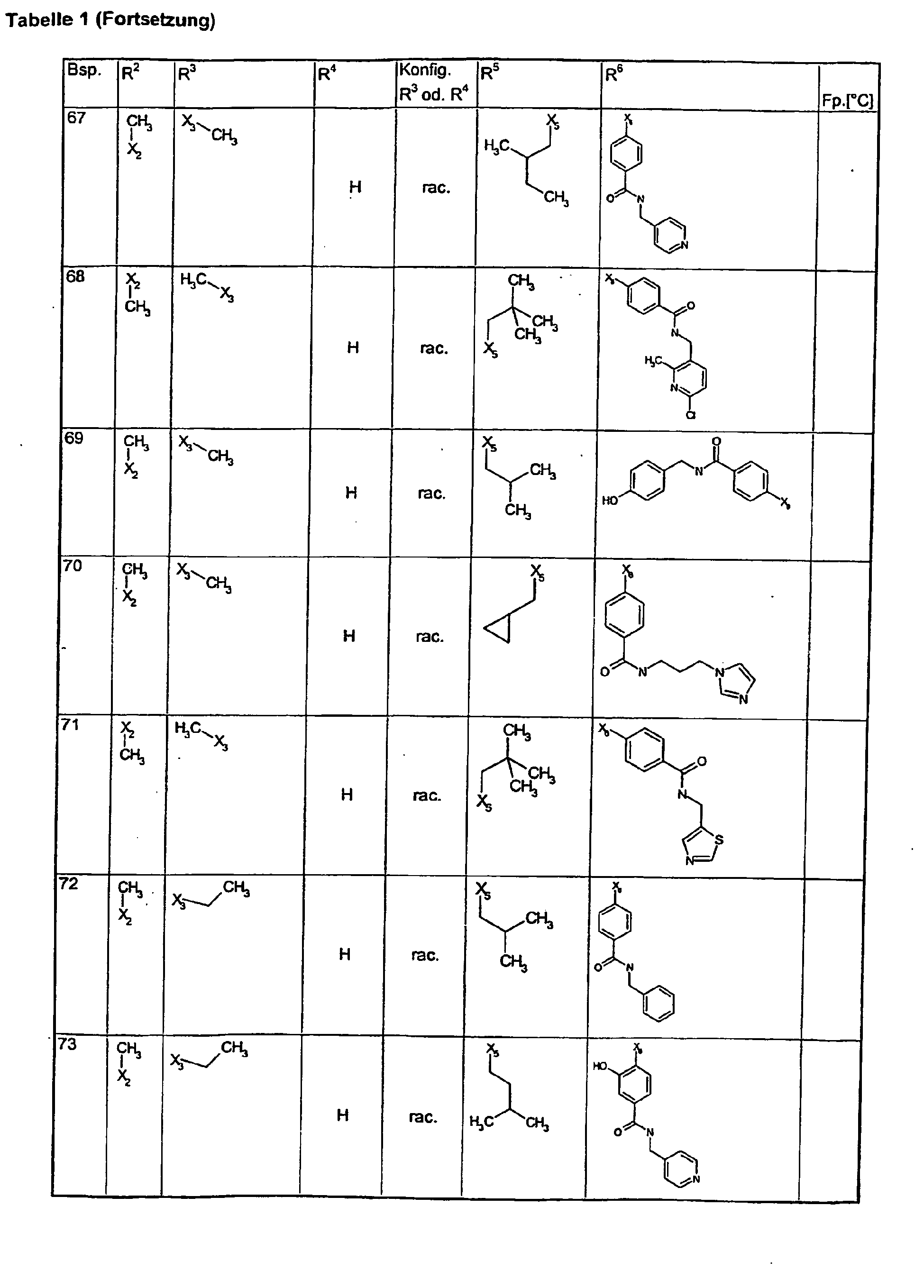

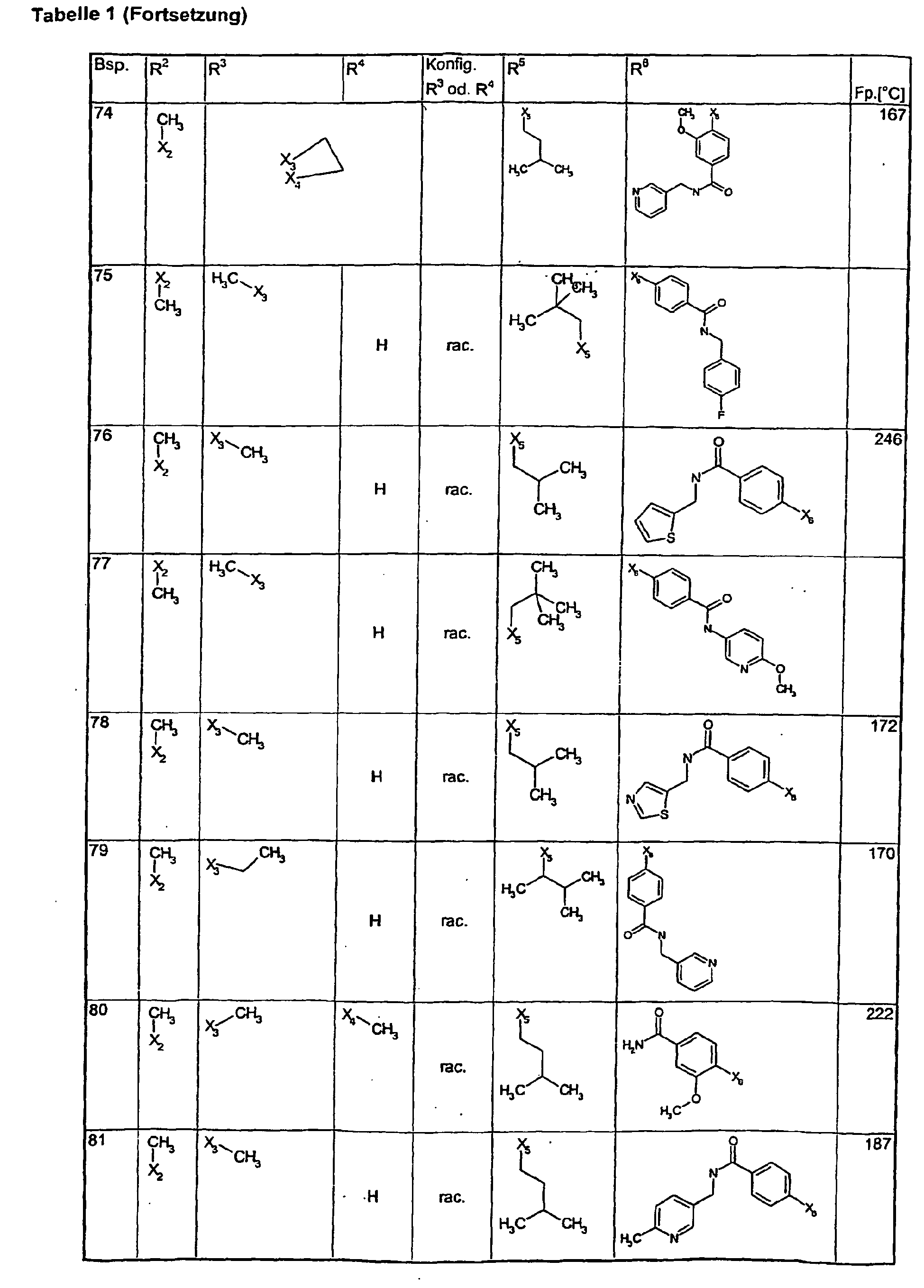

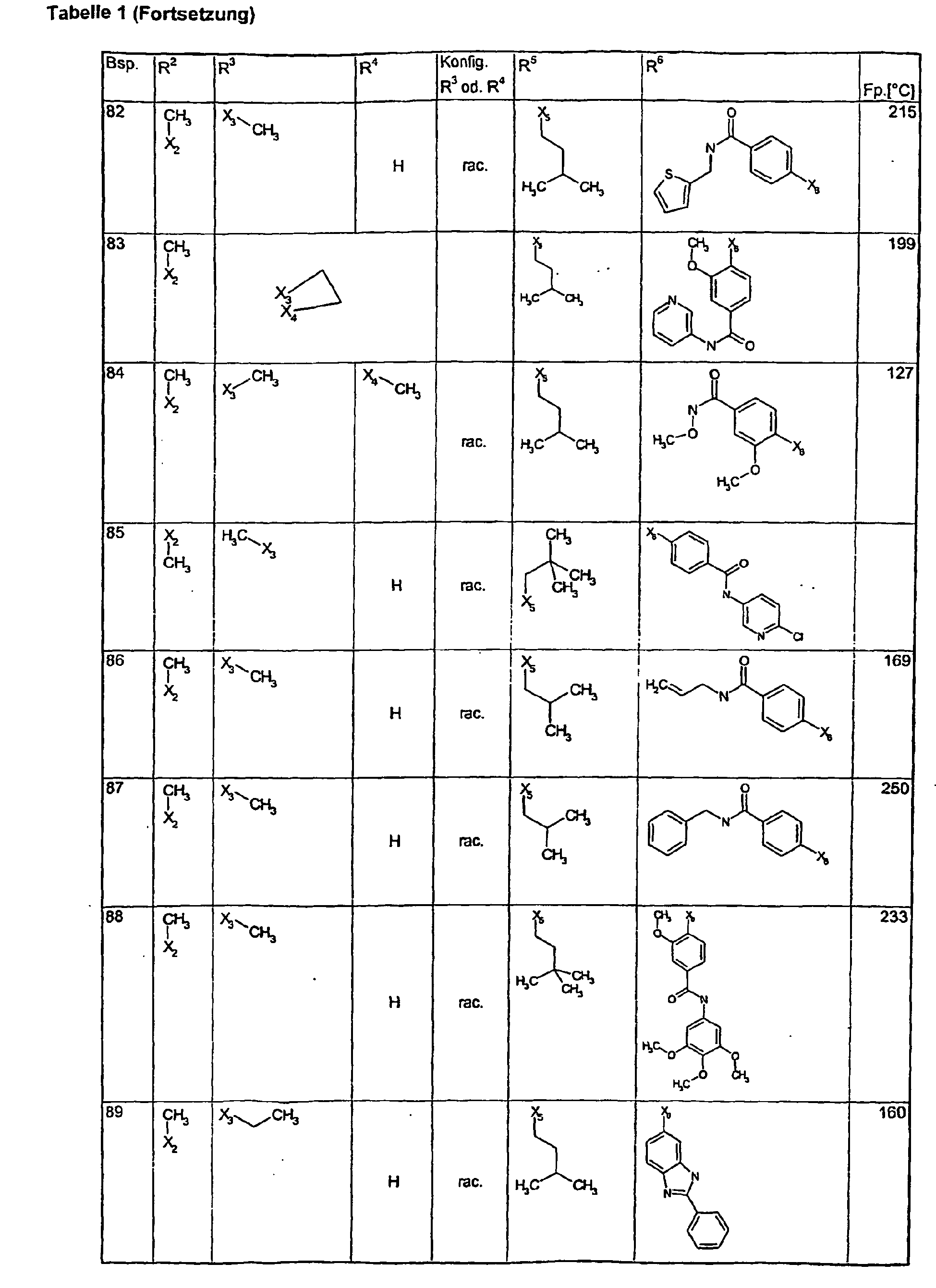

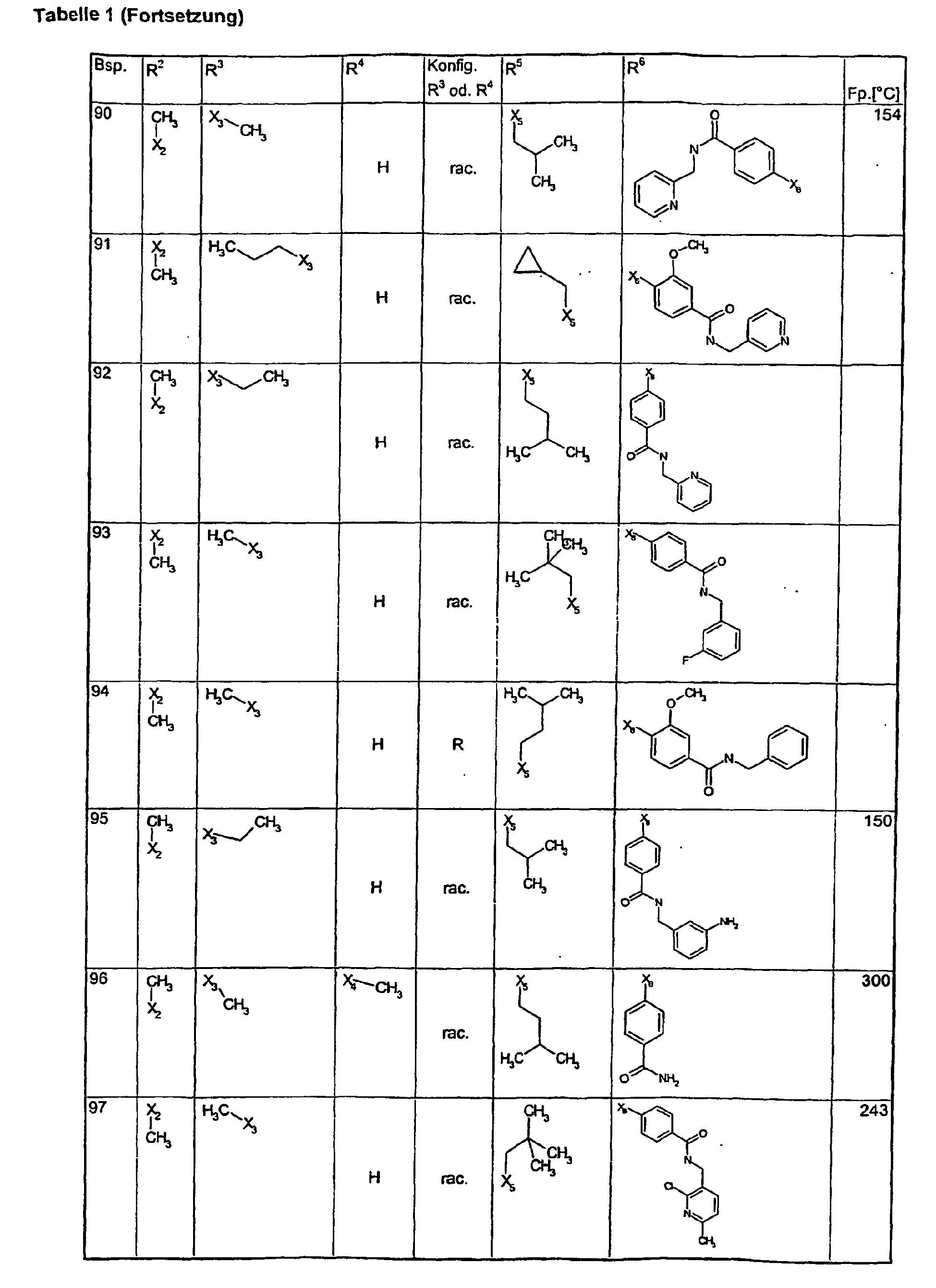

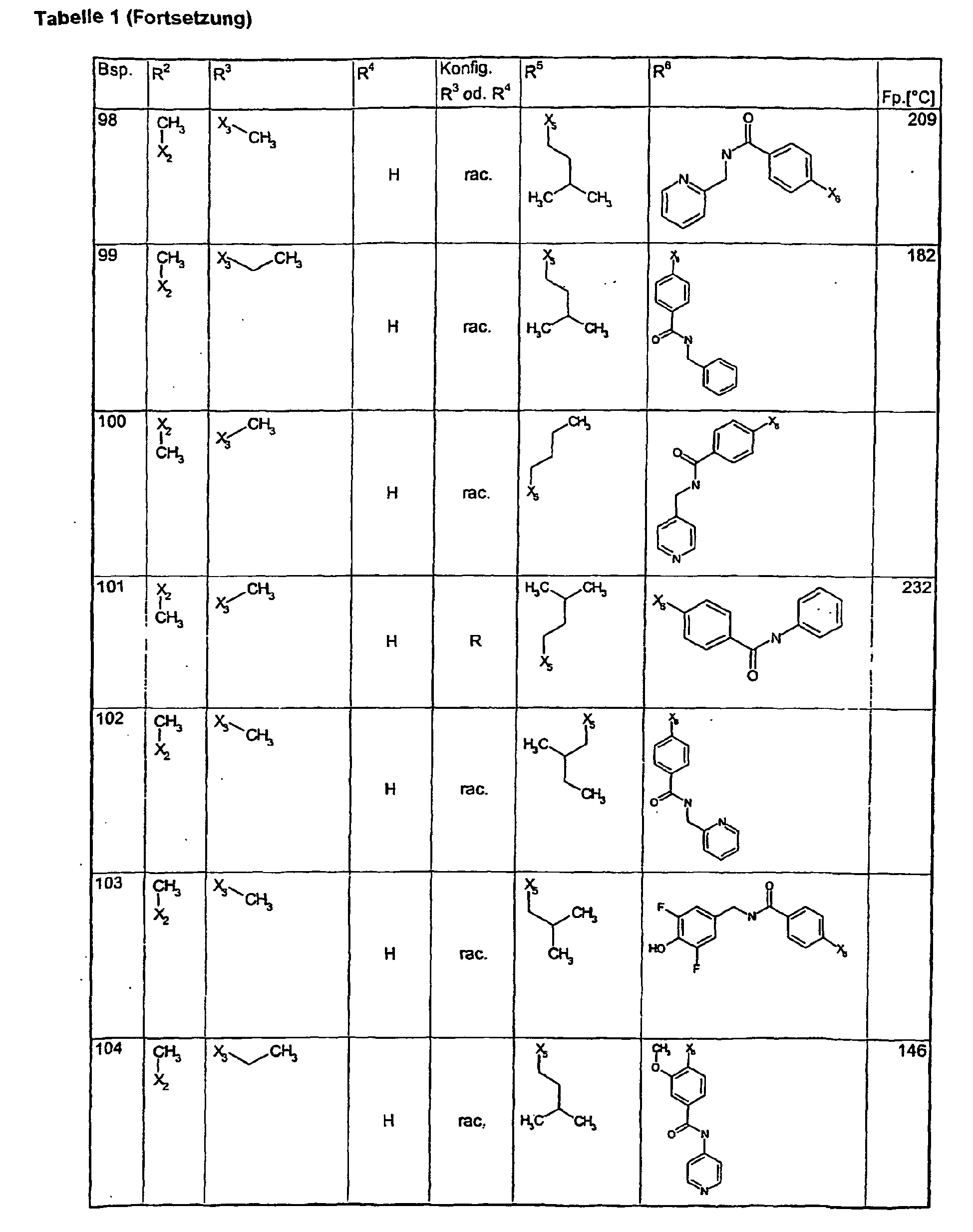

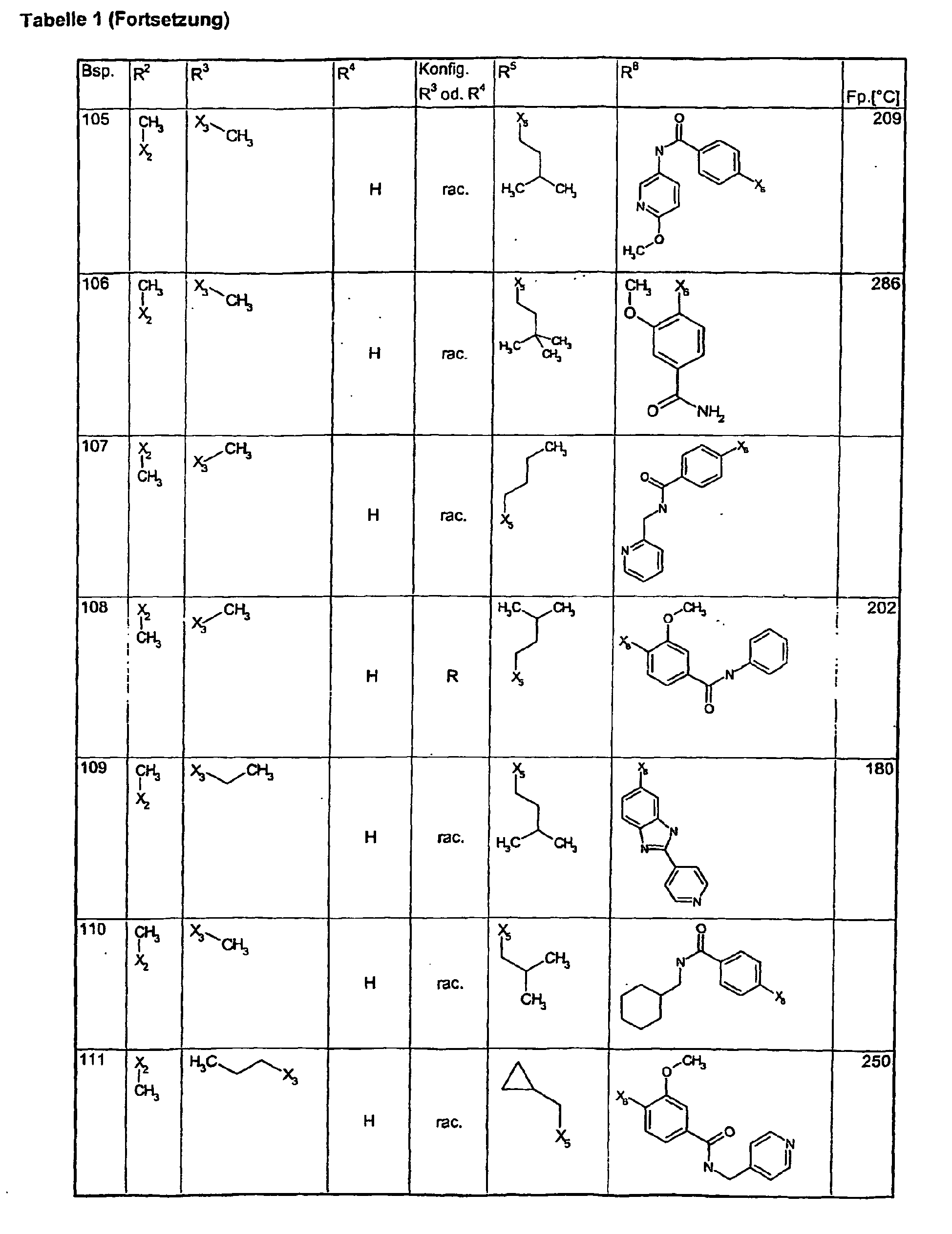

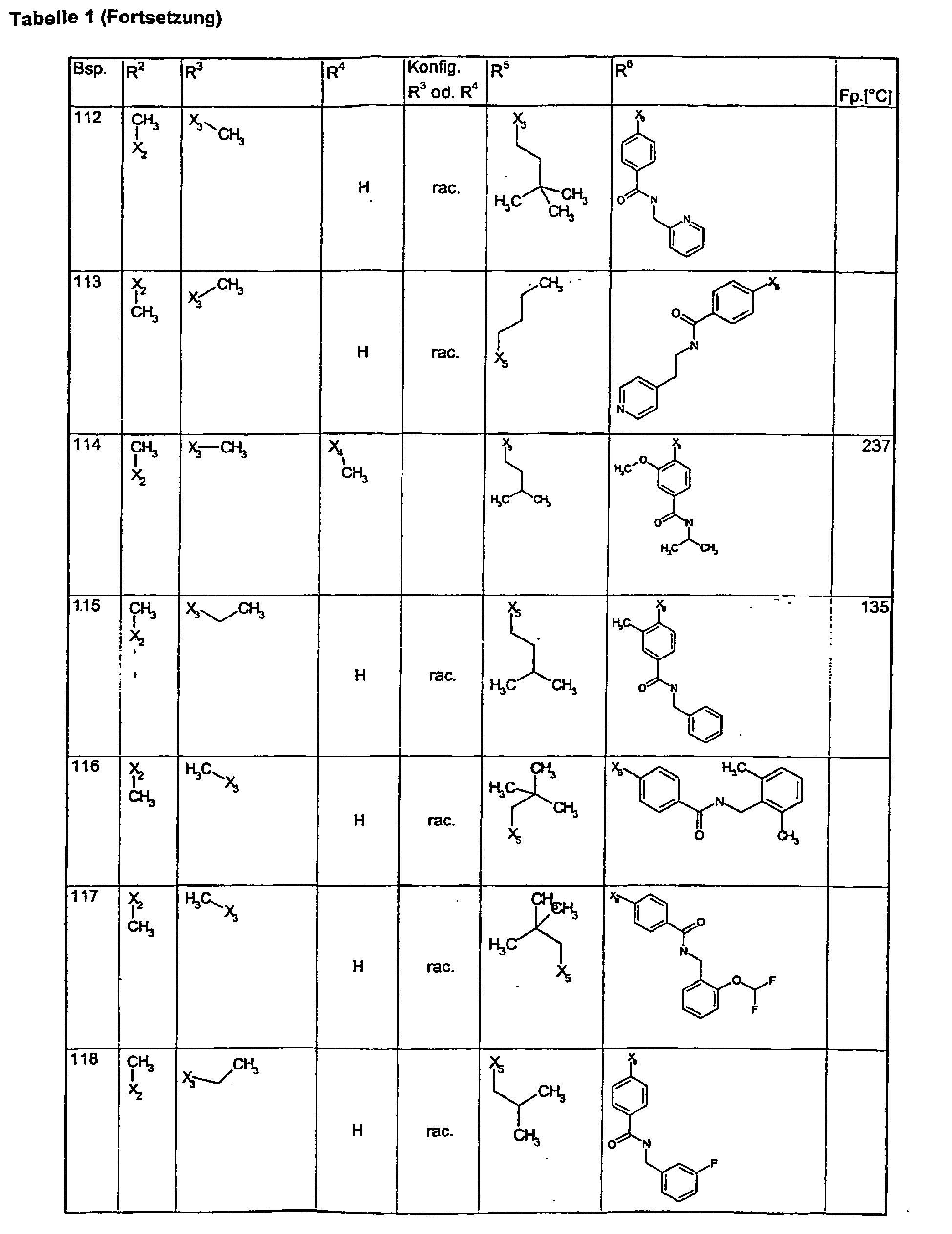

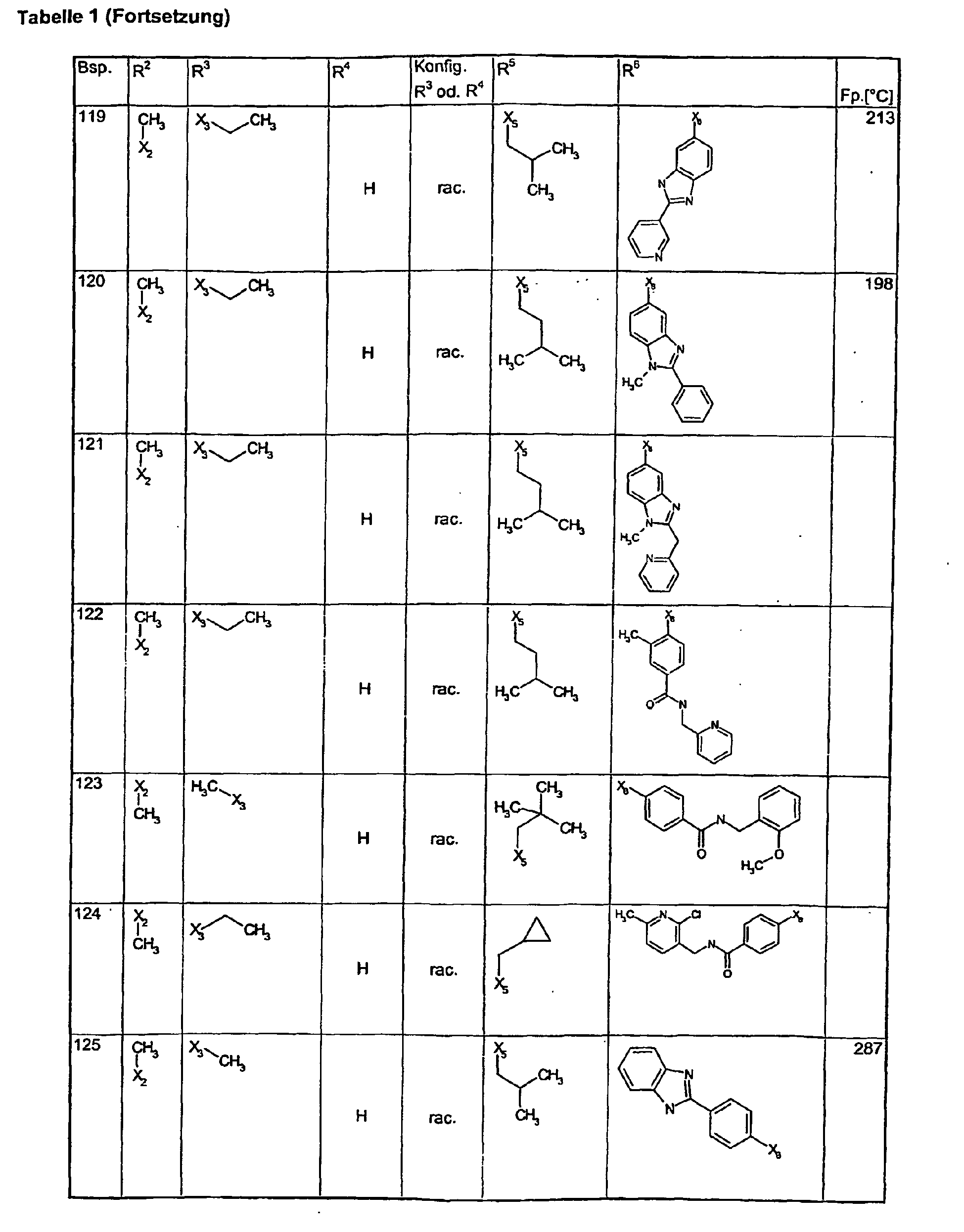

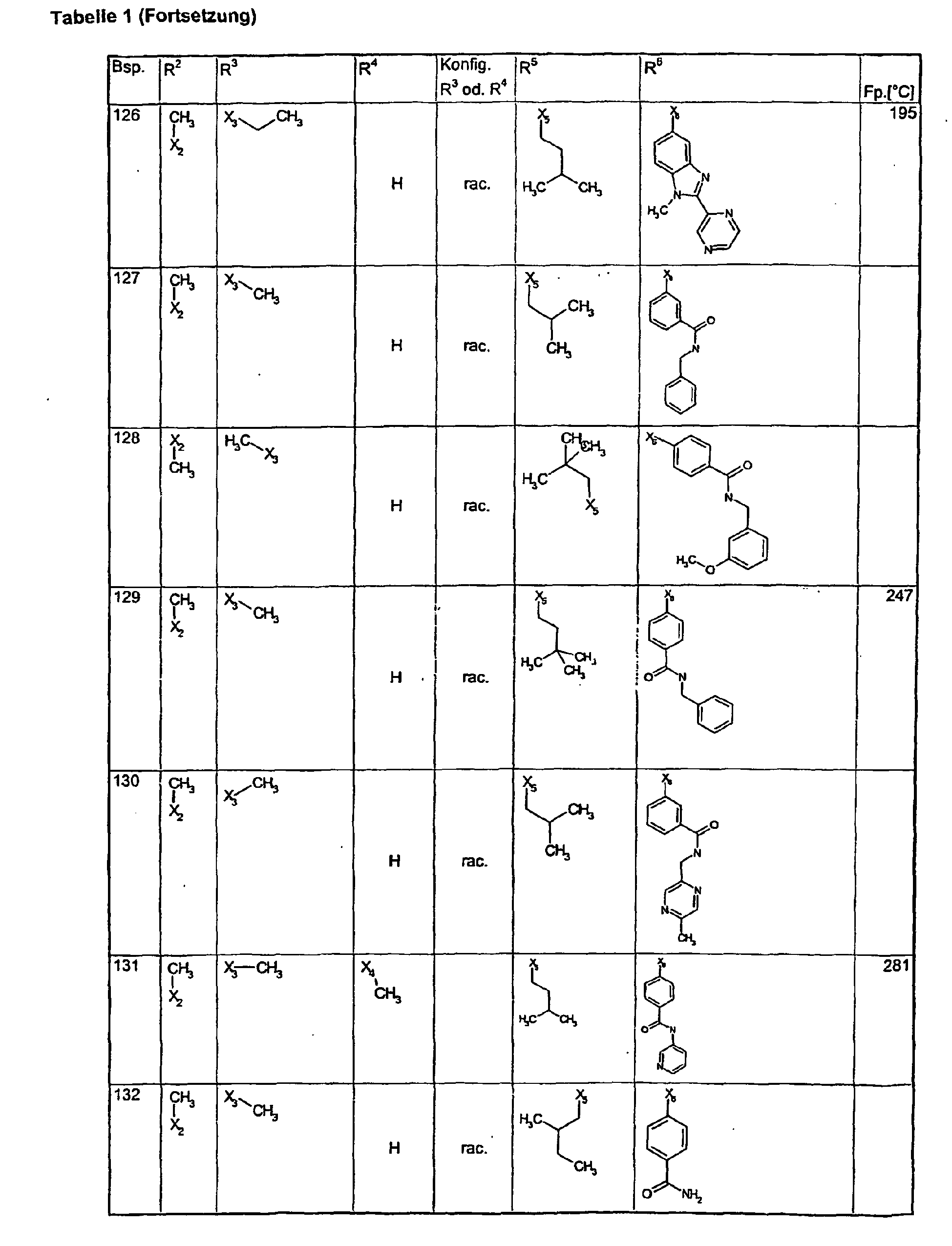

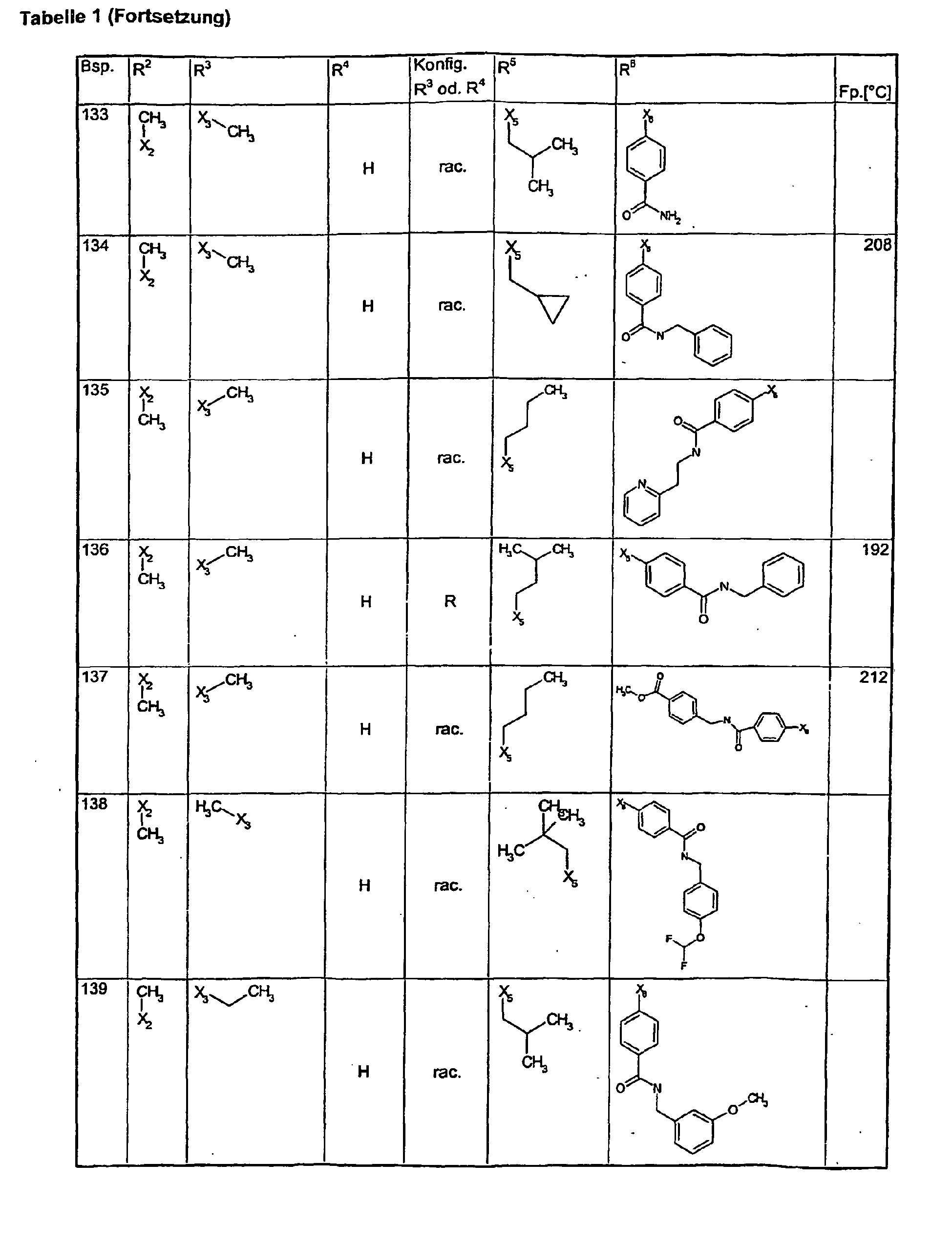

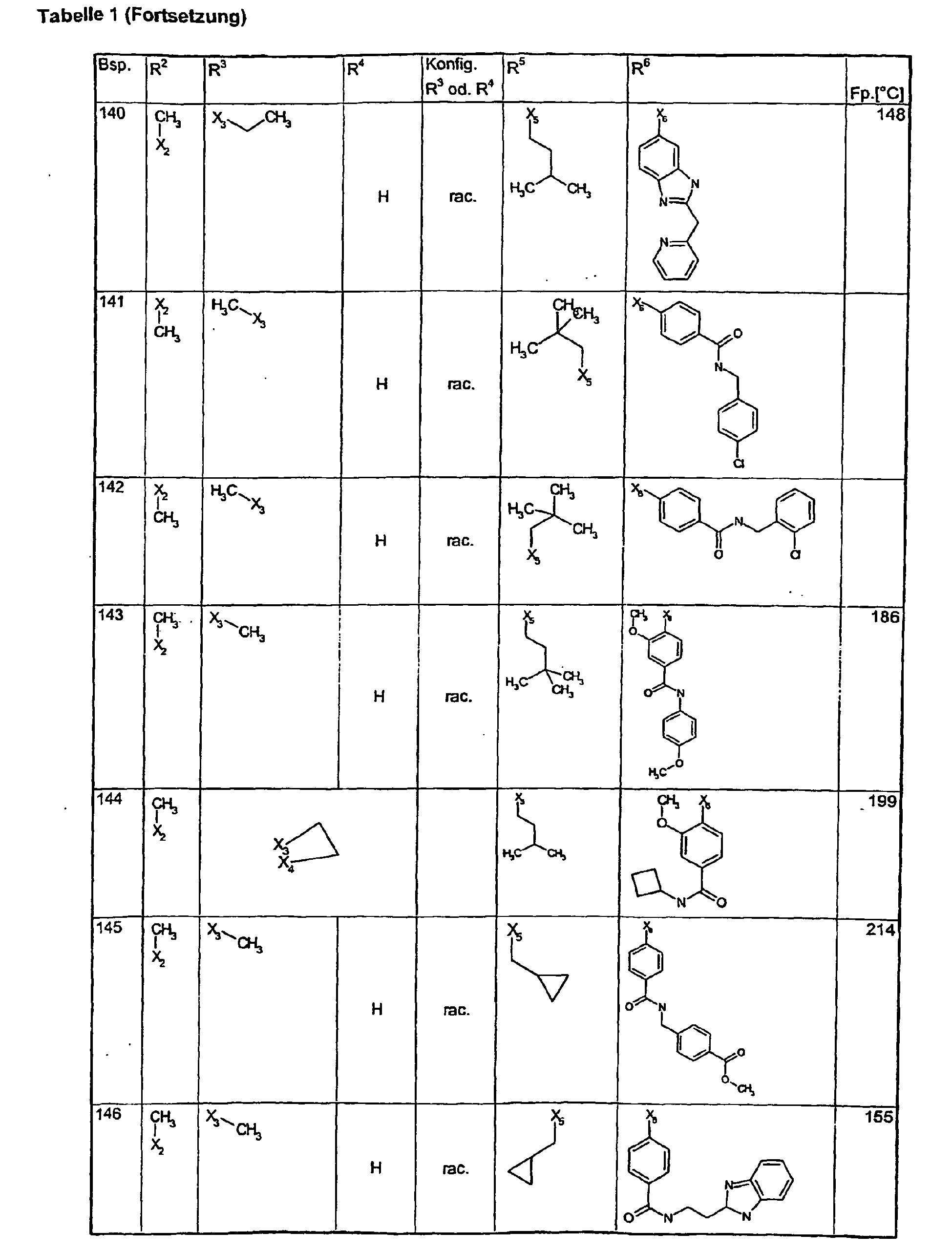

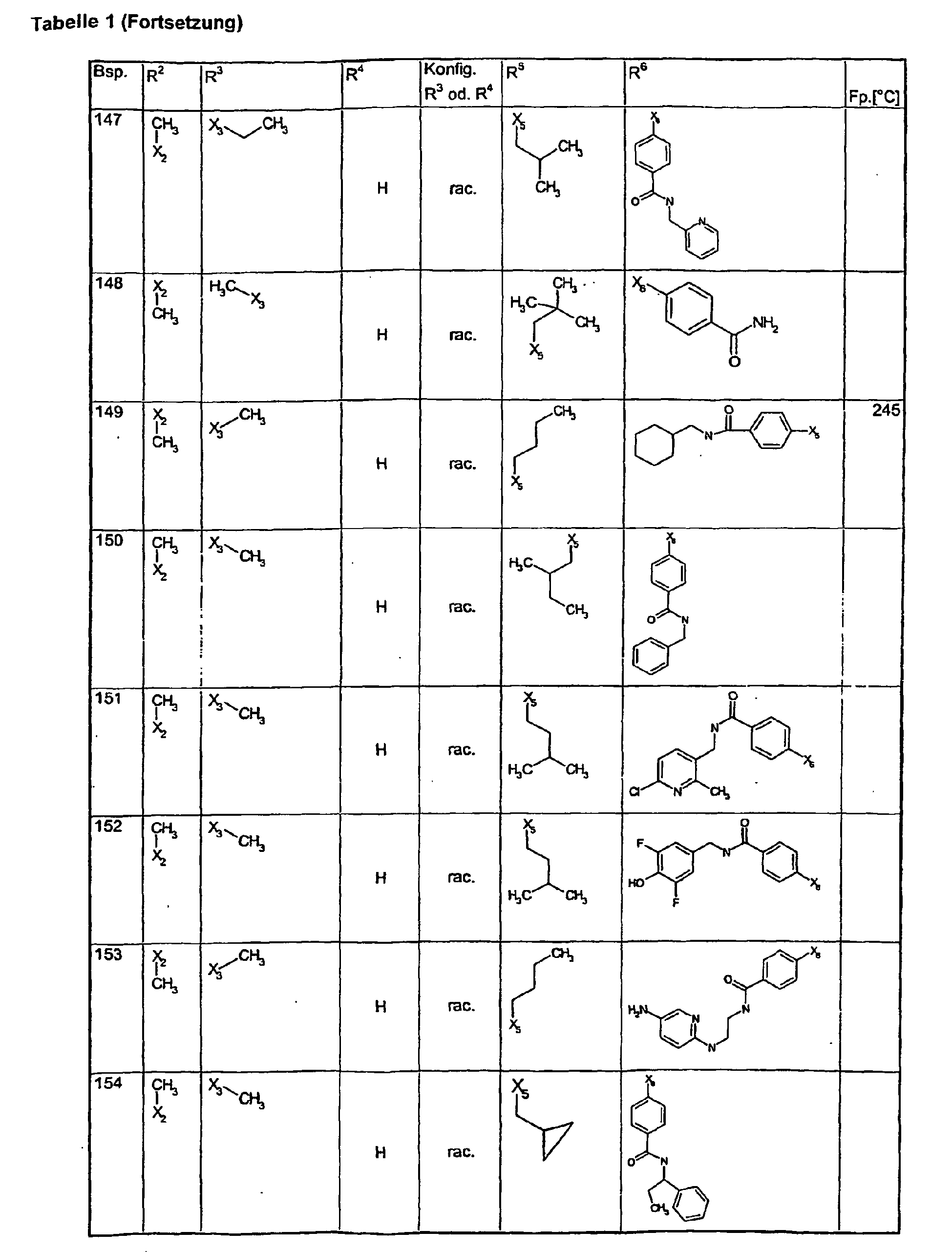

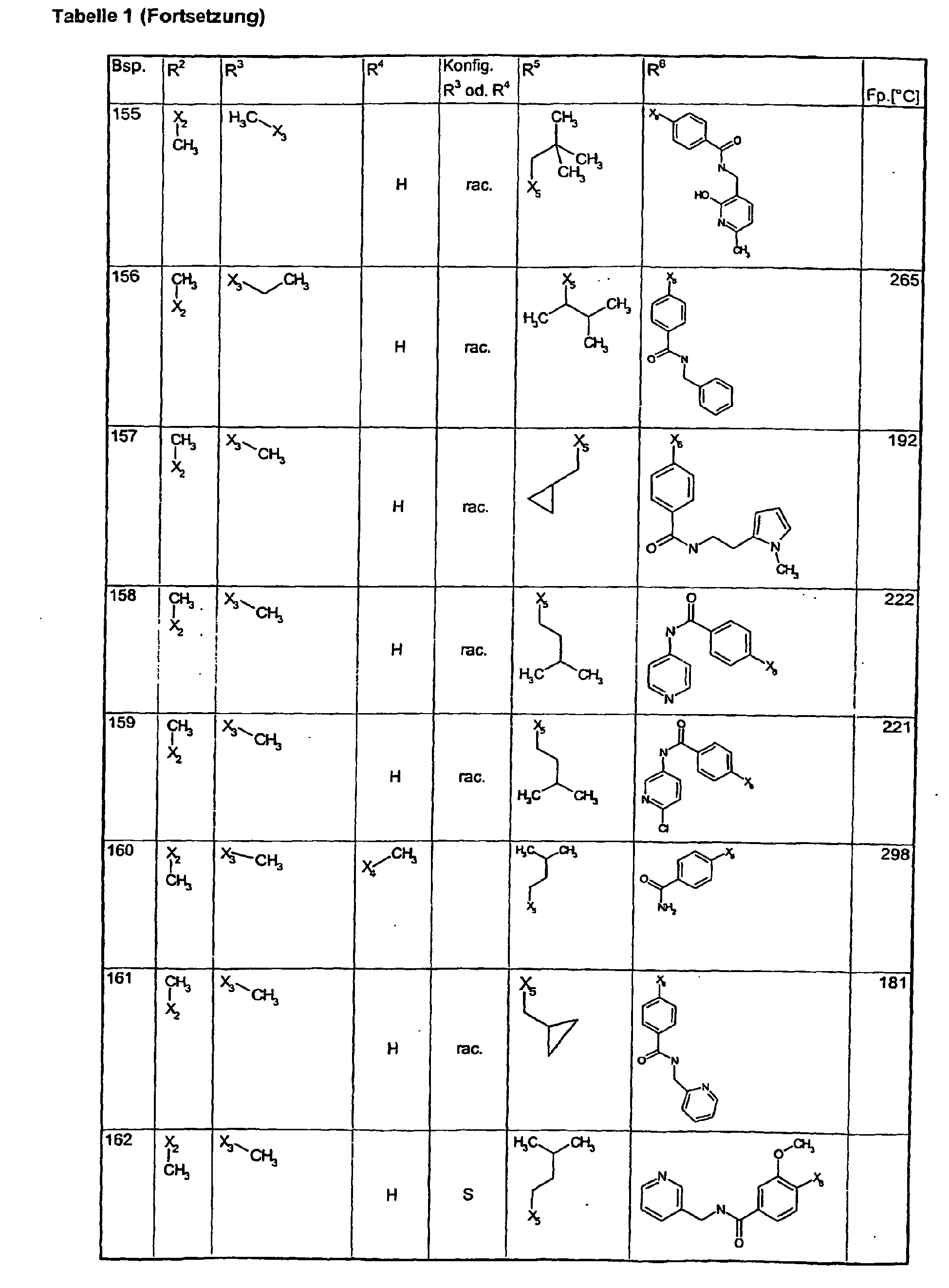

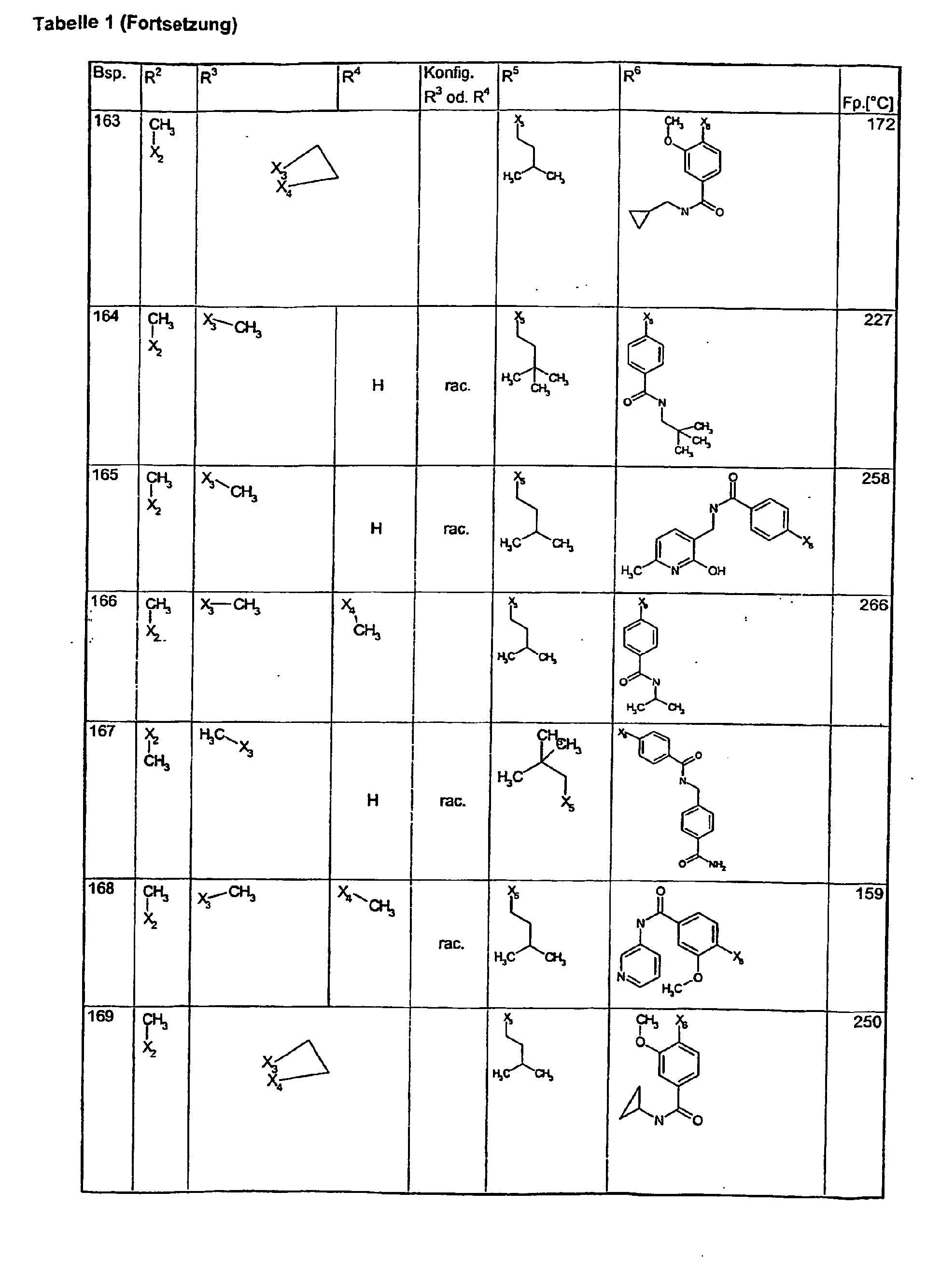

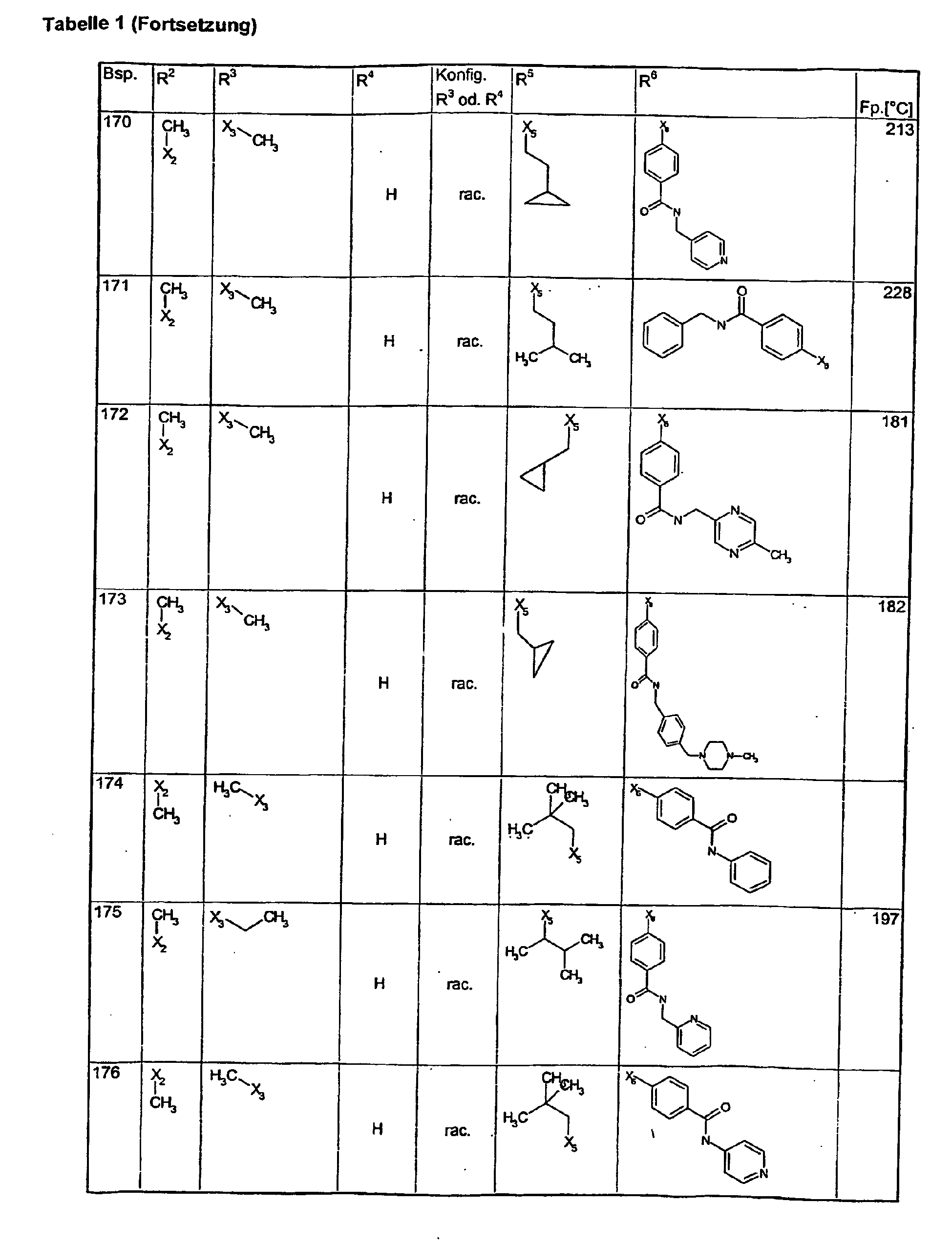

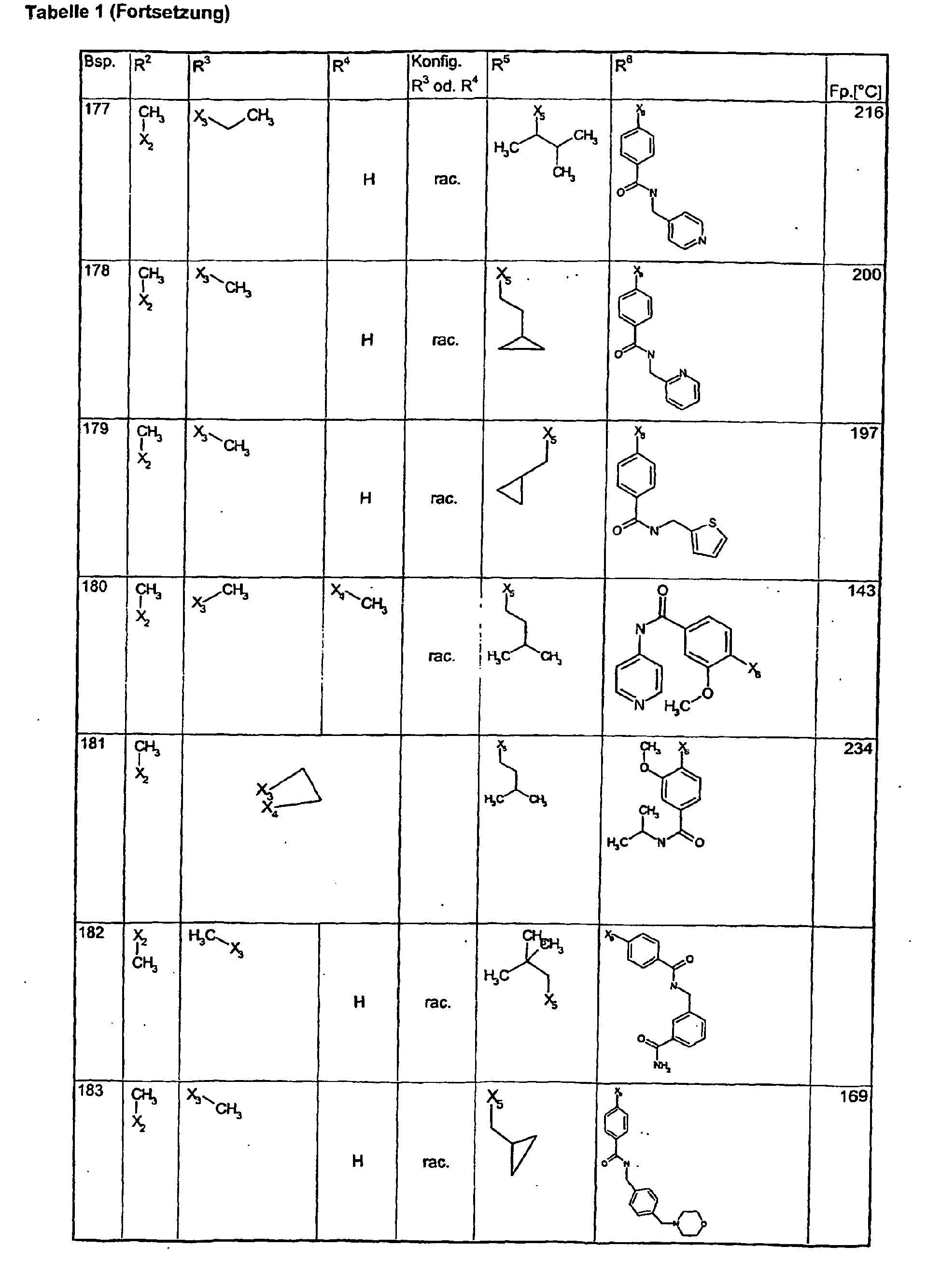

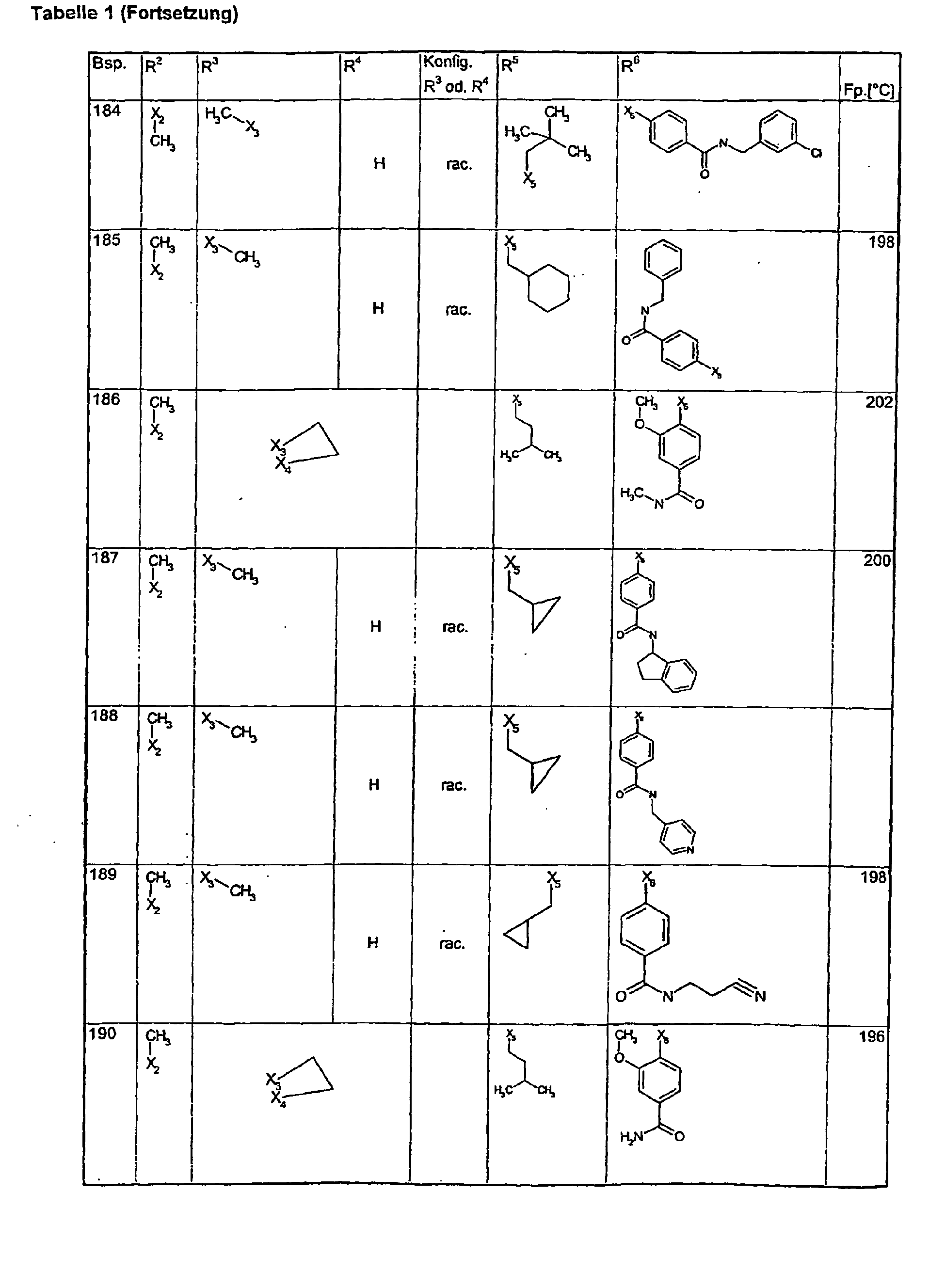

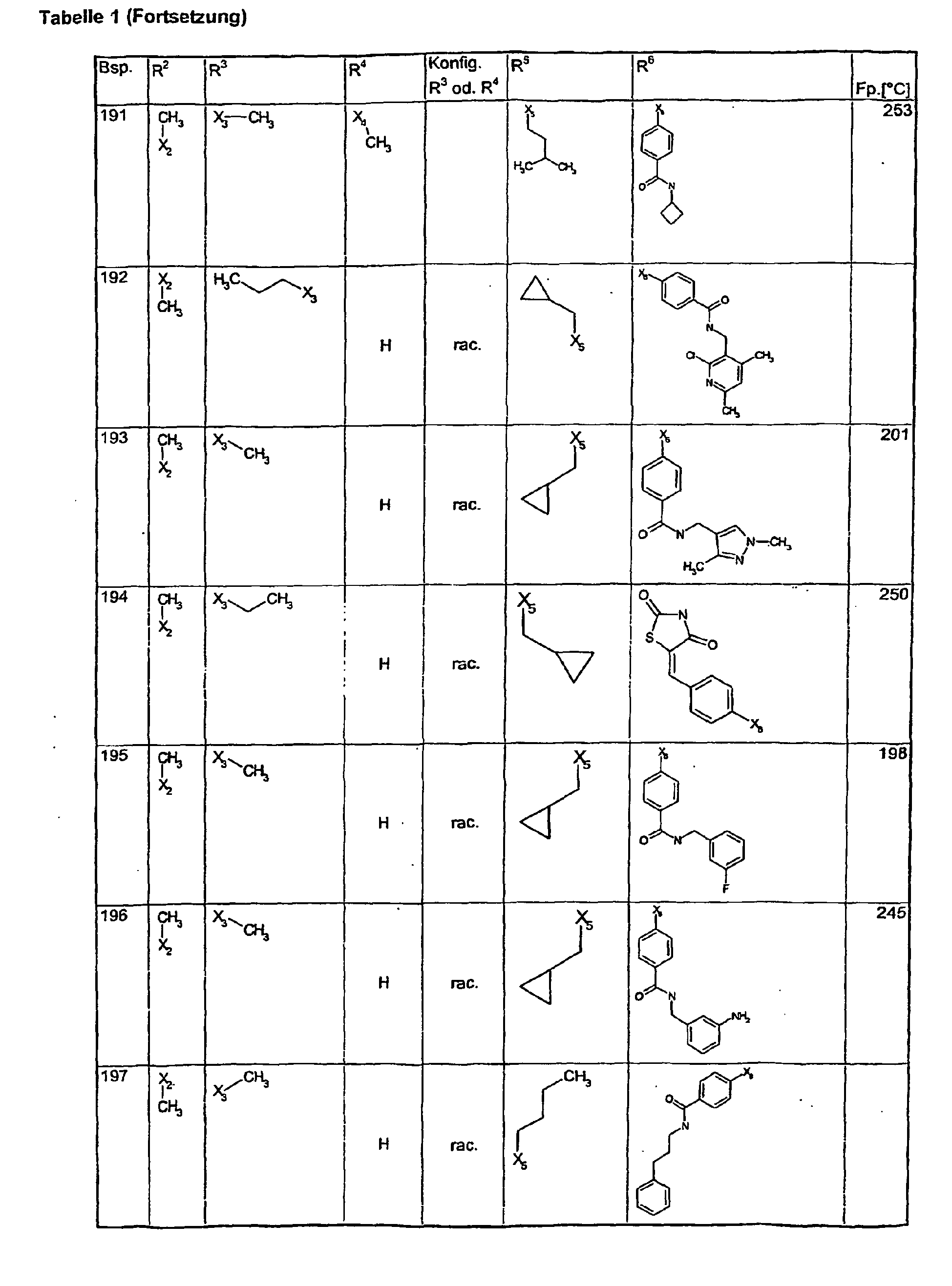

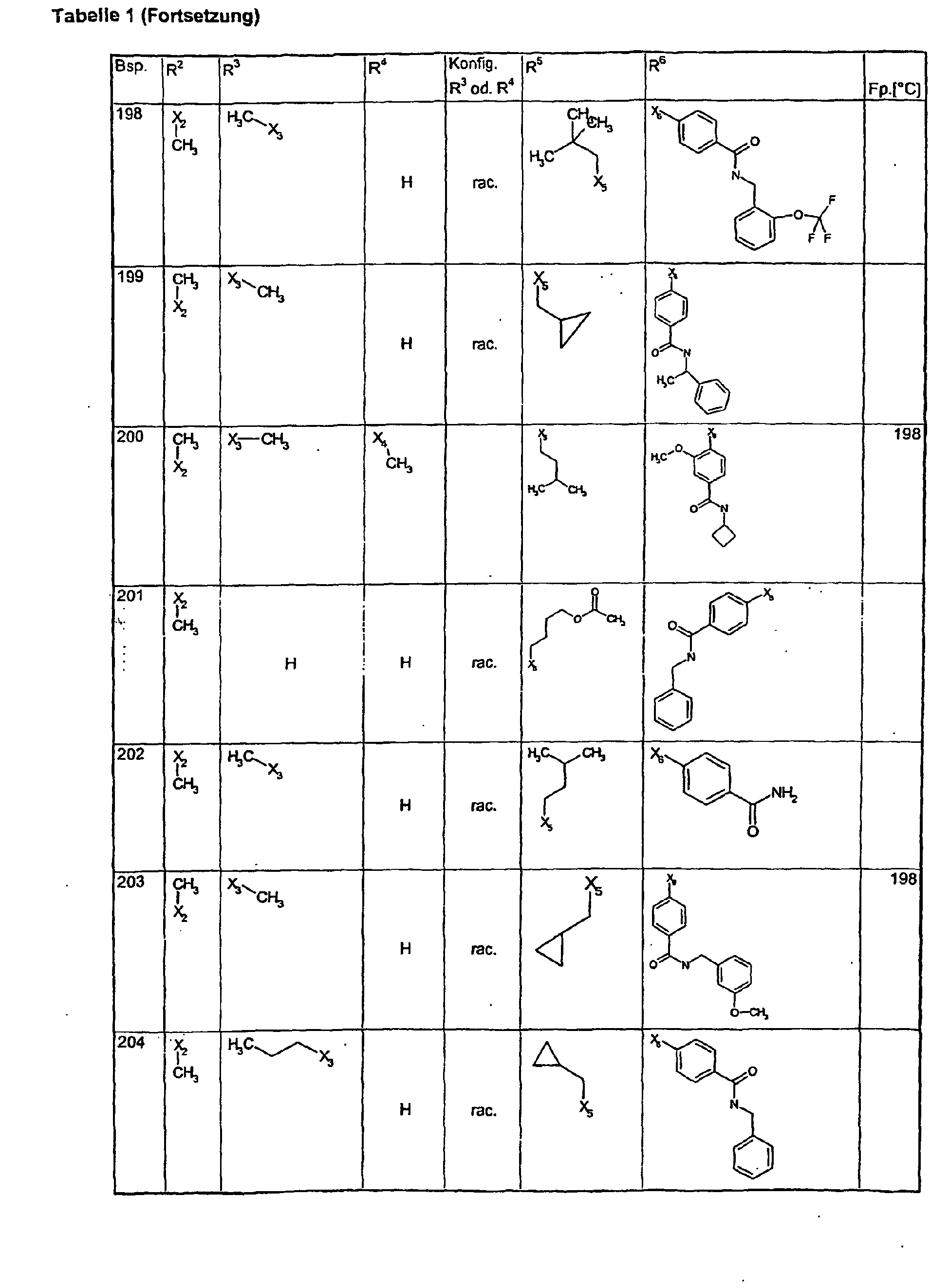

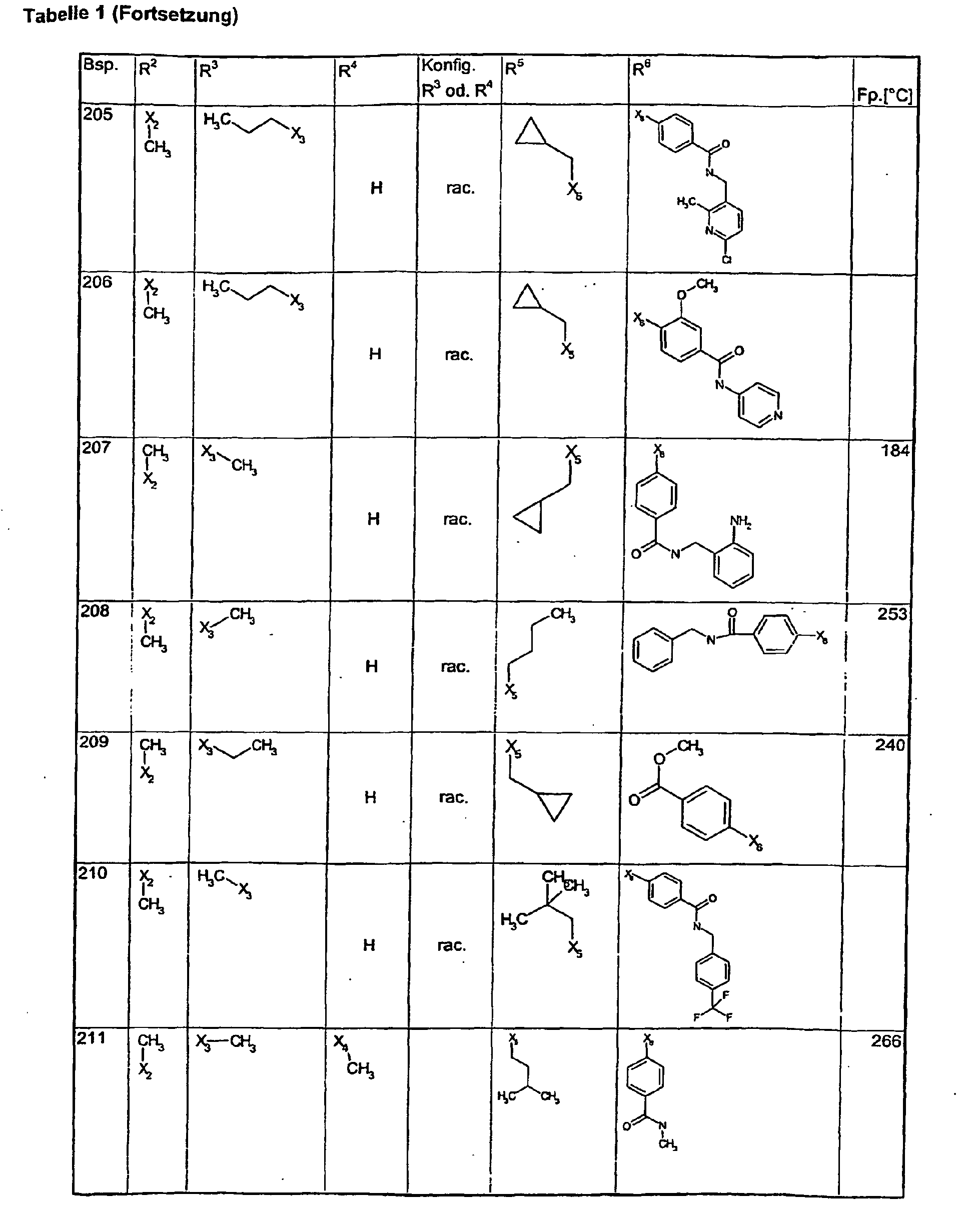

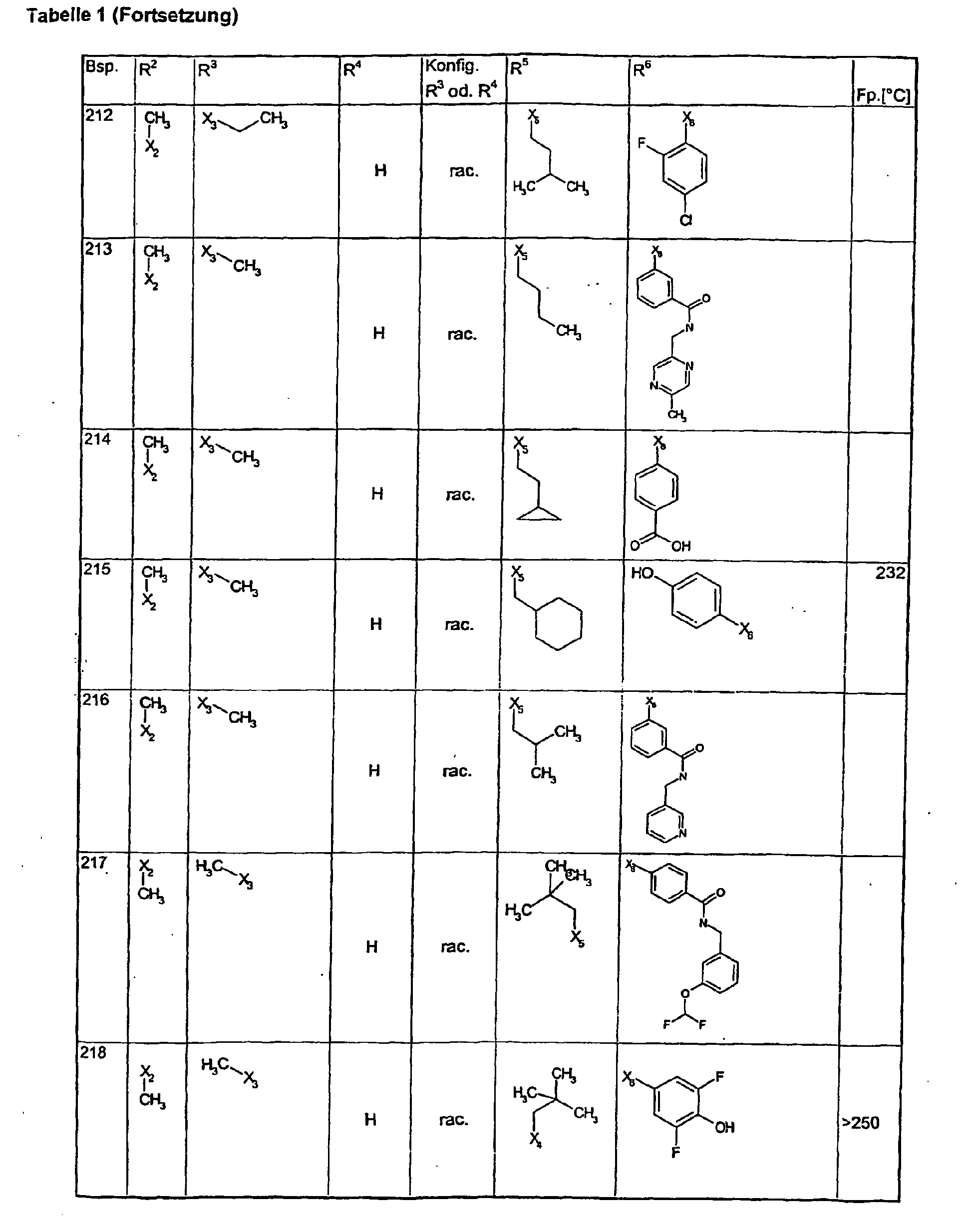

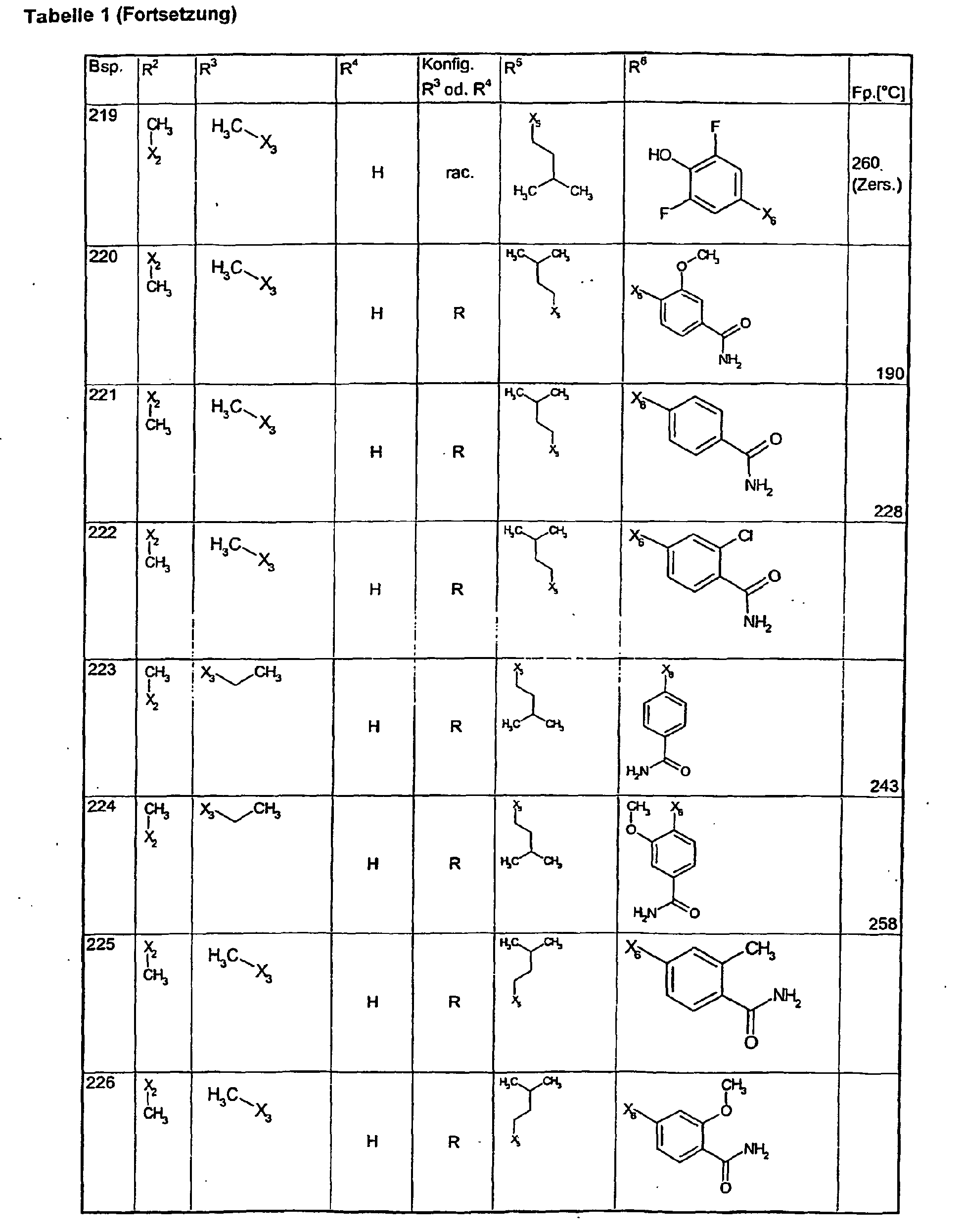

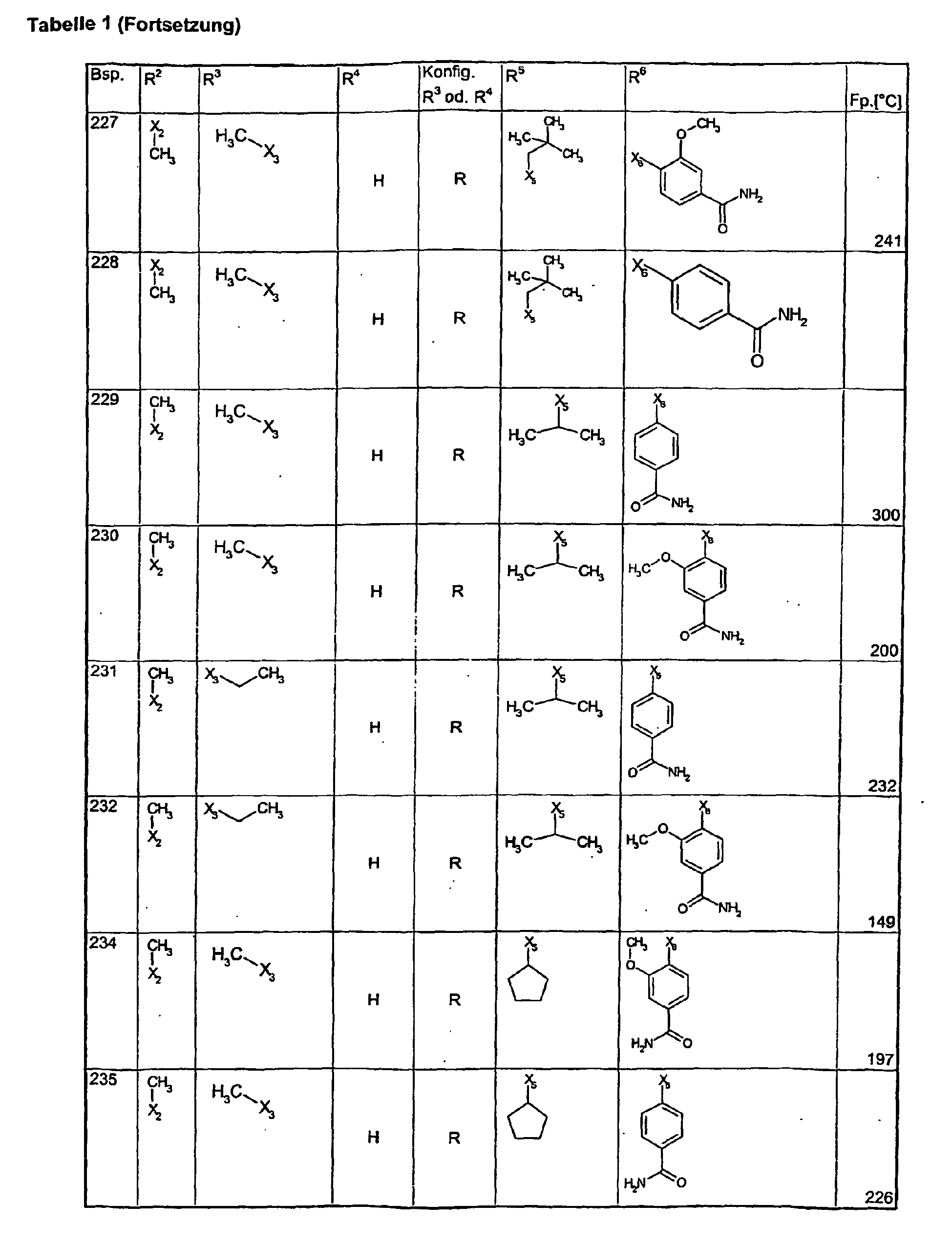

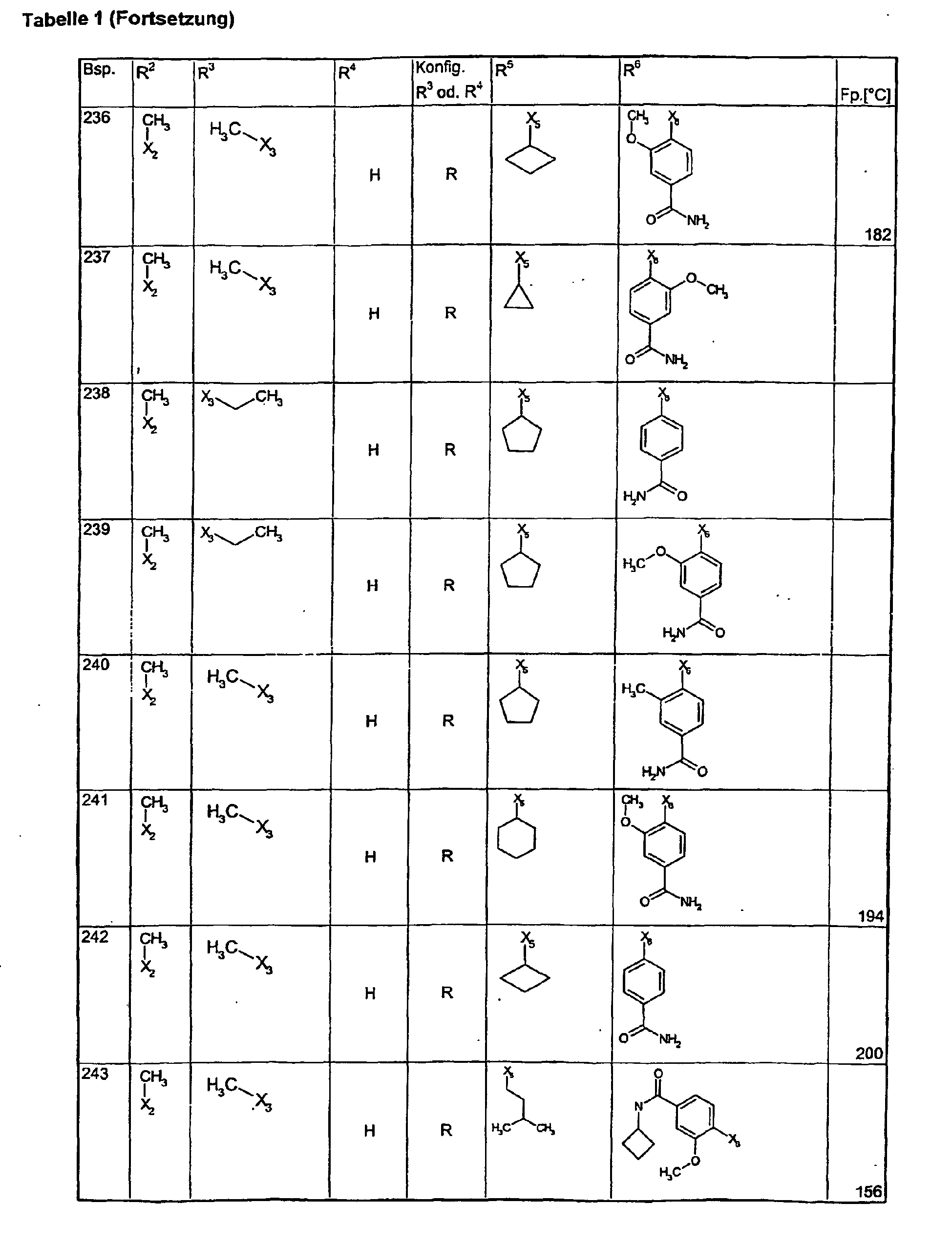

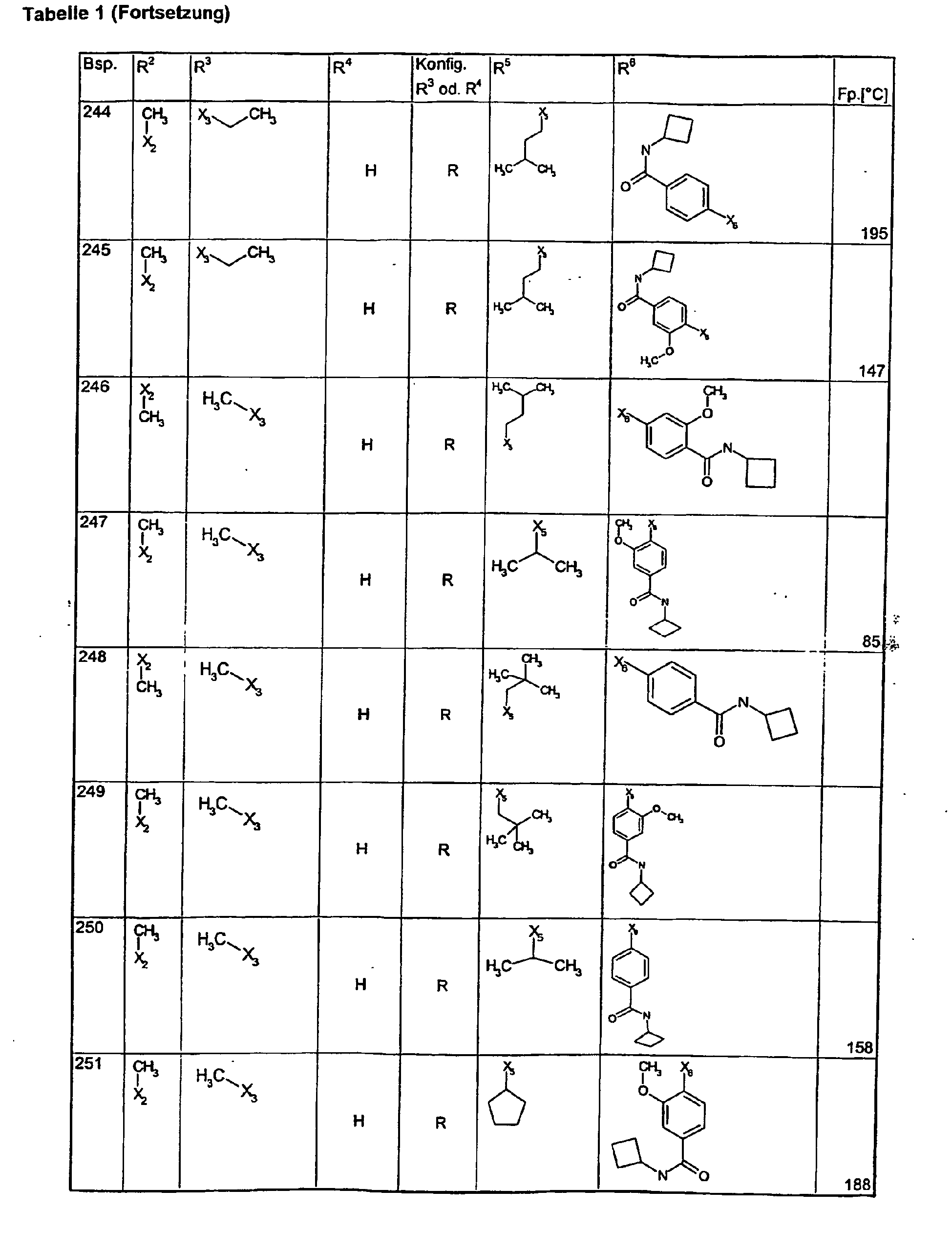

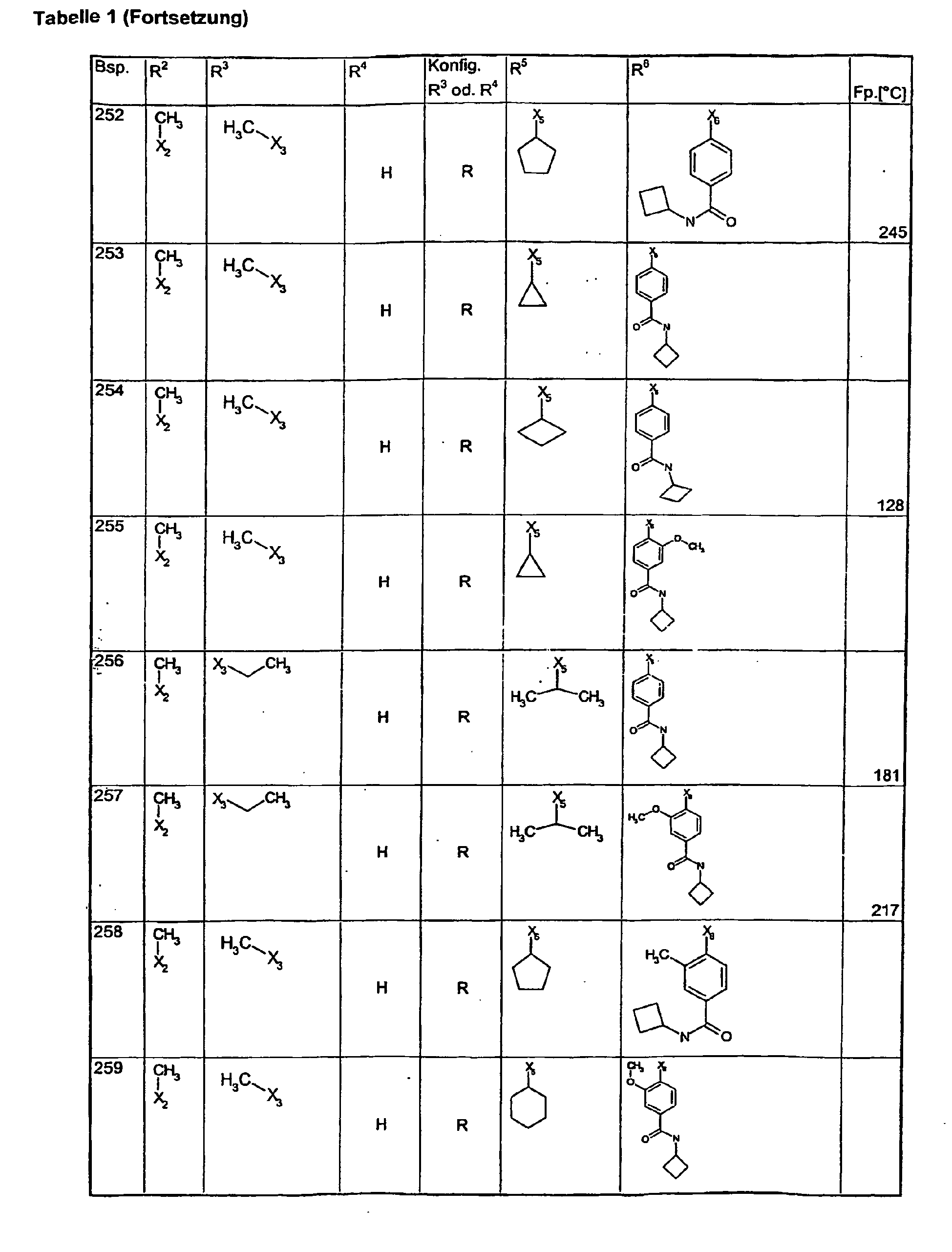

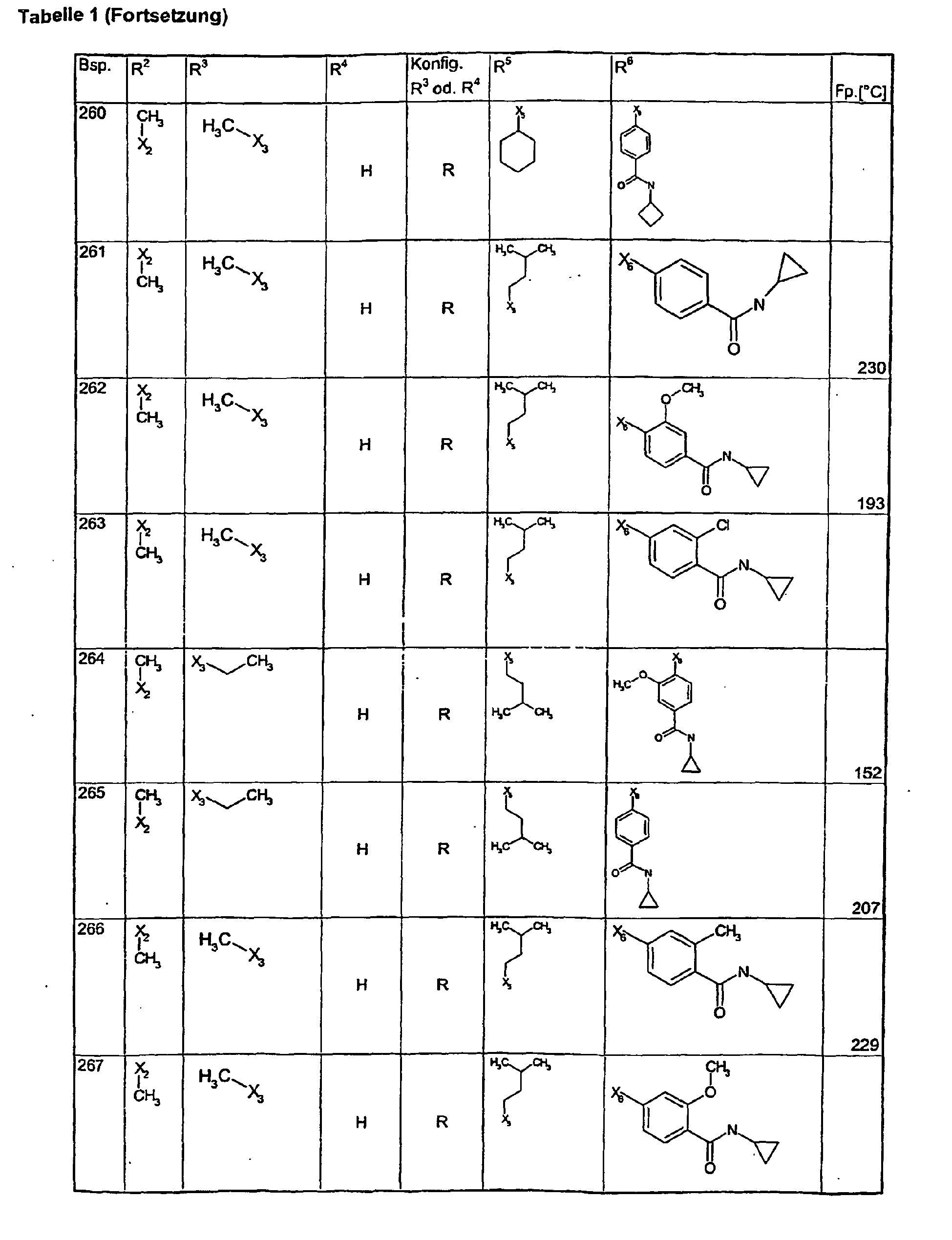

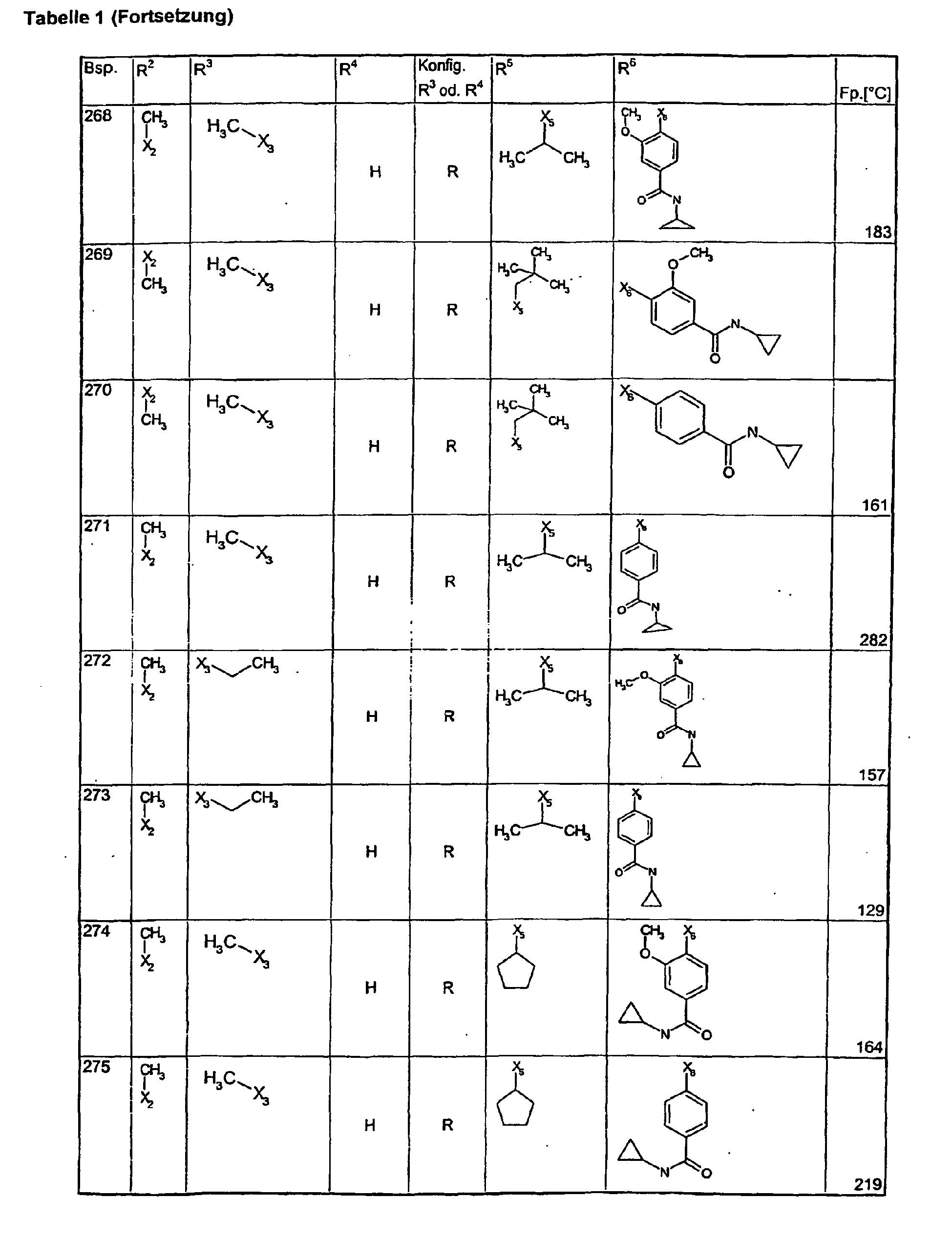

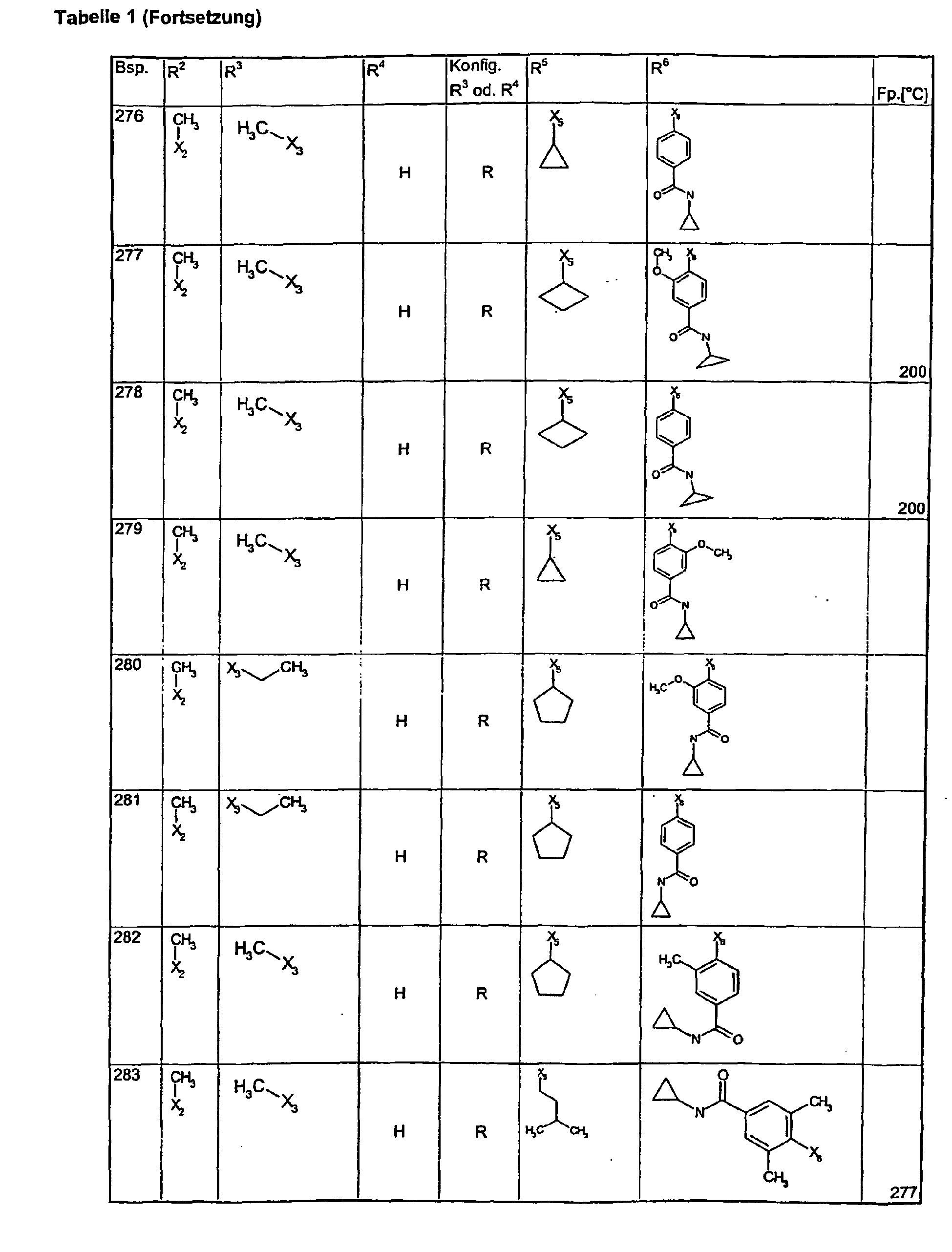

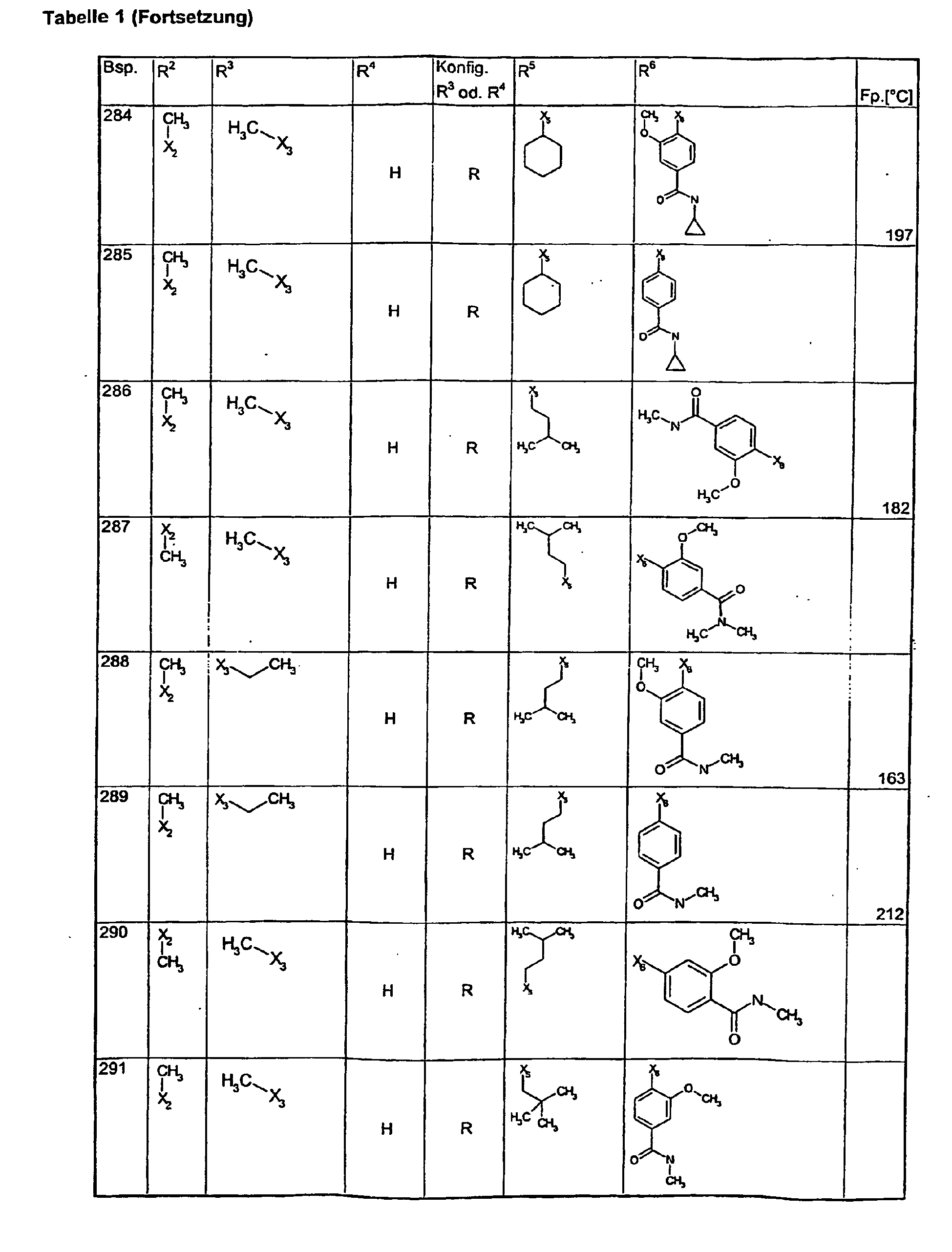

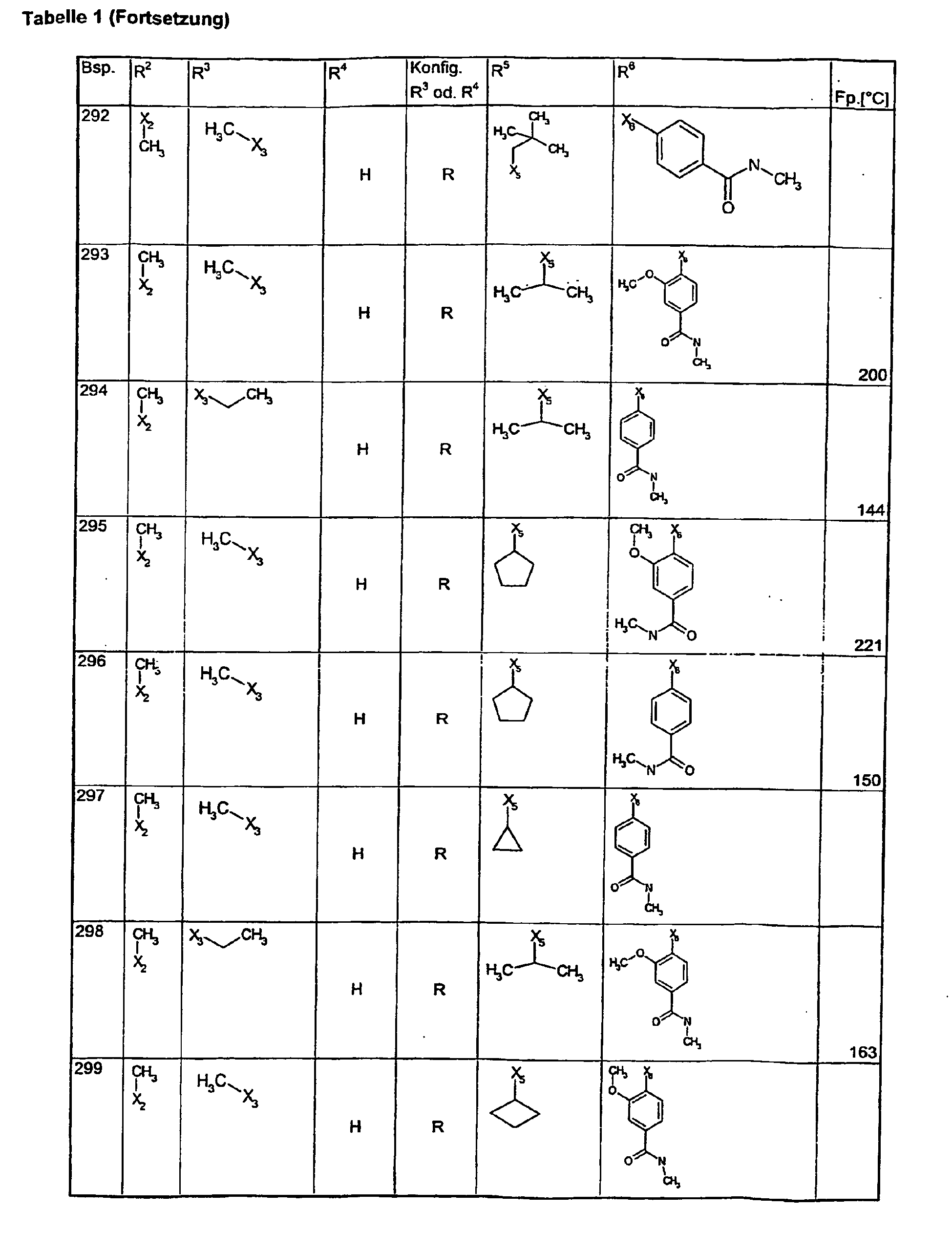

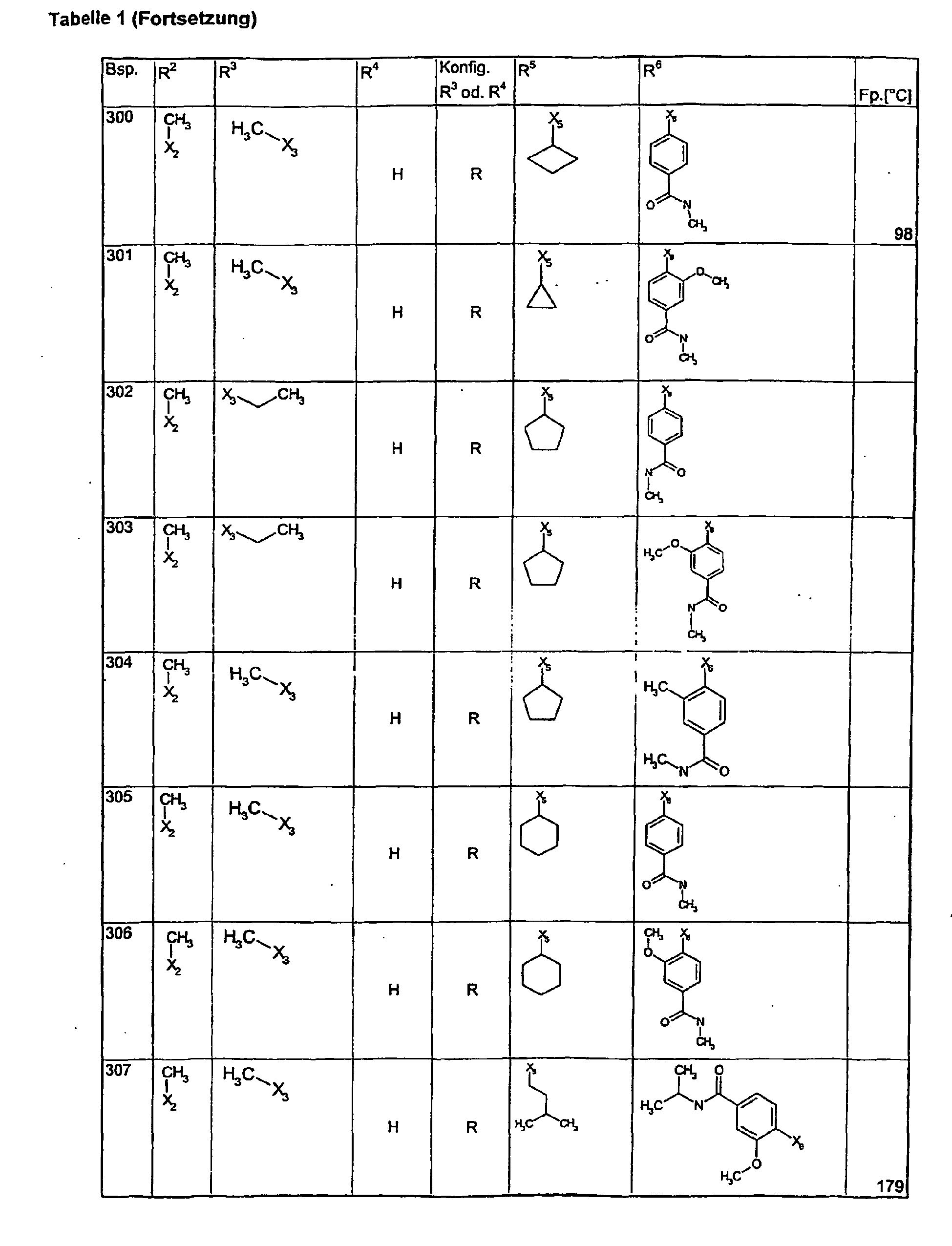

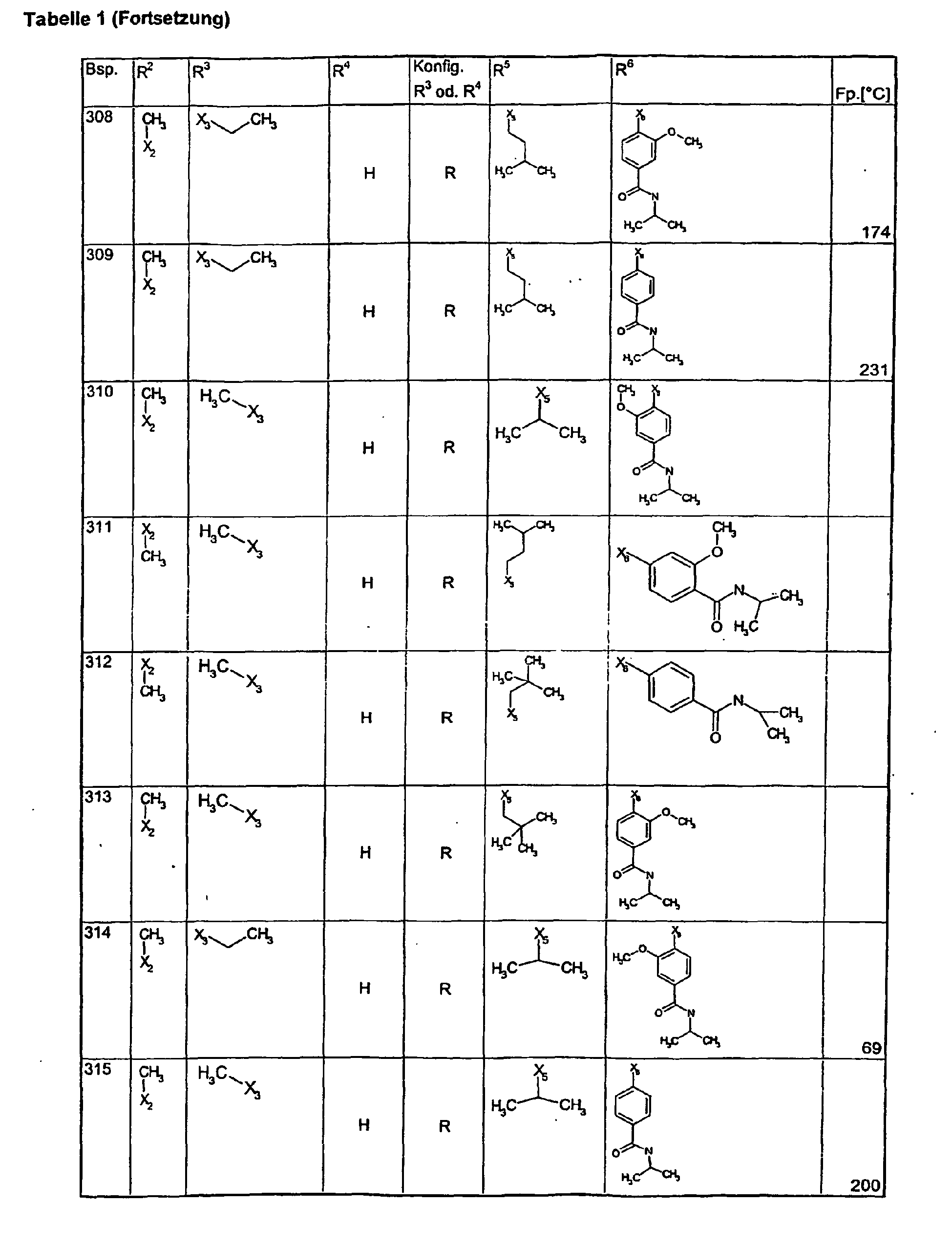

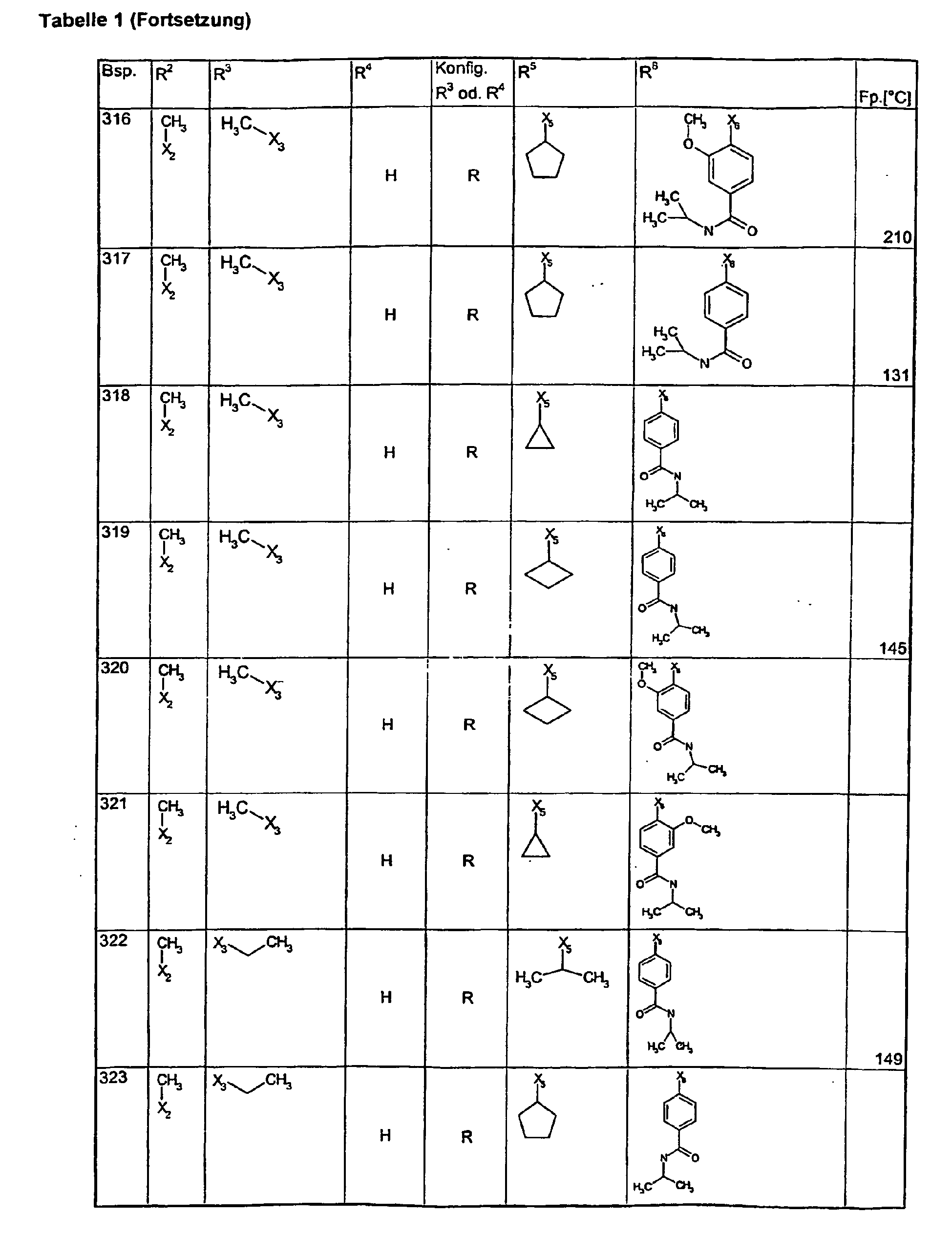

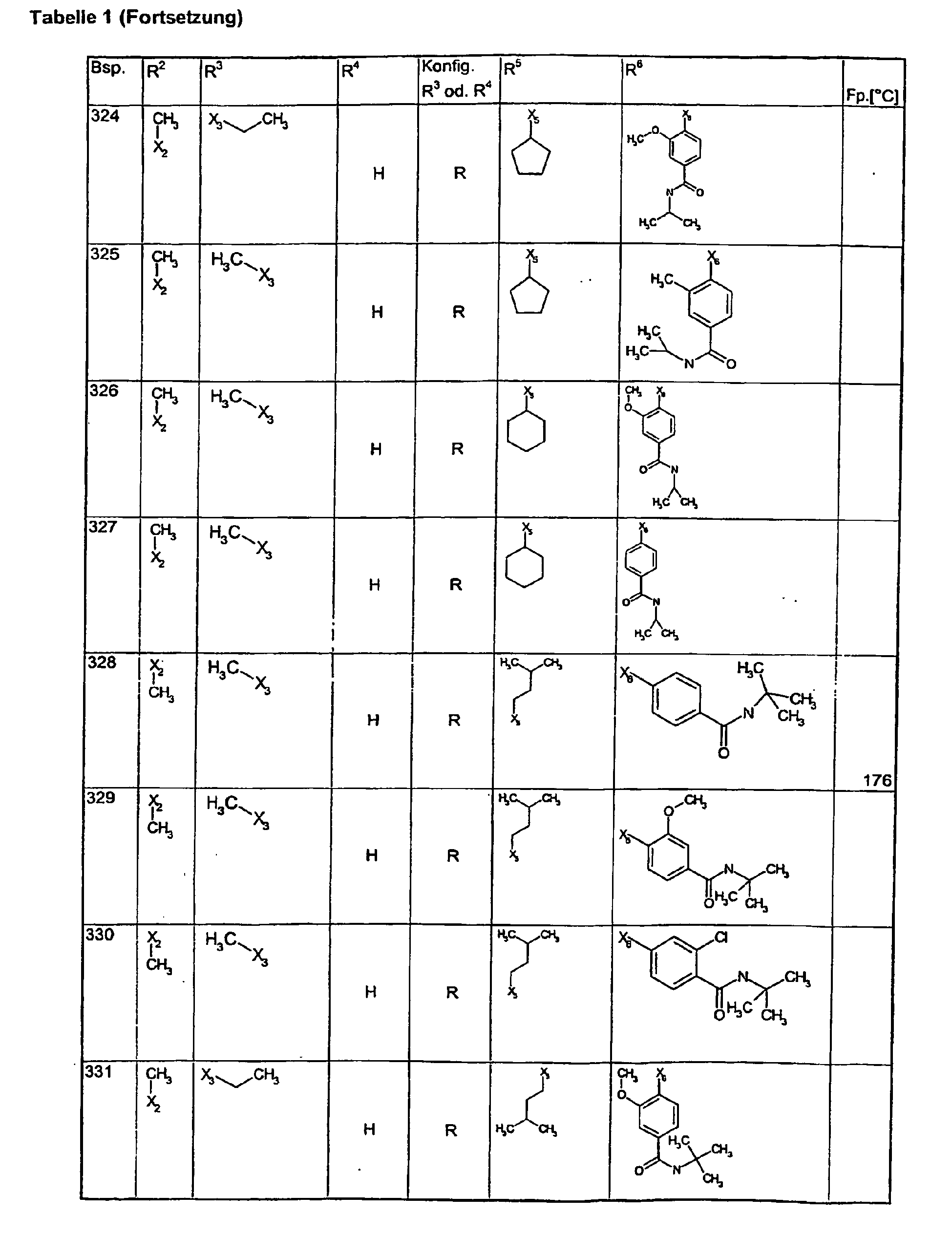

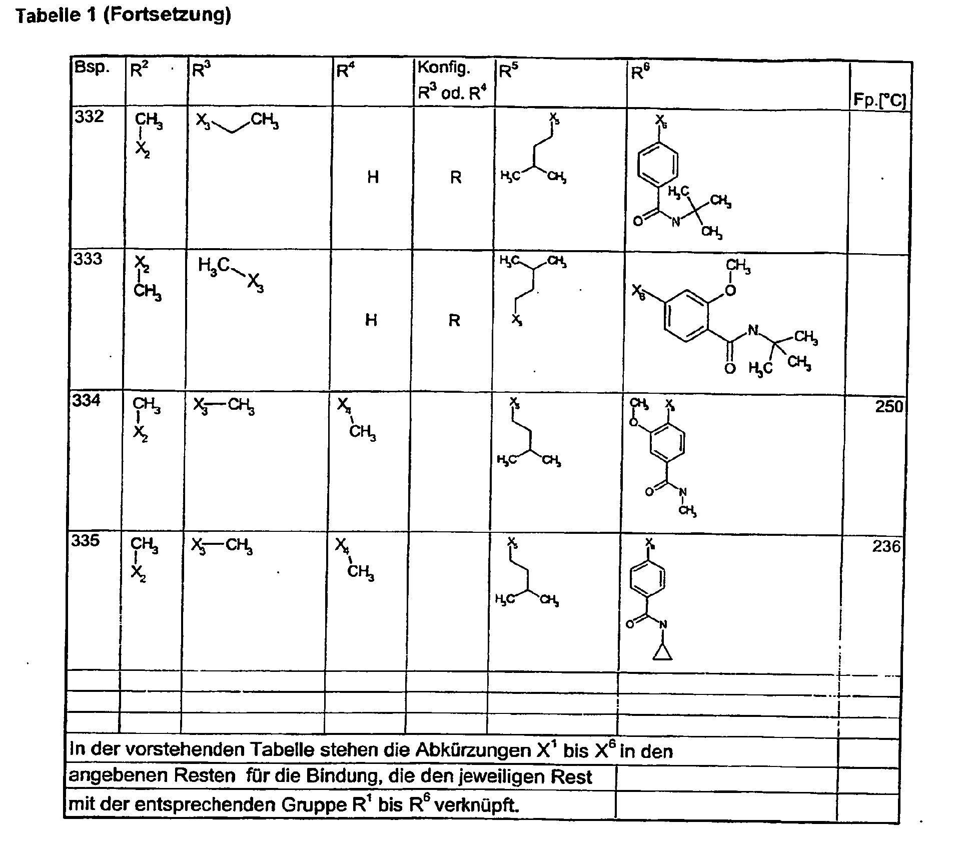

Wie gefunden wurde, zeichnen sich die Verbindungen der allgemeinen Formel (I) durch vielfältige Anwendungsmöglichkeiten auf therapeutischem Gebiet aus. Hervorzuheben sind solche Anwendungsmöglichkeiten, für welche die Inhibition spezifischer Zellzykluskinasen, insbesondere die inhibierende Wirkung auf die Proliferation kultivierter humaner Tumorzellen, aber auch auf die Proliferation anderer Zellen, wie z.B. Endothelzellen, eine Rolle spielen.

Wie durch FACS Analyse gezeigt werden konnte, ist die, durch die erfindungsgemäßen Verbindungen bewirkte, Proliferationsinhibition vermittelt durch einen Arrest der Zellen vor allem in der G2/M Phase des Zellzyklus. Die Zellen arretieren abhängig von den verwendeten Zellen für eine bestimmte Zeitspanne in dieser Zellzyklus Phase, bevor der programmierte Zelltod eingeleitet wird. Ein Arrest in der G2/M Phase des Zellzyklus wird z.B. durch die Inhibition spezifischer Zellzykluskinasen ausgelöst. Studien in Modellorganismen wie *Schizosaccharomyces pombe* oder *Xenopus,* oder Untersuchungen in menschlichen Zellen haben gezeigt, das der Übergang von der G2 Phase zur Mitose durch die CDK1/Cyclin B Kinase reguliert wird (Nurse, 1990). Diese Kinase, die auch als "mitosis promoting factor" (MPF) bezeichnet wird, phosphoryliert und reguliert dadurch eine Vielzahl von Proteinen, wie z.B. nukleäre Lamine, Kinesin-ähnliche Motorproteine, Kondensine und Golgi Matrix Proteine, die eine wichtige Rolle beim Abbau der Kemhülle, bei der Centrosomen Separation, dem Aufbau des mitotischen Spindelapparates, der Chromosomen Kondenation und Abbau des Golgi Apparates, spielen (Nigg. E., 2001). Eine murine Zell-Linie mit einer temperatursensitiven CDK1 Kinasemutante zeigt nach Temperaturerhöhung einen raschen Abbau der CDK1-Kinase und einen darauffolgenden Arrest in der G2/M Phase (Th'ng et al., 1990). Die Behandlung von humanen Tumorzellen mit Inhibitoren gegen CDK1/Cyclin B wie z.B. Butyrolacton führt ebenfalls zu einen Arrest in der G2/M Phase und anschließender Apoptose (Nishio, et al. 1996). Eine weitere Kinase, die in der G2 und Mitose Phase eine Rolle spielt, ist Polo-like Kinase 1 (PIk1), die für die Reifung der Centrosomen, für die Aktivierung der Phosphatase Cdc25C, sowie für die Aktivierung des Anaphase Promoting Complex verantwortlich ist (Glover et al., 1998, Qian, et al., 2001). Die Injektion von Plk1 Antikörpern führt zu einem G2 Arrest in nicht transformierten Zellen während Tumorzellen in der Mitosephase arretieren (Lane und Nigg, 1996). Darüber hinaus wurde auch für die Proteinkinase aurora B eine essentielle Funktion beim Eintritt in die Mitose beschrieben. Aurora B phosphoryliert Histon H3 an Ser11 und leitet damit die Chromosomenkondensation ein (Hsu, J.Y. et al., 2000). Ein spezifischer Zellzyklusarrest in der G2/M Phase kann aber auch z.B. durch Inhibition von spezifischen Phosphatasen wie z.B. Cdc25C (Russell and Nurse, 1986) ausgelöst werden. Hefen mit defektem cdc25 Gen arretieren in der G2 Phase, während eine Überexpression von cdc25 zu einem verfrühten Eintritt in die Mitosephase führt (Russell und Nurse, 1987). Ein Arrest in der G2/M Phase kann aber auch durch Inhibition von bestimmten Motorproteinen, sogenannten Kinesinen wie z.B. Eg5 (Mayer et al., 1999), oder durch Mikrotubuli stabilisierende oder destabilisierende Agentien (z.B. Colchicin, Taxol, Etoposid, Vinblastin, Vincristin) ausgelöst werden (Schiff und Horwitz, 1980).

Auf Grund ihrer biologischen Eigenschaften eignen sich die erfindungsgemäßen Verbindungen der allgemeinen Formel I, deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphoma und solide Tumore; HautErkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut- und Progenitor-Zellen) gegen DNA-Schädigung durch Strahlung, UV-Behandlung und/oder zytostatischer Behandlung (Davis et al., 2001).
Die neuen Verbindungen können zur Prävention, Kurz- oder Langzeitbehandlung der vorstehend genannten Erkrankungen, auch in Kombination mit anderen Wirkstoffen, die für dieselben Indikationen Verwendung finden, z.B. Cytostatika, verwendet werden.

Die Wirkung der erfindungsgemäßen Verbindungen wurde im Cytotoxizitätstest an kultivierten humanen Tumorzellen und/oder in einer FACS-Analyse, beispielsweise an HeLaS3-Zellen, bestimmt. Die Verbindungen zeigten in beiden Testmethoden eine gute bis sehr gute Wirksamkeit, d.h. beispielsweise einen EC₅₀-Wert im HeLaS3-Cytotoxizitätstest kleiner 5 µmol, in der Regel kleiner **1** µmol.

### Messung der Cytotoxizität an kultivierten humanen Tumorzellen

Zur Messung der Cytotoxizität an kultivierten humanen Tumorzellen wurden Zellen der zervikalen Carcinoma Tumorzell-Linie HeLaS3 (erhalten von American Type Culture Collection (ATCC)) in Ham's F12 Medium (Life Technologies) und 10% fötalem Rinderserum (Life Technologies) kultiviert und in der log-Wachstumsphase geerntet. Anschließend wurden die HeLaS3 Zellen in 96-well Platten (Costar) mit einer Dichte von 1000 Zellen pro well eingebracht und Ober Nacht in einem Inkubator (bei 37°C und 5 % CO₂) inkubiert, wobei auf jeder Platte 6 wells nur mit Medium gefüllt wurden (3 wells zur Mediumkontrolle, 3 wells zur Inkubation mit reduzierten AlamarBlue). Die Wirksubstanzen wurden in verschiedenen Konzentrationen (gelöst in DMSO; Endkonzentration: 1 %) zu den Zellen zugegeben (jeweils als Dreifachbestimmung) Nach 72 Stunden Inkubation wurden zu jeden well 20 µl AlamarBlue (AccuMed Intemational) zugesetzt, und die Zellen für weitere 7 Stunden inkubiert. Zur Kontrolle wurde zu 3 wells je 20 µl reduziertes Alamar Blue gegeben (AlamarBlue Reagenz, das für 30 min autoklaviert wurde). Nach 7 h Inkubation wurde der Farbumsatz des AlamarBlue Reagenz in den einzelnen wells in einem Perkin Elmer Fluoreszenzspektrophotometer bestimmt (Exitation 530 nm, Emission 590 nm, Slits 15, Integrate time 0.1). Die Menge an umgesetzten AlamarBlue Reagenz repräsentiert die metabolische Aktivität der Zellen. Die relative Zellaküvität wurde in Prozent der Kontrolle (HeLa S3 Zellen ohne Inhibitor) berechnet und die Wirkstoffkonzentration, die die Zellaktivität zu 50% hemmt (IC⁵⁰) abgeleitet. Die Werte wurden hierbei aus dem Mittelwert von drei Einzelbestimmungen - unter Korrektur des Leerwertes (Mediumkontrolle)- berechnet.

### FACS- Analyse

Propidium lodid (PI) bindet stöchiometrisch an doppelsträngige DNA, und ist damit geeignet den Prozentsatz an Zellen in der G1, S, und G2/M Phase des Zellzykluses auf der Basis des zellulären DNA Gehaltes zu bestimmen. Zellen in der G0 und G1 Phase haben einen diploiden DNA Gehalt (2N), während Zellen in der G2 oder Mitose einen 4N DNA Gehalt haben.
Für eine PI-Färbung wurden beispielsweise 0.4 Mio HeLaS3 Zellen auf eine 75 cm² Zellkulturflasche ausgesät, nach 24 h wurde entweder 1 % DMSO als Kontrolle zugesetzt, bzw. die Substanz in verschiedenen Konzentrationen (in 1 % DMSO). Die Zellen wurden für 24 h mit der Substanz bzw. mit DMSO inkubiert, bevor die Zellen mit 2 x PBS gewaschen und mit Trypsin /EDTA abgelöst wurden. Die Zellen wurden zentrifugiert (1000 Upm, 5 min, 4°C), und das Zellpellet 2 x mit PBS gewaschen, bevor die Zellen in 0.1 ml PBS resuspendiert wurden. Anschließend wurden die Zellen für 16 Stunden bei 4°C oder alternativ für 2 Stunden bei -20°C mit 80% Ethanol fixiert. Die fixierten Zellen (10⁶ Zellen) wurden zentrifugiert (1000 Upm, 5min, 4°C), mit PBS gewaschen und anschließend nochmals zentrifugiert . Das Zellpellet wurde in 2 ml Triton X-100 in 0.25 % PBS resuspendiert, und 5 min auf Eis inkubiert, bevor 5 ml PBS zugeben wurden und erneut zentrifugiert wurde. Das Zellpellet wurde in 350 µl Pl Färbelösung (0.1 mg/ml RNase A, 10 µg/ml Prodium lodid in 1 x PBS) resuspendiert. Die Zellen wurden für 20 min im Dunkeln mit dem Färbepuffer inkubiert, bevor sie in Probenmessgefäße für das FACS Scan überführt werden. Die DNA Messung erfolgte in einem Becton Dickinson FACS Analyzer, mit einen Argonlaser (500 mW, Emission 488 nm), und dem DNA Cell Quest Programm (BD). Die logarithmische PI Fluoreszenz wurde mit einem band-pass Filter (BP 585/42) bestimmt. Die Quantifizierung der Zellpopulationen in den einzelnen Zellzyklusphasen erfolgte mit dem ModFit LT Programm von Becton Dickinson.

Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 - 90 Gew.-%, bevorzugt 0,5 - 50 Gew.-% der Gesamtzusammensetzung liegen, d.h. in Mengen die ausreichend sind, um den unten angegebenen Dosierungsbereich zu erreichen. Die genannten Dosen können, falls erforderlich, mehrmals täglich gegeben werden.
Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kemen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kem auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbessemdes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägem, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Neutratfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserem oder Farbstoffen versetzt werden.
Für den Fall der parenteralen Anwendung können Lösungen der Wirkstoffe unter Verwendung geeigneter flüssiger Trägermaterialien eingesetzt werden.
Die Dosierung für die intravenöse Anwendung liegt bei 1 - 1000 mg pro Stunde, vorzugsweise zwischen 5 - 500 mg pro Stunde.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über der Tag zu verteilen.

Die nachfolgenden Formulierungsbeipiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff | 50 mg |
| | Natriumchlorid | 50 mg |
| | Aqua pro inj. | 5 ml |

Der Wirkstoff wird bei Eigen-pH oder gegebenenfalls bei pH 5,5 - 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel (I), 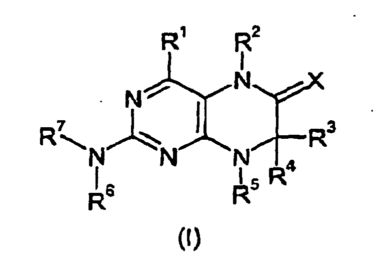 worin
R¹ ein Rest ausgewählt aus der Gruppe bestehend aus Wasserstoff, NH₂, XH, Halogen und einer gegebenenfalls durch ein oder mehrere Halogenatome substituierten C₁-C₃-Alkyl-Gruppe,
R² ein Rest ausgewählt aus der Gruppe bestehend aus Wasserstoff, CHO, XH, -X-C₁-C₂-Alkyl und einer gegebenenfalls substituierten C₁-C₃-Alkyl-Gruppe,
R³, R⁴ gleich oder verschieden, ein Rest ausgewählt aus der Gruppe bestehend aus gegebenenfalls substituiertem C₁-C₁₀-Alkyl, C₂-C₁₀-Alkenyl, C₂-C₁₀-Alkinyl, Aryl, Heteroaryl, C₃-C₈-Cycloalkyl, -X-Aryl, -X-Heteroaryl, X-Cycloalkyl, -NR⁸-Aryl, -NR⁸-Heteroaryl, -NR⁸-Cycloalkyl, oder -NR⁸-Heterocycloalkyl, X-Heterocycloalkyl, C₃-C₈- Heterocycloalkyl, oder ein Rest ausgewählt aus der Gruppe bestehend aus Wasserstoff, Halogen, COXR⁸, CON(R⁸)₂, COR⁸ und XR⁸, oder
R³ und R⁴ gemeinsam eine 2- bis 5-gliedrige Alkylbrücke, die 1 bis 2 Heteroatome enthalten kann,
R⁵ Wasserstoff oder ein Rest ausgewählt aus der Gruppe bestehend aus gegebenenfalls substituiertem C₁-C₁₀-Alkyl, C₂-C₁₀-Alkenyl, C₂-C₁₀-Alkinyl, Aryl, Heteroaryl und -C₃-C₆-Cycloalkyl, oder
R³ und R⁵ oder R⁴ und R⁵ gemeinsam eine gesättigte oder ungesättigte C₃-C₄-Alkylbrücke, die 1 bis 2 Heteroatome enthalten kann,
R⁶ gegebenenfalls substituiertes Aryl oder Heteroaryl,
R⁷ Wasserstoff oder -CO-X-C₁-C₄-Alkyl, und
X jeweils unabhängig voneinander, O oder S,
R⁸ jeweils unabhängig voneinander, Wasserstoff oder ein Rest ausgewählt aus der Gruppe bestehend aus gegebenenfalls substituiertem C₁-C₄-Alkyl, C₂-C₄-Alkenyl, C₂-C₄-Alkinyl und Phenyl.
wobei, soweit nichts anderes erwähnt wurde,
die Substituenten der gegebenenfalls substituierten Alkylgruppen ausgewählt sind aus der Gruppe bestehend aus den Halogenatomen Fluor, Chlor, Brom oder lod, einem Rest ausgewählt aus der Gruppe bestehend aus -CN, -OCOCH₃, Aryl, Heteroaryl, gesättigtes oder ungesättigtes Heterocycloalkyl, einem Aminrest, einen gesättigten oder ungesättigten bicyclischen Ringsystem,
die Substituenten der gegebenenfalls substituierten Alkenylgruppen ausgewählt sind aus der Gruppe bestehend aus den Halogenatomen Fluor, Chlor, Brom oder lod,
die Substituenten der gegebenenfalls substituierten Alkinylgruppen ausgewählt sind aus der Gruppe bestehend aus den Halogenatomen Fluor, Chlor, Brom oder lod,
die Substituenten der gegebenenfalls substituierten Arylgruppen ausgewählt sind aus der Gruppe bestehend aus -OH, -NO₂, -CN, -OCHF₂, - OCF₃, -NH₂, Halogen, -C₁-C₁₀-Alkyl, -O-C₁-C₃-Alkyl, -N-methyl-tetrahydrooxazinyl, -COOH, -COO-C₁-C₄-Alkyl, -CONH₂, -CONH-C₁-C₁₀-Alkyl, -CONH-C₃-C₆-Cycloalkyl, -CONH-Heterocycloalkyl, -CONH-Heteroaryl, -CONH-Aryl, -CONMeC₁-C₃-Alkyl, Benzimidazol oder einen Rest der Formel 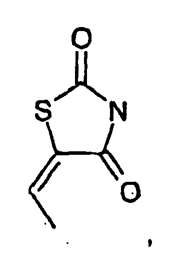 die Substituenten der gegebenenfalls substituierten Heteroarylgruppe ausgewählt sind aus der Gruppe bestehend aus -OH, -NO₂, -CN, -OCHF₂, -OCF₃, -NH₂, Halogen, -C₁-C₁₀-Alkyl, -O-C₁-C₃-Alkyl, -N-methyl-tetrahydrooxazinyl, -COOH, -COO-C₁-C₄-Alkyl, -CONH₂, Phenyl, Heteroaryl, -CONH-C₁-C₁₀-Alkyl, -CONH-C₃-C₈-Cycloalkyl, -CONH-Heteroaryl, -CONH-Aryl, -CONMeC₁-C₃-Alkyl, Benzimidazol oder einen Rest der Formel 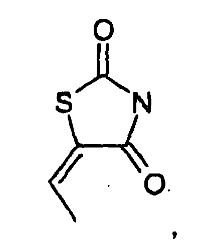 die Substituenten der gegebenenfalls substituierten Cycloalkylgruppe ausgewählt sind aus der Gruppe bestehend aus einem oder mehreren über eine Doppelbindung verknüpfte Sauerstoffatome, wobei die Cycloalkylgruppe auch an einen Benzolring anneliert sein kann,
und wobei, soweit nichts anderes erwähnt wurde,
unter dem voranstehend in den Definitionen erwähnten Ausdruck "Aryl" ein aromatisches Ringsystem mit 6 bis 14 Kohlenstoffatomen zu verstehen ist,
unter dem voranstehend in den Definitionen erwähnten Ausdruck "Heteroaryl" ein 5-10-gliedriger mono- oder bicyclischer Heteroarylring, in dem bis zu drei C-Atome durch ein oder mehrere Hetaroatome ausgewählt aus der Gruppe Sauerstoff, Stickstoff oder Schwefel ersetzt sein kann, zu verstehen ist,
unter dem voranstehend in den Definitionen erwähnten Ausdruck "Cycloalkyl" ein gesättigter oder ungesättigter Cycloalkylrest mit 3 - 8 Kohlenstoffatomen zu verstehen ist,
unter dem voranstehend in den Definitionen erwähnten Ausdruck "Heterocycloalkyl" eln 5- ,6- oder 7-gliedriger, gesättigter oder ungesättigter Heterocyclus, der als Heteroatom Stickstoff. Sauerstoff oder Schwefel enthalten kann, wobei jeder der vorstehend genannten Heterocyclen gegebenenfalls ferner an einen Benzolring anneliert sein kann, zu verstehen ist.
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

2. Verbindungen nach Anspruch 1, worin
X und R⁶ die angegebene Bedeutung aufweisen, und
R¹ Wasserstoff,
R² ein Rest ausgewählt aus der Gruppe bestehend aus einer CHO, OH, und CH₃₋Gruppe,
R³, R⁴ gleich oder verschieden, ein Rest ausgewählt aus der Gruppe bestehend aus Wasserstoff, gegebenenfalls substituiertem C₁-C₆-Alkyl, C₂-C₆-Alkenyl, C₂-C₆-Alkinyl, C₃-C₇-Cycloalkyl, oder
R³ und R⁴ gemeinsam eine C₂-C₅-Alkylbrücke,
R⁵ ein Rest ausgewählt aus der Gruppe bestehend aus gegebenenfalls substituiertem C₁-C₁₀-Alkyl, C₂-C₁₀-Alkenyl, C₂-C₁₀-Alkinyl, und C₃-C₆-Cycloalkyl, oder
R³ und R⁵ oder R⁴und R⁵ gemeinsam eine gesättigte oder ungesättigte C₃-C₄-Alkylbrücke, die 1 bis 2 Heteroatome enthalten kann, und
R⁷ Wasserstoff
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

3. Verbindungen nach Anspruch 1 oder 2, wobei
R¹-R⁵, R⁷, R⁸ und X die angegebene Bedeutung aufweisen, und
R⁶ ein Rest der allgemeinen Formel 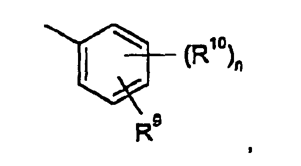
worin
n 1, 2, 3 oder 4.
R⁹ ein Rest ausgewählt aus der Gruppe bestehend aus gegebenenfalls substituiertern C₁-C₆-Alkyl, C₂-C₆-Alkenyl, C₂-C₆-Alkinyl, -CONH-C₁-C₁₀-Alkylen, -O-Aryl, -O-Heteroaryl, -O-Cycloalkyl, -O-Heterocycloalkyl, Aryl, Heteroaryl, Cycloalkyl und Heterocycloalkyl oder ein Rest ausgewählt aus der Gruppe bestehend aus -O-C₁-C₆-Alkyl-Q¹, -CONR⁸-C₁-C₁₀-Alkyl-Q¹, -CONR⁸-C₂-C₁₀-Alkenyl-Q¹, -CONR⁸-Q², Halogen, OH, -SO₂R⁸, -SO₂N(R⁸)₂, -COR⁸,-COOR⁸,-N(R⁸)₂, -NHCOR⁸, CONR⁸OC₁-C₁₀ AlkylQ¹ und CONR⁸OQ².
Q¹ Wasserstoff, -NHCOR⁸, oder ein Rest ausgewählt aus der Gruppe bestehend aus einer gegebenenfalls substituierten -NH-Aryl-, -NH-Heteroaryl-, Aryl-, Heteroaryl-, C₃-C₈-Cycloalkyl- und Heterocycloalkyl-Gruppe.
Q² Wasserstoff oder ein Rest ausgewählt aus der Gruppe bestehend aus einer gegebenenfalls substituierten Aryl-, Heteroaryl-, C₃-C₈-Heterocycloalkyl, C₃-C₈₋Cycloalkyl und C₁-C₄-Alkyl-C₃-C₈-cycloalkyl-Gruppe,
R¹⁰ gleich oder verschieden, ein Rest ausgewählt aus der Gruppe bestehend aus gegebenenfalls substituiertem C₁-C₆-Alkyl , C₂-C₆-Alkenyl und C₂-C₆-Alkinyl,
-O-C₁-C₆-Alkyl, -O-C₂-C₆-Alkenyl, -O-C₂-C₆-Alkinyl, C₃-C₆-Heterocycloalkyl und C₃-C₆-Cycloalkyl, oder
ein Rest ausgewählt aus der Gruppe bestehend aus Wasserstoff, -CONH₂,-COOR⁸, -OCON(R⁸)₂, -N(R⁸)₂, -NHCOR⁸, -NHCON(R⁸)₂, -NO₂ und Halogen, oder
benachbarte Reste R⁹ und R¹⁰ gemeinsam eine Brücke der allgemeinen Formel 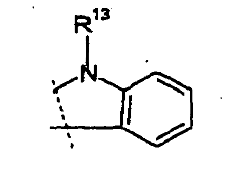 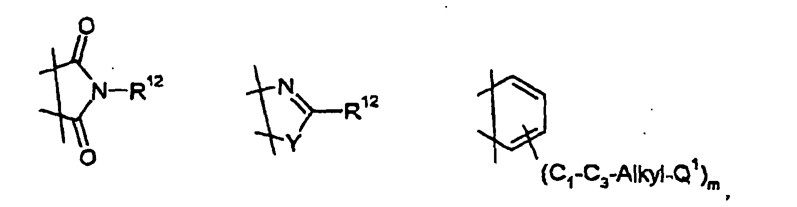
Y O, S oder NR¹¹,
m 0, 1 oder 2
R¹¹ Wasserstoff oder C₁-C₂-Alkyl, und
R¹² Wasserstoff oder ein Rest ausgewählt aus der Gruppe bestehend aus gegebenenfalls substituiertem Phenyl, Pyridyl, Pyrazinyl, Pyrimidinyl, Pyridazinyl, -C₁-C₃-Alkyl-Phenyl, -C₁-C₃-Alkyl-Pyridyl, -C₁-C₃-Alkyl-Pyrazinyl, -C₁-C₃-Alkyl-Pyrimidinyl und -C₁-C₃-Alkyl-Pyridazinyl,
R¹³ C₁-C₆-Alkyl,
bedeuten,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, bedeuten.

4. Verbindungen nach einem der Ansprüche 1 bis 3, worin
R³-R⁶, R⁸ und X die angegebene Bedeutung aufweisen, und
R¹ Wasserstoff,
R² CH₃, und
R⁷ Wasserstoff,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

5. Verbindung der Formel I nach einem der Ansprüche 1 bis 4 zur Verwendung als Arzneimittel.

6. Verbindung der Formel I nach einem der Ansprüche 1 bis 4 zur Verwendung als Arzneimittel mit antiproliferativer Wirkung.

7. Verwendung einer Verbindung der Formel I zur Herstellung eines Arzneimittels zur Behandlung und/oder Prävention von Krebs, Infektionen, Entründungs- und Autoimmunerkrankungen.

8. Pharmazeutische Zubereitungen, enthaltend als Wirkstoff eine oder mehrere Verbindungen der allgemeinen Formel (I) gemäß einem der Ansprüche 1 bis 4 oder deren physiologisch verträgliche Salze gegebenenfalls in Kombination mit üblichen Hilfs- und/oder Trägerstoffen.

9. Verfahren zur Herstellung einer Verbindung der allgemeinen Formel (I), 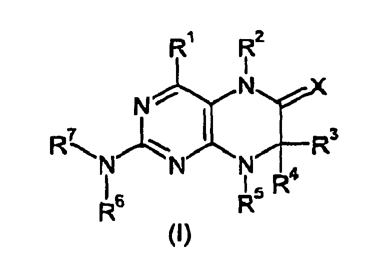 worin
R¹-R⁷ und X die in den Ansprüchen 1 bis 4 angegebene Bedeutung aufweisen, **dadurch gekennzeichnet, daß** eine Verbindung der allgemeinen Formel (II) 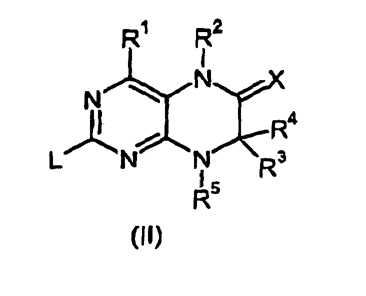 worin
R¹-R⁵ und X die in den Ansprüchen 1 bis 4 angegebene Bedeutung aufweisen und L eine Abgangsgruppe ist,
mit einer gegebenenfalls substituierten Verbindung der allgemeinen Formel (III) 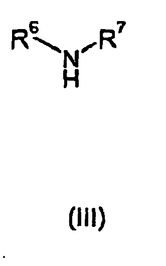 worin
R⁶ und R⁷ die in den Ansprüchen 1 bis 4 angegebene Bedeutung aufweisen, umgesetzt wird.

10. Verbindung der Formel (II), 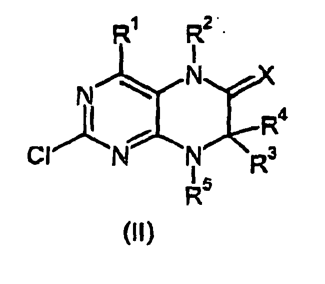 worin
R¹-R⁵ und X die in den Ansprüchen 1 bis 4 angegebene Bedeutung aufweisen.

11. Verfahren zur Herstellung einer Verbindung der allgemeinen Formel (I), 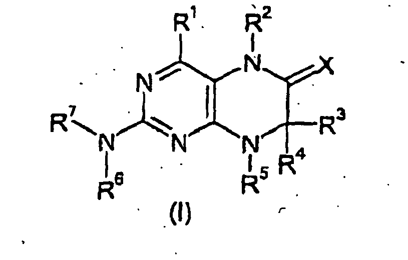 worin
R⁶ ein Rest der allgemeinen Formel, 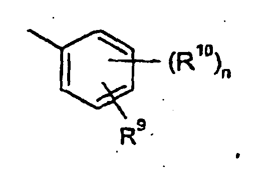 R⁹ ein gegebenenfalls substituierter Rest -CONH-C₁-C₁₀-Alkylen oder ein Rest ausgewählt aus der Gruppe bestehend aus -CONR⁸-C₁-C₁₀-Alkyl-Q¹, -CONR⁸-C₂-C₁₀-Alkenyl-Q¹, -CONR⁸-Q², und -COOR⁸ bedeutet, R¹-R⁵, R⁷, R¹⁰, n und X die in den Ansprüchen 1 bis 4 angegebene Bedeutung aufweisen, und R⁸ die in Anspruch 1 angegebene Bedeutung aufweist,
**dadurch gekennzeichnet, daß** eine Verbindung der allgemeinen Formel (IA) 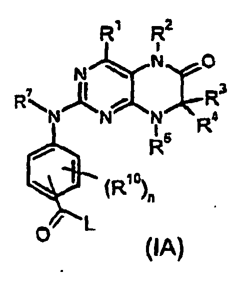 worin
R¹ bis R⁵, R⁷,R¹⁰ und n die in den Ansprüchen 1 bis 4 angegebene Bedeutung aufweisen, und
L eine Abgangsgruppe bedeutet,
mit einem primären oder sekundären Amin zu dem entsprechenden Amid oder mit einem Alkohol zu dem entsprechenden Ester umsetzt wird.

## Claims

1. Compounds of general formula (I), 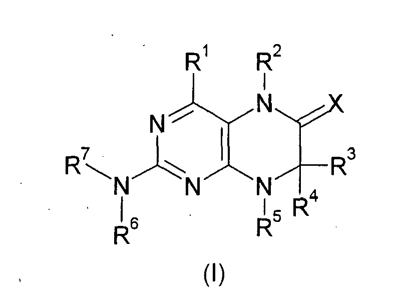 wherein
R¹ denotes a group selected from among hydrogen, NH₂, XH, halogen and a C₁-C₃-alkyl group optionally substituted by one or more halogen atoms,
R² denotes a group selected from among hydrogen, CHO, XH, -X-C₁-C₂₋alkyl and an optionally substituted C₁-C₃-alkyl group,
R³, R⁴ which may be identical or different denote a group selected from among optionally substituted C₁-C₁₀-alkyl, C₂-C₁₀-alkenyl, C₂-C₁₀₋alkynyl, aryl, heteroaryl, C₃-C₈-cycloalkyl, -X-aryl, -X-heteroaryl, -X-cycloalkyl, -NR⁸-aryl, -NR⁸-heteroaryl, -NR⁸-cycloalkyl, or -NR⁸⁻heterocycloalkyl, X-heterocycloalkyl, C₃-C₈-heterocycloalkyl, or a group selected from among hydrogen, halogen, COXR⁸, CON(R⁸)₂, COR⁸ and XR⁸, or
R³ and R⁴ together denote a 2- to 5-membered alkyl bridge which may contain 1 to 2 heteroatoms,
R⁵ denotes hydrogen or a group selected from among optionally substituted C₁-C₁₀-alkyl, C₂-C₁₀-alkenyl, C₂-C₁₀-alkynyl, aryl, heteroaryl and -C₃-C₆-cycloalkyl , or
R³ and R⁵ or R⁴ and R⁵ together denote a saturated or unsaturated C₃-C₄-alkyl bridge which may contain 1 to 2 heteroatoms,
R⁶ denotes optionally substituted aryl or heteroaryl,
R⁷ denotes hydrogen or -CO-X-C₁-C₄-alkyl, and
X in each case independently of one another denotes O or S,
R⁸ in each case independently of one another denotes hydrogen or a group selected from among optionally substituted C₁-C₄-alkyl, C₂-C₄-alkenyl, C₂-C₄-alkynyl and phenyl,
wherein, unless stated otherwise,
the substituents of the optionally substituted alkyl groups are selected from among the halogen atoms fluorine, chlorine, bromine or iodine, a group selected from among -CN, -OCOCH₃, aryl, heteroaryl, saturated or unsaturated heterocycloalkyl, an amine group, a saturated or unsaturated bicyclic ring system,
the substituents of the optionally substituted alkenyl groups are selected from among the halogen atoms fluorine, chlorine, bromine or iodine,
the substituents of the optionally substituted alkynyl groups are selected from among the halogen atoms fluorine, chlorine, bromine or iodine,
the substituents of the optionally substituted aryl groups are selected from among -OH,-NO₂, -CN, -OCHF₂, -OCF₃, -NH₂, halogen, -C₁-C₁₀-alkyl, -O-C₁-C₃-alkyl, -N-methyl-tetrahydrooxazinyl, -COOH, -COO-C₁-C₄-alkyl, -CONH₂, -CONH-C₁-C₁₀-alkyl, -CONH-C₃-C₈₋cycloalkyl, -CONH-heterocycloalkyl, -CONH-heteroaryl, -CONH-aryl, -CONHMeC₁-C₃-alkyl, benzimidazole or a group of the formula 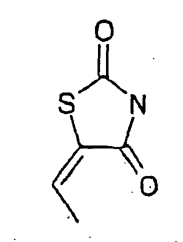 the substituents of the optionally substituted heteroaryl group are selected from among -OH,-NO₂, -CN, -OCHF₂, -OCF₃, -NH₂, halogen, -C₁-C₁₀-alkyl, -O-C₁-C₃-alkyl, -N-methyl-tetrahydrooxazinyl, -COOH, -COO-C₁-C₄-alkyl, -CONH₂, phenyl, heteroaryl, -CONH-C₁-C₁₀-alkyl, -CONH-C₃-C₈-cycloalkyl, -CONH-heteroaryl, -CONH-aryl, -CONHMeC₁-C₃-alkyl, benzimidazole or a group of the formula 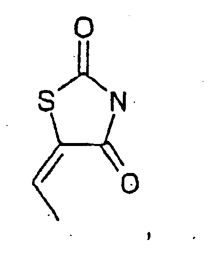 the substituents of the optionally substituted cycloalkyl group are selected from among one or more oxygen atoms linked via a double bond, wherein the cycloalkyl group may also be anellated to a benzene ring,
and, unless stated otherwise,
by the term "aryl" used in the definitions hereinbefore is meant an aromatic ring system with 6 to 14 carbon atoms,
by the term "heteroaryl" used in the definitions hereinbefore is meant a 5-10-membered mono- or bicyclic heteroaryl ring in which up to three carbon atoms may be replaced by one or more heteroatoms selected from among oxygen, nitrogen or sulphur,
by the term "cycloalkyl" used in the definitions hereinbefore is meant a saturated or unsaturated cycloalkyl group having 3 to 8 carbon atoms,
by the term "cycloalkyl" used in the definitions hereinbefore is meant a 5-, 6- or 7-membered, saturated or unsaturated, heterocyclic group which may contain nitrogen, oxygen or sulphur as the heteroatom, while each of the above-mentioned heterocyclic groups may optionally also be anellated to a benzene ring,
optionally in the form of the tautomers, the racemates, the enantiomers, the diastereomers and the mixtures thereof, and optionally the pharmacologically acceptable acid addition salts thereof.

2. Compounds according to claim 1, wherein
X and R⁶ have the meaning indicated, and
R¹ denotes hydrogen,
R² denotes a group selected from among a CHO, OH, and CH₃ group,
R³, R⁴ which may be identical or different denote a group selected from among hydrogen, optionally substituted C₁-C₆-alkyl, C₂-C₆-alkenyl, C₂-C₆-alkynyl, C₃-C₇-cycloalkyl, or
R³ and R⁴ together denote a C₂-C₅-alkyl bridge,
R⁵ denotes a group selected from among optionally substituted C₁-C₁₀₋alkyl, C₂-C₁₀-alkenyl, C₂-C₁₀-alkynyl and C₃-C₆-cycloalkyl, or
R³ and R⁵ or R⁴ and R⁵ together denote a saturated or unsaturated C₃-C₄-alkyl bridge which may contain 1 to 2 heteroatoms, and
R⁷ denotes hydrogen,
optionally in the form of the tautomers, the racemates, the enantiomers, the diastereomers and the mixtures thereof, and optionally the pharmacologically acceptable acid addition salts thereof.

3. Compounds according to claim 1 or 2, wherein
R¹-R⁵, R⁷, R⁸ and X have the meaning indicated, and
R⁶ denotes a group of general formula 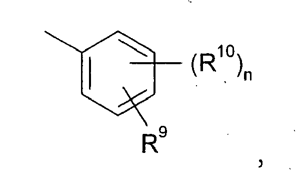
wherein
n denotes 1, 2, 3 or 4,
R⁹ denotes a group selected from among optionally substituted C₁-C₆₋alkyl, C₂-C₆-alkenyl, C₂-C₆-alkynyl, -CONH-C₁-C₁₀-alkylene, -O-aryl, -O-heteroaryl, -O-cycloalkyl, -O-heterocycloalkyl, aryl, heteroaryl, cycloalkyl and heterocycloalkyl or
a group selected from among -O-C₁-C₆-alkyl-Q¹, -CONR⁸-C₁-C₁₀₋alkyl-Q¹, -CONR⁸-C₂-C₁₀-alkenyl-Q¹, -CONR⁸-Q², halogen, OH, -SO₂R⁸, -SO₂N(R⁸)₂, -COR⁸, -COOR⁸, -N(R⁸)₂, -NHCOR⁸, CONR⁸OC₁-C₁₀ alkylQ¹ and CONR⁸OQ²,
Q¹ denotes hydrogen, -NHCOR⁸, or a group selected from among an optionally substituted -NH-aryl, -NH-heteroaryl, aryl, heteroaryl, C₃₋C₈-cycloalkyl and heterocycloalkyl group,
Q² denotes hydrogen or a group selected from among an optionally substituted aryl, heteroaryl, C₃-C₈-heterocycloalkyl, C₃-C₈-cycloalkyl and C₁-C₄-alkyl-C₃-C₈-cycloalkyl group,
R¹⁰ which may be identical or different denotes a group selected from among optionally substituted C₁-C₆-alkyl, C₂-C₆-alkenyl and C₂-C₆-alkynyl, -O-C₁-C₆-alkyl, -O-C₂-C₆-alkenyl, -O-C₂-C₆-alkynyl, C₃-C₆-heterocycloalkyl and C₃-C₆-cycloalkyl, or
a group selected from among hydrogen, -CONH₂, -COOR⁸, -OCON(R⁸)₂, -N(R⁸)₂, -NHCOR⁸, -NHCON(R⁸)₂, -NO₂ and halogen,
or
adjacent groups R⁹ and R¹⁰ together denote a bridge of general formula 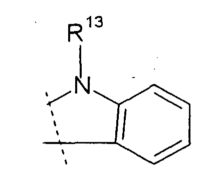 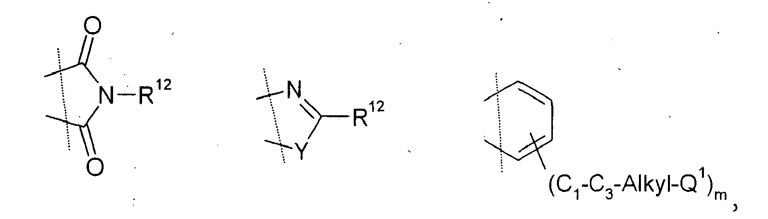
Y denotes O, S or NR¹¹,
m denotes 0, 1 or 2
R¹¹ denotes hydrogen or C₁-C₂-alkyl, and
R¹² denotes hydrogen or a group selected from among optionally substituted phenyl, pyridyl, pyrazinyl, pyrimidinyl, pyridazinyl, -C₁-C₃₋alkyl-phenyl, -C₁-C₃-alkyl-pyridyl, -C₁-C₃-alkyl-pyrazinyl, -C₁-C₃-alkyl-pyrimidinyl and -C₁-C₃-alkyl-pyridazinyl,
R¹³ denotes C₁-C₆-alkyl
optionally in the form of the tautomers, the racemates, the enantiomers, the diastereomers and the mixtures thereof, and optionally the pharmacologically acceptable acid addition salts thereof.

4. Compounds according to one of claims 1 to 3, wherein
R³-R⁶, R⁸ and X have the meaning indicated, and
R¹ denotes hydrogen,
R² denotes CH₃ , and
R⁷ denotes hydrogen,
optionally in the form of the tautomers, the racemates, the enantiomers, the diastereomers and the mixtures thereof, and optionally the pharmacologically acceptable acid addition salts thereof.

5. Compound of formula I according to one of claims 1 to 4 for use as a pharmaceutical composition.

6. Compound of formula I according to one of claims 1 to 4 for use as a pharmaceutical composition with an antiproliferative activity.

7. Use of a compound of formula I for preparing a pharmaceutical composition for the treatment and/or prevention of cancer, infections, inflammatory and autoimmune diseases.

8. Pharmaceutical preparations, containing as active substance one or more compounds of general formula (I) according to one of claims 1 to 4 or the physiologically acceptable salts thereof, optionally combined with conventional excipients and/or carriers.

9. Process for preparing a compound of general formula (I), 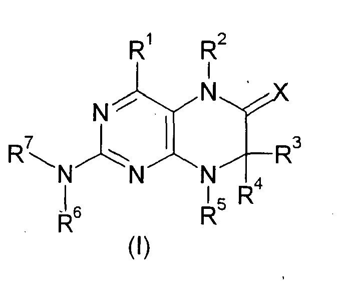 wherein
R¹-R⁷ and X have the meanings given in claims 1 to 4,
**characterised in that** a compound of general formula (II) 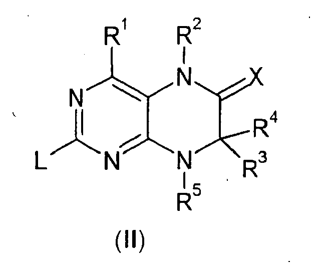 wherein
R¹-R⁵ and X have the meanings given in claims 1 to 4 and L is a leaving group,
is reacted with an optionally substituted compound of general formula (III) 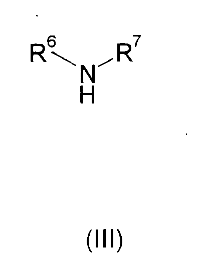 wherein
R⁶ and R⁷ have the meanings given in claims 1 to 4.

10. Compound of formula (II), 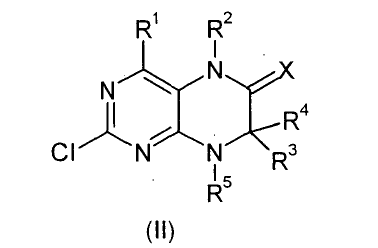 wherein
R¹-R⁵ and X have the meanings given in claims 1 to 4.

11. Process for preparing a compound of general formula (I), 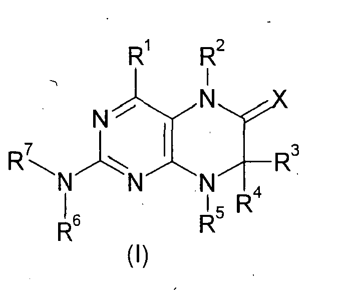 wherein
R⁶ denotes a group of general formula, 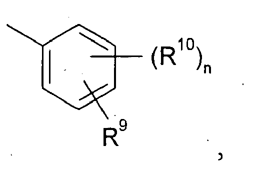 R⁹ denotes an optionally substituted group -CONH-C₁-C₁₀-alkylene or a group selected from among -CONR⁸-C₁-C₁₀-alkyl-Q¹, -CONR⁸-C₂-C₁₀₋alkenyl-Q¹, -CONR⁸-Q², and -COOR⁸,
R¹-R⁵, R⁷, R¹⁰, n and X are as defined as in claims 1 to 4, and R⁸ is as defined in claim 1,
**characterised in that** a compound of general formula (IA) 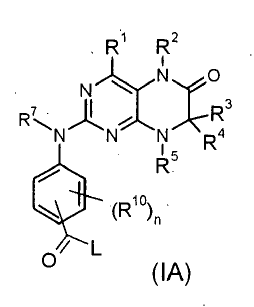 wherein
R¹ to R⁵, R⁷, R¹⁰ and n are as defined as in claims 1 to 4, and
L denotes a leaving group,
is reacted with a primary or secondary amine to form the corresponding amide or is reacted with an alcohol to form the corresponding ester.

## Revendications

1. Composés de formule générale (I) 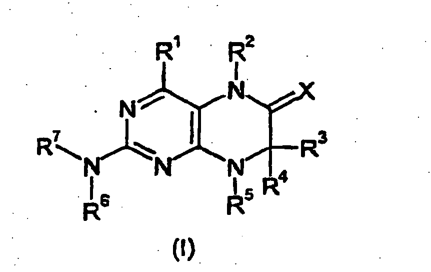 où
R¹ représente un groupement choisi dans le groupe consistant en l'hydrogène, NH₂, XH, halogène et un groupe C₁-C₃-alkyle éventuellement substitué par un ou plusieurs atomes d'halogène,
R² représente un groupement choisi dans le groupe consistant en l'hydrogène, CHO, XH, -X-C₁-C₂-alkyle et un groupe C₁-C₃-alkyle éventuellement substitué,
R³, R⁴, identiques ou différents, représentent un groupement choisi dans le groupe consistant en C₁-C₁₀-alkyle, C₂-C₁₀-alcényle, C₂-C₁₀-alcynyle, aryle, hétéroaryle, C₃-C₈-cycloalkyle, -X-aryle, -X-hétéroaryle, -X-cycloalkyle, -NR⁸-aryle, -NR⁸⁻hétéroaryle, -NR⁸-cycloalkyle ou -NR⁸-hétérocycloalkyle, X-hétérocycloalkyle, C₃-C₈-hétérocycloalkyle éventuellement substitué ou un groupement choisi dans le groupe consistant en l'hydrogène, halogène, COXR⁸, CON(R⁸)₂, COR⁸ et XR⁸, ou
R³ et R⁴ représentent ensemble un pont alkyle à 2 à 5 membres qui peut contenir 1 à 2 hétéroatomes,
R⁵ représente l'hydrogène ou un groupement choisi dans le groupe consistant en C₁-C₁₀-alkyle, C₂-C₁₀-alcényle, C₂-C₁₀-alcynyle, aryle, hétéroaryle et C₃-C₆₋cycloalkyle éventuellement substitué, ou
R³ et R⁵ ou R⁴ et R⁵ représentent ensemble un pont C₃-C₄-alkyle saturé ou insaturé qui peut contenir 1 à 2 hétéroatomes,
R⁶ représente aryle ou hétéroaryle éventuellement substitué,
R⁷ représente l'hydrogène ou -CO-X-C₁-C₄-alkyle, et
X représente dans chaque cas indépendamment O ou S,
R⁸ représente à chaque fois indépendamment l'hydrogène ou un groupement choisi dans le groupe consistant en C₁-C₄-alkyle, C₂-C₄-alcényle, C₂-C₄-alcynyle et phényle éventuellement substitué,
où, sauf indication contraire,
les substituants des groupes alkyle éventuellement substitués sont choisis dans le groupe consistant en les atomes d'halogène fluor, chlore, brome ou iode, un groupement choisi dans le groupe consistant en -CN, -OCOCH₃, aryle, hétéroaryle, hétérocycloalkyle saturé ou insaturé, un groupement amine, un système cyclique bicyclique saturé ou insaturé,
les substituants des groupes alcényle éventuellement substitués sont choisis dans le groupe consistant en les atomes d'halogène fluor, chlore, brome ou iode,
les substituants des groupes alcynyle éventuellement substitués sont choisis dans le groupe consistant en les atomes d'halogène fluor, chlore, brome ou iode,
les substituants des groupes aryle éventuellement substitués sont choisis dans le groupe consistant en -OH, -NO₂, -CN, -OCHF₂, -OCF₃, -NH₂, halogène, -C₁₋C₁₀-alkyle, -O-C₁-C₃-alkyle, -N-méthyl-tétrahydrooxazinyle, -COOH, -COO-C₁₋C₄-alkyle, -CONH₂, -CONH-C₁-C₁₀-alkyle, -CONH-C₃-C₆-cycloalkyle, -CONH-hétérocycloalkyle, -CONH-hétéroaryle, -CONH-aryle, -CONMe-C₁-C₃-alkyle, benzimidazole ou un groupement de formule 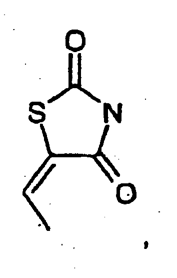 les substituants du groupe hétéroaryle éventuellement substitué sont choisis dans le groupe consistant en -OH, -N02, -CN, -OCHF₂, -OCF₃, -NH₂, halogène, -C₁₋C₁₀-alkyle, -O-C₁-C₃-alkyle, -N-méthyl-tétrahydrooxazinyle, -COOH, -COO-C₁₋C₄-alkyle, -CONH₂, phényle, hétéroaryle, -CONH-C₁-C₁₀-alkyle, -CONH-C₃-C₆₋cycloalkyle, -CONH-hétéroaryle, -CONH-aryle, -CONMe-C₁-C₃-alkyle, benzimidazole ou un groupement de formule 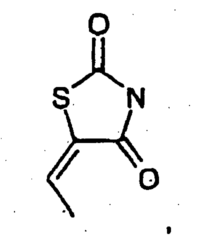 les substituants du groupe cycloalkyle éventuellement substitué sont choisis dans le groupe consistant en un ou plusieurs atomes d'oxygène liés par le biais d'une double liaison, où le groupe cycloalkyle peut aussi être condensé à un cycle benzénique,
et où, sauf indication contraire,
par l'expression « aryle » citée précédemment dans les définitions, on doit comprendre un système cyclique aromatique ayant 6 à 14 atomes de carbone,
par l'expression « hétéroaryle » citée précédemment dans les définitions, on doit comprendre un cycle hétéroaryle mono- ou bicyclique à 5-10 membres dans lequel jusqu'à trois atomes C peuvent être remplacés par un ou plusieurs hétéroatomes choisis dans le groupe oxygène, azote ou soufre,
par l'expression « cycloalkyle » citée précédemment dans les définitions, on doit comprendre un groupement cycloalkyle saturé ou insaturé ayant 3-8 atomes de carbone,
par l'expression « hétérocycloalkyle » citée précédemment dans les définitions, on doit comprendre un hétérocycle saturé ou insaturé à 5, 6 ou 7 membres qui peut contenir comme hétéroatome l'azote, l'oxygène ou le soufre, où chacun des hétérocycles cités précédemment peut en outre éventuellement être condensé à un cycle benzénique,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

2. Composés selon la revendication 1 où
X et R⁶ présentent la signification indiquée, et
R¹ représente l'hydrogène,
R² représente un groupement choisi dans le groupe consistant en un groupe CHO, OH et CH₃,
R³, R⁴, identiques ou différents, représentent un groupement choisi dans le groupe consistant en l'hydrogène, C₁-C₆-alkyle, C₂-C₆-alcényle, C₂-C₆-alcynyle, C₃-C₇₋cycloalkyle éventuellement substitué, ou
R³ et R⁴ représentent ensemble un pont C₂-C₅-alkyle,
R⁵ représente un groupement choisi dans le groupe consistant en C₁-C₁₀-alkyle, C₂-C₁₀-alcényle, C₂-C₁₀-alcynyle et C₃-C₆-cycloalkyle éventuellement substitué, ou
R³ et R⁵ ou R⁴ et R⁵ représentent ensemble un pont C₃-C₄-alkyle saturé ou insaturé qui peut contenir 1 à 2 hétéroatomes, et
R⁷ représente l'hydrogène
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

3. Composés selon la revendication 1 ou 2 où
R¹-R⁵, R⁷, R⁸ et X présentent la signification indiquée, et
R⁶ représente un groupement de formule générale 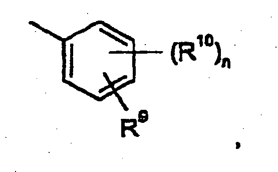 où
n représente 1, 2, 3 ou 4,
R⁹ représente un groupement choisi dans le groupe consistant en C₁-C₆-alkyle, C₂₋C₆-alcényle, C₂-C₆-alcynyle, -CONH-C₁-C₁₀-alkylène, -O-aryle, -O-hétéroaryle, -O-cycloalkyle, -O-hétérocycloalkyle, aryle, hétéroaryle, cycloalkylé et hétérocycloalkyle éventuellement substitué ou
un groupement choisi dans le groupe consistant en -O-C₁-C₆-alkyl-Q¹, -CONR⁸⁻C₁-C₁₀-alkyl-Q¹, -CONR⁸-C₂-C₁₀-alcényl-Q¹, -CONR⁸-Q², halogène, OH, -SO₂R⁸, -SO₂N(R⁸)₂, -COR⁸, -COOR⁸, -N(R⁸)₂, -NHCOR⁸, CONR⁸OC₁-C₁₀-alkyl-Q¹ et CONR⁸OQ²,
Q¹ représente l'hydrogène, -NHCOR⁸ ou un groupement choisi dans le groupe consistant en un groupe -NH-aryle, -NH-hétéroaryle, aryle, hétéroaryle, C₃-C₈₋cycloalkyle et hétérocycloalkyle éventuellement substitué,
Q² représente l'hydrogène ou un groupement choisi dans le groupe consistant en un groupe aryle, hétéroaryle, C₃-C₈-hétérocycloalkyle, C₃-C₈-cycloalkyle et C₁-C₄₋alkyl-C₃-C₈-cycloalkyle éventuellement substitué,
R¹⁰, identiques ou différents, représentent un groupement choisi dans le groupe consistant en C₁-C₆-alkyle, C₂-C₆-alcényle et C₂-C₆-alcynyle, -O-C₁-C₆-alkyle, -OC₂-C₆-alcényle, -O-C₂-C₆-alcynyle, -C₃-C₆-hétérocycloalkyle et -C₃-C₆₋cycloalkyle éventuellement substitué, ou
un groupement choisi dans le groupe consistant en l'hydrogène, -CONH₂, -COOR⁸, -OCON(R⁸)₂, -N(R⁸)₂, -NHCOR⁸, -NHCON(R⁸)₂, -NO₂ et halogène, ou
des groupements R⁹ et R¹⁰ voisins représentent ensemble un pont de formule générale 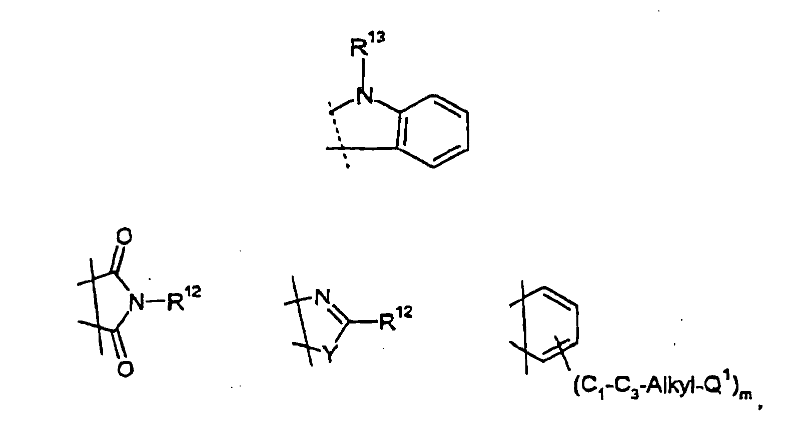
Y représente O, S ou NR¹¹,
m représente 0, 1 ou 2,
R¹¹ représente l'hydrogène ou C₁-C₂-alkyle, et
R¹² représente l'hydrogène ou un groupement choisi dans le groupe consistant en phényle, pyridyle, pyrazinyle, pyrimidinyle, pyridazinyle, -C₁-C₃-alkyl-phényle, -C₁-C₃-alkyl-pyridyle, -C₁-C₃-alkyl-pyrazinyle, -C₁-C₃-alkyl-pyrimidinyle et -C₁₋C₃-alkyl-pyridazinyle,
R¹³ représente -C₁-C₆-alkyle,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

4. Composés selon l'une des revendications 1 à 3 où
R³ - R⁶, R⁸ et X présentent la signification indiquée, et
R¹ représente l'hydrogène,
R² représente CH₃ et
R⁷ représente l'hydrogène,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

5. Composé de formule I selon l'une des revendications 1 à 4 destiné à être utilisé comme médicament.

6. Composé de formule I selon l'une des revendications 1 à 4 destiné à être utilisé comme médicament à effet antiprolifératif.

7. Utilisation d'un composé de formule I pour la production d'un médicament pour le traitement et/ou la prévention du cancer, des infections, des maladies inflammatoires et auto-immunes.

8. Préparations pharmaceutiques contenant comme principe actif un ou plusieurs composés de formule générale (I) selon l'une des revendications 1 à 4 ou leurs sels physiologiquement acceptables éventuellement en combinaison avec des adjuvants et/ou supports courants.

9. Procédé de production d'un composé de formule générale (I) 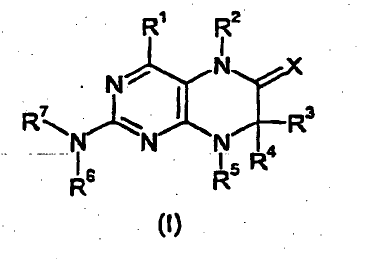 où
R¹ - R⁷ et X présentent la signification indiquée dans les revendications 1 à 4, **caractérisé en ce qu'**un composé de formule générale (II) 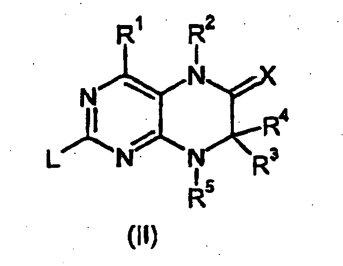
où
R¹ - R⁵ et X présentent la signification indiquée dans les revendications 1 à 4 et L est un groupe partant
est mis à réagir avec un composé éventuellement substitué de formule générale (III) 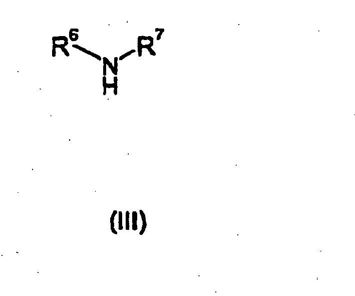
où
R⁶ et R⁷ présentent la signification indiquée dans les revendications 1 à 4.

10. Composé de formule (II) 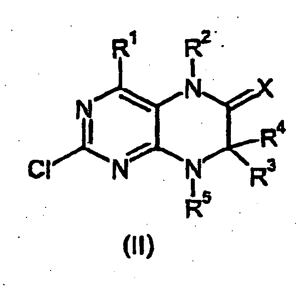 où
R¹ - R⁵ et X présentent la signification indiquée dans les revendications 1 à 4.

11. Procédé de production d'un composé de formule générale (I) 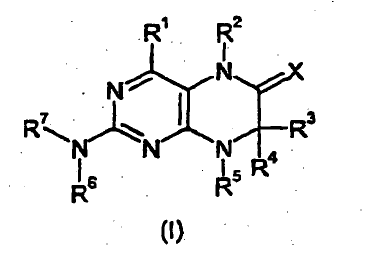 où
R⁶ représente un groupement de formule générale 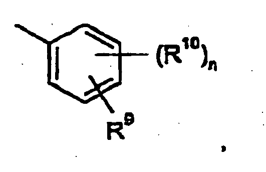
R⁹ représente un groupement -CONH-C₁-C₁₀-alkylène éventuellement substitué ou un groupement choisi dans le groupe consistant en -CONR⁸-C₁-C₁₀-alkyl-Q¹, -CONR⁸-C₂-C₁₀-alcényl-Q¹, -CONR⁸-Q² et -COOR⁸,
R¹ - R⁵, R⁷, R¹⁰, n et X présentent la signification indiquée dans les revendications 1 à 4, et R⁸ présente la signification indiquée dans la revendication 1,
**caractérisé en ce qu'**un composé de formule générale (IA) 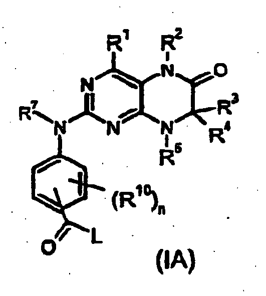 où
R¹ à R⁵, R⁷, R¹⁰ et n présentent la signification indiquée dans les revendications 1 à 4, et
L représente un groupe partant,
est converti avec une amine primaire ou secondaire en l'amide correspondant ou avec un alcool en l'ester correspondant.
